# EUROPEAN PATENT SPECIFICATION

(11) **EP 1 379 283 B1**
(45) Date of publication and mention of the grant of the patent: **13.02.2008**
(21) Application number: 01994412.3
(22) Date of filing: 19.12.2001
(51) Int. Cl.: A61K 51/00, C07K 1/04, C07K 5/10, C07K 14/47

(54) **IDENTIFICATION OF TARGET-SPECIFIC FOLDING SITES IN PEPTIDES AND PROTEINS**
IDENTIFIZIERUNG ZIELGERICHTETER FALTUNGSSTELLEN IN PEPTIDEN UND PROTEINEN
IDENTIFICATION DE SITES DE REPLIEMENT SPECIFIQUES A LA CIBLE DANS DES PEPTIDES ET DES PROTEINES

(30) Priority: 19.12.2000 US 256842 P; 11.07.2001 US 304835 P; 04.10.2001 US 327835 P
(43) Date of publication of application: 14.01.2004
(73) Proprietor: Palatin Technologies, Inc., Princeton, NJ 08540 (US)
(72) Inventor: SHARMA, Shubh, D., Cranbury, NJ 08512 (US); SHI, Yi-Qun, East Brunswick, NJ 08816 (US)
(74) Representative: Kupecz, Arpad
(86) International application number: PCT/US2001/050075
(87) International publication number: WO 2002/064734

(56) References cited:
- WO-A-00/36136
- WO-A-01/13112
- US-A- 5 410 020
- US-A- 5 891 418
- US-A- 6 027 711
- US-A- 6 083 758
- US-B1- 6 331 285
- AL-OBEIDI F ET AL: "DESIGN, SYNTHESIS, AND BIOLOGICAL ACTIVITIES OF A POTENT AND SELECTIVE MELANOTROPIN ANTAGONIST" INTERNATIONAL JOURNAL OF PEPTIDE AND PROTEIN RESEARCH, MUNKSGAARD, COPENHAGEN, DK, vol. 35, no. 3, 1 March 1990 (1990-03-01), pages 228-234, XP000099724 ISSN: 0367-8377
- MACCOLL ET AL.: 'Interrelationships among biological activity, disulfide bonds, secondary structure and metal ion binding for a chemically synthesized 34-amino-acid peptide derived from alpha-fetoprotein' BIOCHEM. BIOPHYS. ACTA vol. 1528, no. 2-3, 03 October 2001, pages 127 - 134, XP004312009

### SEQUENCE LISTING

<110> Palatin Technologies, Inc.
   Sharma, Shubh D.
   Shi, Yi-Qun
<120> Identification of Target-Specific Folding Sites in Peptides and Proteins
<130> 70025-PCT-14
<150> US 60/256,842
   <151> 2000-12-19
<150> US 60/304,835
   <151> 2001-02-13
<150> US 60/327,835
   <151> 2001-10-04
<160> 171
<170> PatentIn version 3.1
<210> 1
   <211> 5
   <212> PRT
   <213> Artificial Sequence
<220>
   <223> Artificial metal binding protein
<400> 1
   Ala Ala Ala Cys Ala
   1               5
<210> 2
   <211> 11
   <212> PRT
   <213> Homo sapiens
<220>
   <221> misc_feature
   <223> Amino terminal fragment (positions 21-30) of urokinase-type tissue plasminogen activator
<400> 2
   Val Ser Asn Lys Tyr Phe Ser Asn Ile His Trp
   1               5                   10
<210> 3
   <211> 12
   <212> PRT
   <213> Artificial Sequence
<220>
   <223> UPA receptor metallopeptide library
<400> 3
   Val Ser Asn Lys Tyr Phe Ser Asn Ile His Cys Trp
   1               5                   10
<210> 4
   <211> 12
   <212> PRT
   <213> Artificial Sequence
<220>
   <223> UPA receptor metallopeptide library
<400> 4
   Val Ser Cys Asn Lys Tyr Phe Ser Asn Ile His Trp
   1               5                   10
<210> 5
   <211> 12
   <212> PRT
   <213> Artificial Sequence
<220>
   <223> UPA receptor metallopeptide library
<400> 5
   Val Ser Asn Cys Lys Tyr Phe Ser Asn Ile His Trp
   1 5 10
<210> 6
   <211> 12
   <212> PRT
   <213> Artificial Sequence
<220>
   <223> UPA receptor metallopeptide library
<400> 6
   Val Ser Asn Lys Cys Tyr Phe Ser Asn Ile His Trp
   1 5 10
<210> 7
   <211> 12
   <212> PRT
   <213> Artificial Sequence
<220>
   <223> UPA receptor metallopeptide library
<400> 7
   Val Ser Asn Lys Tyr Cys Phe Ser Asn Ile His Trp
   1 5 10
<210> 8
   <211> 12
   <212> PRT
   <213> Artificial Sequence
<220>
   <223> UPA receptor metallopeptide library
<400> 8
   Val Ser Asn Lys Tyr Phe Cys Ser Asn Ile His Trp
   1 5 10
<210> 9
   <211> 12
   <212> PRT
   <213> Artificial Sequence
<220>
   <223> UPA receptor metallopeptide library
<400> 9
   Val Ser Asn Lys Tyr Phe Ser Cys Asn Ile His Trp
   1 5 10
<210> 10
   <211> 12
   <212> PRT
   <213> Artificial Sequence
<220>
   <223> Artificial Sequence
<400> 10
   Val Ser Asn Lys Tyr Phe Ser Asn Cys Ile His Trp
   1 5 10
<210> 11
   <211> 12
   <212> PRT
   <213> Artificial Sequence
<220>
   <223> UPA receptor metallopeptide library
<400> 11
   Val Ser Asn Lys Tyr Phe Ser Asn Ile Cys His Trp
   1 5 10
<210> 12
   <211> 12
   <212> PRT
   <213> Artificial Sequence
<220>
   <223> UPA receptor metallopeptide library
<400> 12
   Val Ser Asn Lys Tyr Phe Ser Asn Ile His Trp Cys
   1 5 10
<210> 13
   <211> 11
   <212> PRT
   <213> Artificial Sequence
<220>
   <223> Amyloid beta-protein related peptide
<400> 13
   His Glu Lys Leu Val Phe Phe Ala Glu Asp Val
   1 5 10
<210> 14
   <211> 5
   <212> PRT
   <213> Artificial Sequence
<220>
   <223> Amyloid beta-protein related peptide
<400> 14
   Leu Ala Phe Phe Asp
   1 5
<210> 15
   <211> 5
   <212> PRT
   <213> Artificial Sequence
<220>
   <223> Amyloid beta-protein related peptide
<400> 15
   Leu Pro Phe Phe Asp
   1 5
<210> 16
   <211> 12
   <212> PRT
   <213> Artificial Sequence
<220>
   <223> Amyloid beta-protein metallopeptide library
<220>
   <221> MISC_FEATURE
   <222> (5)..(5)
   <223> Val, Pro, Gly or Ala
<400> 16
   His Gln Lys Leu Xaa Phe Phe Ala Glu Asp Val Cys
   1 5 10
<210> 17
   <211> 12
   <212> PRT
   <213> Artificial Sequence
<220>
   <223> Amyloid beta-protein metallopeptide library
<220>
   <221> MISC_FEATURE
   <222> (5)..(5)
   <223> Val, Pro, Gly or Ala
<400> 17
   His Gln Lys Leu Xaa Phe Phe Ala Glu Asp Cys Val
   1 5 10
<210> 18
   <211> 12
   <212> PRT
   <213> Artificial Sequence
<220>
   <223> Amyloid beta-protein metallopeptide library
<220>
   <221> MISC_FEATURE
   <222> (5)..(5)
   <223> Val, Pro, Gly or Ala
<400> 18
   His Gln Lys Leu Xaa Phe Phe Ala Glu Cys Asp Val
   1 5 10
<210> 19
   <211> 12
   <212> PRT
   <213> Artificial Sequence
<220>
   <223> Amyloid beta-protein metallopeptide library
<220>
   <221> MISC_FEATURE
   <222> (5)..(5)
   <223> Val, Pro, Gly or Ala
<400> 19
   His Gln Lys Leu Xaa Phe Phe Ala Cys Glu Asp Val
   1 5 10
<210> 20
   <211> 12
   <212> PRT
   <213> Artificial Sequence
<220>
   <223> Amyloid beta-protein metallopeptide library
<220>
   <221> MISC_FEATURE
   <222> (5)..(5)
   <223> Val, Pro, Gly or Ala
<400> 20
   His Gln Lys Leu Xaa Phe Phe Cys Ala Glu Asp Val
   1 5 10
<210> 21
   <211> 12
   <212> PRT
   <213> Artificial Sequence
<220>
   <223> Amyloid beta-protein metallopeptide library
<220>
   <221> MISC_FEATURE
   <222> (5)..(5)
   <223> Val, Gly or Ala
<400> 21
   His Gln Lys Leu Xaa Phe Phe Cys Ala Glu Asp Val
   1 5 10
<210> 22
   <211> 12
   <212> PRT
   <213> Artificial Sequence
<220>
   <223> Amyloid beta-protein metallopeptide library
<220>
   <221> MISC_FEATURE
   <222> (5)..(5)
   <223> Val, Gly or Ala
<400> 22
   His Gln Lys Leu Xaa Phe Cys Phe Ala Glu Asp Val
   1 5 10
<210> 23
   <211> 12
   <212> PRT
   <213> Artificial Sequence
<220>
   <223> Amyloid beta-protein metallopeptide library
<220>
   <221> MISC_FEATURE
   <222> (5)..(5)
   <223> Val, Gly or Ala
<400> 23
   His Gln Lys Leu Xaa Cys Phe Phe Ala Glu Asp Val
   1 5 10
<210> 24
   <211> 12
   <212> PRT
   <213> Artificial Sequence
<220>
   <223> Amyloid beta-protein metallopeptide library
<220>
   <221> MISC_FEATURE
   <222> (6)..(6)
   <223> Val, Pro, Gly or Ala
<400> 24
   His Gln Lys Leu Cys Xaa Phe Phe Ala Glu Asp Val
   1 5 10
<210> 25
   <211> 12
   <212> PRT
   <213> Artificial Sequence
<220>
   <223> Amyloid beta-protein metallopeptide library
<220>
   <221> MISC_FEATURE
   <222> (6)..(6)
   <223> Val, Pro, Gly or Ala
<400> 25
   His Gln Lys Cys Leu Xaa Phe Phe Ala Glu Asp Val
   1 5 10
<210> 26
   <211> 12
   <212> PRT
   <213> Artificial Sequence
<220>
   <223> Amyloid beta-protein metallopeptide library
<220>
   <221> MISC_FEATURE
   <222> (6)..(6)
   <223> Val, Pro, Gly or Ala
<400> 26
   His Gln Cys Lys Leu Xaa Phe Phe Ala Glu Asp Val
   1 5 10
<210> 27
   <211> 8
   <212> PRT
   <213> Artificial Sequence
<220>
   <223> N-terminus sequence for amyloid beta-protein metallopeptide library
<220>
   <221> MISC_FEATURE
   <222> (4)..(4)
   <223> Val, Pro, Gly or Ala
<400> 27
   Gln Lys Leu Xaa Phe Phe Ala Glu
   1 5
<210> 28
   <211> 7
   <212> PRT
   <213> Artificial Sequence
<220>
   <223> N-terminus sequence for amyloid beta-protein metallopeptide library
<220>
   <221> MISC_EEATURE
   <222> (3)..(3)
   <223> Val, Pro, Gly or Ala
<400> 28
   Lys Leu Xaa Phe Phe Ala Glu
   1 5
<210> 29
   <211> 6
   <212> PRT
   <213> Artificial Sequence
<220>
   <223> N-terminus sequence for amyloid beta-protein metallopeptide library
<220>
   <221> MISC_FEATURE
   <222> (2)..(2)
   <223> Val, Pro, Gly or Ala
<400> 29
   Leu Xaa Phe Phe Ala Glu
   1 5
<210> 30
   <211> 5
   <212> PRT
   <213> Artificial Sequence
<220>
   <223> N-terminus sequence for amyloid beta-protein metallopeptide library
<220>
   <221> MISC_FEATURE
   <222> (1)..(1)
   <223> Val, Pro, Gly or Ala
<400> 30
   Xaa Phe Phe Ala Glu
   1 5
<210> 31
   <211> 4
   <212> PRT
   <213> Artificial Sequence
<220>
   <223> N-terminus sequence for amyloid beta-protein metallopeptide library
<400> 31
   Phe Phe Ala Glu
   1
<210> 32
   <211> 8
   <212> PRT
   <213> Artificial Sequence
<220>
   <223> N-terminus sequence for amyloid beta-protein metallopeptide library
<220>
   <221> MISC_FEATURE
   <222> (5)..(5)
   <223> Val, Pro, Gly or Ala
<400> 32
   His Gln Lys Leu Xaa Phe Phe Ala
   1 5
<210> 33
   <211> 7
   <212> PRT
   <213> Artificial Sequence
<220>
   <223> N-terminus sequence for amyloid beta-protein metallopeptide library
<220>
   <221> MISC_FEATURE
   <222> (4)..(4)
   <223> Val, Pro, Gly or Ala
<400> 33
   Gln Lys Leu Xaa Phe Phe Ala
   1 5
<210> 34
   <211> 6
   <212> PRT
   <213> Artificial Sequence
<220>
   <223> N-terminus sequence for amyloid beta-protein metallopeptide library
<220>
   <221> MISC_FEATURE
   <222> (3)..(3)
   <223> Val, Pro, Gly or Ala
<400> 34
   Lys Leu Xaa Phe Phe Ala
   1               5
<210> 35
   <211> 5
   <212> PRT
   <213> Artificial Sequence
<220>
   <223> N-terminus sequence for amyloid beta-protein metallopeptide library
<220>
   <221> MISC_FEATURE
   <222> (2)..(2)
   <223> Val, Pro, Gly or Ala
<400> 35
   Leu Xaa Phe Phe Ala
   1               5
<210> 36
   <211> 7
   <212> PRT
   <213> Artificial Sequence
<220>
   <223> N-terminus sequence for amyloid beta-protein metallopeptide library
<220>
   <221> MISC_FEATURE
   <222> (5)..(5)
   <223> Val, Pro, Gly or Ala
<400> 36
   His Gln Lys Leu Xaa Phe Phe
   1               5
<210> 37
   <211> 6
   <212> PRT
   <213> Artificial Sequence
<220>
   <223> N-terminus sequence for amyloid beta-protein metallopeptide library
<220>
   <221> MISC_FEATURE
   <222> (4)..(4)
   <223> Val, Pro, Gly or Ala
<400> 37
   Gln Lys Leu Xaa Phe Phe
   1 5
<210> 38
   <211> 5
   <212> PRT
   <213> Artificial Sequence
<220>
   <223> N-terminus sequence for amyloid beta-protein metallopeptide library
<220>
   <221> MISC_FEATURE
   <222> (3)..(3)
   <223> Val, Pro, Gly or Ala
<400> 38
   Lys Leu Xaa Phe Phe
   1 5
<210> 39
   <211> 6
   <212> PRT
   <213> Artificial Sequence
<220>
   <223> N-terminus sequence for amyloid beta-protein metallopeptide library
<220>
   <221> MISC_FEATURE
   <222> (5)..(5)
   <223> Val, Pro, Gly or Ala
<400> 39
   His Gln Lys Leu Xaa Phe
   1 5
<210> 40
   <211> 5
   <212> PRT
   <213> Artificial Sequence
<220>
   <223> N-terminus sequence for amyloid beta-protein metallopeptide library
<220>
   <221> MISC_FEATURE
   <222> (4)..(4)
   <223> Val, Pro, Gly or Ala
<400> 40
   Gln Lys Leu Xaa Phe
   1 5
<210> 41
   <211> 5
   <212> PRT
   <213> Artificial Sequence
<220>
   <223> N-terminus sequence for amyloid beta-protein metallopeptide library
<220>
   <221> MISC_FEATURE
   <222> (5)..(5)
   <223> Val, Pro, Gly or Ala
<400> 41
   His Gln Lys Leu Xaa
   1 5
<210> 42
   <211> 5
   <212> PRT
   <213> Artificial Sequence
<220>
   <223> N-terminus sequence for amyloid beta-protein metallopeptide library
<220>
   <221> MISC_FEATURE
   <222> (5)..(5)
   <223> Val, Gly or Ala
<400> 42
   His Gln Lys Leu Xaa
   1 5
<210> 43
   <211> 4
   <212> PRT
   <213> Artificial Sequence
<220>
   <223> C-terminus sequence for amyloid beta-protein metallopeptide library
<400> 43
   Ala Glu Asp Val
   1
<210> 44
   <211> 4
   <212> PRT
   <213> Artificial Sequence
<220>
   <223> N-terminus sequence for amyloid beta-protein metallopeptide library
<400> 44
   His Gln Lys Leu
   1
<210> 45
   <211> 5
   <212> PRT
   <213> Artificial Sequence
<220>
   <223> C-terminus sequence for amyloid beta-protein metallopeptide library
<400> 45
   Phe Ala Glu Asp Val
   1 5
<210> 46
   <211> 4
   <212> PRT
   <213> Artificial Sequence
<220>
   <223> C-terminus sequence for amyloid beta-protein metallopeptide library
<400> 46
   Phe Ala Glu Asp
   1
<210> 47
   <211> 6
   <212> PRT
   <213> Artificial Sequence
<220>
   <223> C-terminus sequence for amyloid beta-protein metallopeptide library
<400> 47
   Phe Phe Ala Glu Asp Val
   1               5
<210> 48
   <211> 5
   <212> PRT
   <213> Artificial Sequence
<220>
   <223> C-terminus sequence for amyloid beta-protein metallopeptide library
<400> 48
   Phe Phe Ala Glu Asp
   1               5
<210> 49
   <211> 4
   <212> PRT
   <213> Artificial Sequence
<220>
   <223> C-terminus sequence for amyloid beta-protein metallopeptide library
<400> 49
   Phe Phe Ala Glu
   1
<210> 50
   <211> 7
   <212> PRT
   <213> Artificial Sequence
<220>
   <223> C-terminus sequence for amyloid beta-protein metallopeptide library
<220>
   <221> MISC FEATURE
   <222> (1)..(1)
   <223> Val, Pro, Gly or Ala
<400> 50
   Xaa Phe Phe Ala Glu Asp Val
   1 5
<210> 51
   <211> 6
   <212> PRT
   <213> Artificial Sequence
<220>
   <223> C-terminus sequence for amyloid beta-protein metallopeptide library
<220>
   <221> MISC_FEATURE
   <222> (1)..(1)
   <223> Val, Pro, Gly or Ala
<400> 51
   Xaa Phe Phe Ala Glu Asp
   1 5
<210> 52
   <211> 5
   <212> PRT
   <213> Artificial Sequence
<220>
   <223> C-terminus sequence for amyloid beta-protein metallopeptide library
<220>
   <221> MISC FEATURE
   <222> (1)..(1)
   <223> Val, Pro, Gly or Ala
<400> 52
   Xaa Phe Phe Ala Glu
   1 5
<210> 53
   <211> 7
   <212> PRT
   <213> Artificial Sequence
<220>
   <223> C-terminus sequence for amyloid beta-protein metallopeptide library
<220>
   <221> MISC FEATURE
   <222> (1)..(1)
   <223> Val, Gly or Ala
<400> 53
   Xaa Phe Phe Ala Glu Asp Val
   1 5
<210> 54
   <211> 6
   <212> PRT
   <213> Artificial Sequence
<220>
   <223> C-terminus sequence for amyloid beta-protein metallopeptide library
<220>
   <221> MISC FEATURE
   <222> (1)..(1)
   <223> Val, Gly or Ala
<400> 54
   Xaa Phe Phe Ala Glu Asp
   1 5
<210> 55
   <211> 5
   <212> PRT
   <213> Artificial Sequence
<220>
   <223> C-terminus sequence for amyloid beta-protein metallopeptide library
<220>
   <221> MISC_FEATURE
   <222> (1)..(1)
   <223> Val, Gly or Ala
<400> 55
   Xaa Phe Phe Ala Glu
   1 5
<210> 56
   <211> 4
   <212> PRT
   <213> Artificial Sequence
<220>
   <223> Amyloid beta-protein metallopeptide library
<400> 56
   His Gln Lys Cys
   1
<210> 57
   <211> 8
   <212> PRT
   <213> Artificial Sequence
<220>
   <223> C-terminus sequence for amyloid beta-protein metallopeptide library
<220>
   <221> MISC_FEATURE
   <222> (2)..(2)
   <223> Val, Pro, Gly or Ala
<400> 57
   Leu Xaa Phe Phe Ala Glu Asp Val
   1 5
<210> 58
   <211> 7
   <212> PRT
   <213> Artificial Sequence
<220>
   <223> C-terminus sequence for amyloid beta-protein metallopeptide library
<220>
   <221> MISC_FEATURE
   <222> (2)..(2)
   <223> Val, Pro, Gly or Ala
<400> 58
   Leu Xaa Phe Phe Ala Glu Asp
   1 5
<210> 59
   <211> 6
   <212> PRT
   <213> Artificial Sequence
<220>
   <223> C-terminus sequence for amyloid beta-protein metallopeptide library
<220>
   <221> MISC FEATURE
   <222> (2)..(2)
   <223> Val, Pro, Gly or Ala
<400> 59
   Leu Xaa Phe Phe Ala Glu
   1 5
<210> 60
   <211> 5
   <212> PRT
   <213> Artificial Sequence
<220>
   <223> C-terminus sequence for amyloid beta-protein metallopeptide library
<220>
   <221> MISC FEATURE
   <222> (2)..(2)
   <223> Val, Pro, Gly or Ala
<400> 60
   Leu Xaa Phe Phe Glu
   1 5
<210> 61
   <211> 9
   <212> PRT
   <213> Artificial Sequence
<220>
   <223> C-terminus sequence for amyloid beta-protein metallopeptide library
<220>
   <221> MISC FEATURE
   <222> (3)..(3)
   <223> Val, Pro, Gly or Ala
<400> 61
   Lys Leu Xaa Phe Phe Ala Glu Asp Val
   1 5
<210> 62
   <211> 8
   <212> PRT
   <213> Artificial Sequence
<220>
   <223> C-terminus sequence for amyloid beta-protein metallopeptide library
<220>
   <221> MISC FEATURE
   <222> (3)..(3)
   <223> Val, Pro, Gly or Ala
<400> 62
   Lys Leu Xaa Phe Phe Ala Glu Asp
   1               5
<210> 63
   <211> 7
   <212> PRT
   <213> Artificial Sequence
<220>
   <223> C-terminus sequence for amyloid beta-protein metallopeptide library
<220>
   <221> MISC FEATURE
   <222> (3)..(3)
   <223> Val, Pro, Gly or Ala
<400> 63
   Lys Leu Xaa Phe Phe Ala Glu
   1               5
<210> 64
   <211> 6
   <212> PRT
   <213> Artificial Sequence
<220>
   <223> C-terminus sequence for amyloid beta-protein metallopeptide library
<220>
   <221> MISC_FEATURE
   <222> (3)..(3)
   <223> Val, Pro, Gly or Ala
<400> 64
   Lys Leu Xaa Phe Phe Glu
   1               5
<210> 65
   <211> 5
   <212> PRT
   <213> Artificial Sequence
<220>
   <223> C-terminus sequence for amyloid beta-protein metallopeptide library
<220>
   <221> MISC FEATURE
   <222> (3)..(3)
   <223> Val, Pro, Gly or Ala
<400> 65
   Lys Leu Xaa Phe Phe
   1               5

<210> 66
   <211> 13
   <212> PRT
   <213> Artificial Sequence
<220>
   <223> Prion disease treatment related peptide
<400> 66
   Asp Ala Pro Ala Ala Pro Ala Gly Pro Ala Val Pro Val
   1               5                   10
<210> 67
   <211> 14
   <212> PRT
   <213> Artificial Sequence
<220>
   <223> Prion disease treatment metallopeptide library
<220>
   <221> MISC_FEATURE
   <222> (12)..(12)
   <223> Gly or Ala
<400> 67
   Asp Ala Pro Ala Ala Pro Ala Gly Pro Ala Val Xaa Val Cys
   1               5                   10
<210> 68
   <211> 14
   <212> PRT
   <213> Artificial Sequence
<220>
   <223> Prion disease treatment metallopeptide library
<220>
   <221> MISC_FEATURE
   <222> (12)..(12)
   <223> Gly or Ala
<400> 68
   Asp Ala Pro Ala Ala Pro Ala Gly Pro Ala Val Xaa Cys Val
   1 5 10
<210> 69
   <211> 14
   <212> PRT
   <213> Artificial Sequence
<220>
   <223> Prion disease treatment metallopeptide library
<220>
   <221> MISC_FEATURE
   <222> (9)..(9)
   <223> Pro, Gly or Ala
<220>
   <221> MISC_FEATURE
   <222> (13)..(13)
   <223> Pro, Gly or Ala
<400> 69
   Asp Ala Pro Ala Ala Pro Ala Gly Xaa Ala Val Cys Xaa Val
   1 5 10
<210> 70
   <211> 14
   <212> PRT
   <213> Artificial Sequence
<220>
   <223> Prion disease treatment metallopeptide library
<220>
   <221> MISC_FEATURE
   <222> (9)..(9)
   <223> Gly or Ala
<400> 70
   Asp Ala Pro Ala Ala Pro Ala Gly Xaa Ala Cys Val Pro Val
   1 5 10
<210> 71
   <211> 14
   <212> PRT
   <213> Artificial Sequence
<220>
   <223> Prion disease treatment metallopeptide library
<220>
   <221> MISC_FEATURE
   <222> (9)..(9)
   <223> Gly or Ala
<400> 71
   Asp Ala Pro Ala Ala Pro Ala Gly Xaa Cys Ala Val Pro Val
   1 5 10
<210> 72
   <211> 14
   <212> PRT
   <213> Artificial Sequence
<220>
   <223> Prion disease treatment metallopeptide library
<220>
   <221> MISC_FEATURE
   <222> (6)..(6)
   <223> Pro, Gly or Ala
<220>
   <221> MISC_FEATURE
   <222> (10)..(10)
   <223> Pro, Gly or Ala
<400> 72
   Asp Ala Pro Ala Ala Xaa Ala Gly Cys Xaa Ala Val Pro Val
   1 5 10
<210> 73
   <211> 14
   <212> PRT
   <213> Artificial Sequence
<220>
   <223> Prion disease treatment metallopeptide library
<220>
   <221> MISC_FEATURE
   <222> (6)..(6)
   <223> Gly or Ala
<400> 73
   Asp Ala Pro Ala Ala Xaa Ala Cys Gly Pro Ala Val Pro Val
   1 5 10
<210> 74
   <211> 14
   <212> PRT
   <213> Artificial Sequence
<220>
   <223> Prion disease treatment metallopeptide library
<220>
   <221> MISC_FEATURE
   <222> (6)..(6)
   <223> Gly or Ala
<400> 74
   Asp Ala Pro Ala Ala Xaa Cys Ala Gly Pro Ala Val Pro Val
   1               5                   10
<210> 75
   <211> 14
   <212> PRT
   <213> Artificial Sequence
<220>
   <223> Prion disease treatment metallopeptide library
<220>
   <221> MISC_FEATURE
   <222> (3)..(3)
   <223> Pro, Gly or Ala
<220>
   <221> MISC_FEATURE
   <222> (7)..(7)
   <223> Pro, Gly or Ala
<400> 75
   Asp Ala Xaa Ala Ala Cys Xaa Ala Gly Pro Ala Val Pro Val
   1               5                   10
<210> 76
   <211> 14
   <212> PRT
   <213> Artificial Sequence
<220>
   <223> Prion disease treatment metallopeptide library
<220>
   <221> MISC_FEATURE
   <222> (3)..(3)
   <223> Gly or Ala
<400> 76
   Asp Ala Xaa Ala Cys Ala Pro Ala Gly Pro Ala Val Pro Val
   1 5 10
<210> 77
   <211> 14
   <212> PRT
   <213> Artificial Sequence
<220>
   <223> Prion disease treatment metallopeptide library
<220>
   <221> MISC_FEATURE
   <222> (3)..(3)
   <223> Gly or Ala
<400> 77
   Asp Ala Xaa Cys Ala Ala Pro Ala Gly Pro Ala Val Pro Val
   1 5 10
<210> 78
   <211> 14
   <212> PRT
   <213> Artificial Sequence
<220>
   <223> Prion disease treatment metallopeptide library
<220>
   <221> MISC_FEATURE
   <222> (4)..(4)
   <223> Pro, Gly or Ala
<400> 78
   Asp Ala Cys Xaa Ala Ala Pro Ala Gly Pro Ala Val Pro Val
   1 5 10
<210> 79
   <211> 11
   <212> PRT
   <213> Artificial Sequence
<220>
   <223> N-terminus sequence for prion disease treatment metallopeptide library
<400> 79
   Asp Ala Pro Ala Ala Pro Ala Gly Pro Ala Val
   1 5 10
<210> 80
   <211> 10
   <212> PRT
   <213> Artificial Sequence
<220>
   <223> N-terminus sequence for prion disease treatment metallopeptide library
<400> 80
   Ala Pro Ala Ala Pro Ala Gly Pro Ala Val
   1 5 10
<210> 81
   <211> 9
   <212> PRT
   <213> Artificial Sequence
<220>
   <223> N-terminus sequence for prion disease treatment metallopeptide library
<400> 81
   Pro Ala Ala Pro Ala Gly Pro Ala Val
   1 5
<210> 82
   <211> 8
   <212> PRT
   <213> Artificial Sequence
<220>
   <223> N-terminus sequence for prion disease treatment metallopeptide library
<400> 82
   Ala Ala Pro Ala Gly Pro Ala Val
   1 5
<210> 83
   <211> 7
   <212> PRT
   <213> Artificial Sequence
<220>
   <223> N-terminus sequence for prion disease treatment metallopeptide library
<400> 83
   Ala Pro Ala Gly Pro Ala Val
   1 5
<210> 84
   <211> 6
   <212> PRT
   <213> Artificial Sequence
<220>
   <223> N-terminus sequence for prion disease treatment metallopeptide library
<400> 84
   Pro Ala Gly Pro Ala Val
   1 5
<210> 85
   <211> 5
   <212> PRT
   <213> Artificial Sequence
<220>
   <223> N-terminus sequence for prion disease treatment metallopeptide library
<400> 85
   Ala Gly Pro Ala Val
   1 5
<210> 86
   <211> 4
   <212> PRT
   <213> Artificial Sequence
<220>
   <223> N-terminus sequence for prion disease treatment metallopeptide library
<400> 86
   Gly Pro Ala Val
   1
<210> 87
   <211> 10
   <212> PRT
   <213> Artificial Sequence
<220>
   <223> N-terminus sequence for prion disease treatment metallopeptide library
<400> 87
   Asp Ala Pro Ala Ala Pro Ala Gly Pro Ala
   1 5 10
<210> 88
   <211> 9
   <212> PRT
   <213> Artificial Sequence
<220>
   <223> N-terminus sequence for prion disease treatment metallopeptide library
<400> 88
   Ala Pro Ala Ala Pro Ala Gly Pro Ala
   1 5
<210> 89
   <211> 8
   <212> PRT
   <213> Artificial Sequence
<220>
   <223> N-terminus sequence for prion disease treatment metallopeptide library
<400> 89
   Pro Ala Ala Pro Ala Gly Pro Ala
   1 5
<210> 90
   <211> 7
   <212> PRT
   <213> Artificial Sequence
<220>
   <223> N-terminus sequence for prion disease treatment metallopeptide library
<400> 90
   Ala Ala Pro Ala Gly Pro Ala
   1 5
<210> 91
   <211> 6
   <212> PRT
   <213> Artificial Sequence
<220>
   <223> N-terminus sequence for prion disease treatment metallopeptide library
<400> 91
   Ala Pro Ala Gly Pro Ala
   1               5
<210> 92
   <211> 5
   <212> PRT
   <213> Artificial Sequence
<220>
   <223> N-terminus sequence for prion disease treatment metallopeptide library
<400> 92
   Pro Ala Gly Pro Ala
   1               5
<210> 93
   <211> 4
   <212> PRT
   <213> Artificial Sequence
<220>
   <223> N-terminus sequence for prion disease treatment metallopeptide library
<400> 93
   Ala Gly Pro Ala
   1
<210> 94
   <211> 9
   <212> PRT
   <213> Artificial Sequence
<220>
   <223> N-terminus sequence for prion disease treatment metallopeptide library
<220>
   <221> MISC_FEATURE
   <222> (9)..(9)
   <223> Pro, Gly or Ala
<400> 94
   Asp Ala Pro Ala Ala Pro Ala Gly Xaa
   1 5
<210> 95
   <211> 8
   <212> PRT
   <213> Artificial Sequence
<220>
   <223> N-terminus sequence for prion disease treatment metallopeptide library
<220>
   <221> MISC_FEATURE
   <222> (8)..(8)
   <223> Pro, Gly or Ala
<400> 95
   Ala Pro Ala Ala Pro Ala Gly Xaa
   1 5
<210> 96
   <211> 7
   <212> PRT
   <213> Artificial Sequence
<220>
   <223> N-terminus sequence for prion disease treatment metallopeptide library
<220>
   <221> MISC_FEATURE
   <222> (7)..(7)
   <223> P, G or A
<220>
   <221> MISC_FEATURE
   <222> (7)..(7)
   <223> Pro, Gly or Ala
<400> 96
   Pro Ala Ala Pro Ala Gly Xaa
   1 5
<210> 97
   <211> 6
   <212> PRT
   <213> Artificial Sequence
<220>
   <223> N-terminus sequence for prion disease treatment metallopeptide library
<220>
   <221> MISC_FEATURE
   <222> (6)..(6)
   <223> Pro, Gly or Ala
<400> 97
   Ala Ala Pro Ala Gly Xaa
   1 5
<210> 98
   <211> 5
   <212> PRT
   <213> Artificial Sequence
<220>
   <223> N-terminus sequence for prion disease treatment metallopeptide library
<220>
   <221> MISC_FEATURE
   <222> (5)..(5)
   <223> Pro, Gly or Ala
<400> 98
   Ala Pro Ala Gly Xaa
   1 5
<210> 99
   <211> 8
   <212> PRT
   <213> Artificial Sequence
<220>
   <223> N-terminus sequence for prion disease treatment metallopeptide library
<220>
   <221> MISC_FEATURE
   <222> (4)..(4)
   <223> P, G or A
<400> 99
   Asp Ala Pro Ala Ala Pro Ala Gly
   1 5
<210> 100
   <211> 7
   <212> PRT
   <213> Artificial Sequence
<220>
   <223> N-terminus sequence for prion disease treatment metallopeptide library
<400> 1.00
<210> 101
   <211> 6
   <212> PRT
   <213> Artificial Sequence
<220>
   <223> N-terminus sequence for prion disease treatment metallopeptide library
<400> 101
   Pro Ala Ala Pro Ala Gly
   1 5
<210> 102
   <211> 5
   <212> PRT
   <213> Artificial Sequence
<220>
   <223> N-terminus sequence for prion disease treatment metallopeptide library
<400> 102
   Ala Ala Pro Ala Gly
   1 5
<210> 103
   <211> 4
   <212> PRT
   <213> Artificial Sequence
<220>
   <223> N-terminus sequence for prion disease treatment metallopeptide library
<400> 103
   Ala Pro Ala Gly
   1
<210> 104
   <211> 7
   <212> PRT
   <213> Artificial Sequence
<220>
   <223> Prion disease treatment metallopeptide library
<220>
   <221> MISC_FEATURE
   <222> (2)..(2)
   <223> Gly or Ala
<400> 104
   Gly Xaa Cys Ala Val Pro Val
   1               5
<210> 105
   <211> 7
   <212> PRT
   <213> Artificial Sequence
<220>
   <223> N-terminus sequence for prion disease treatment metallopeptide library
<400> 105
   Asp Ala Pro Ala Ala Pro Ala
   1               5
<210> 106
   <211> 6
   <212> PRT
   <213> Artificial Sequence
<220>
   <223> N-terminus sequence for prion disease treatment metallopeptide library
<400> 106
   Ala Pro Ala Ala Pro Ala
   1               5
<210> 107
   <211> 5
   <212> PRT
   <213> Artificial Sequence
<220>
   <223> N-terminus sequence for prion disease treatment metallopeptide library
<400> 107
   Pro Ala Ala Pro Ala
   1 5
<210> 108
   <211> 4
   <212> PRT
   <213> Artificial Sequence
<220>
   <223> N-terminus sequence for prion disease treatment metallopeptide library
<400> 108
   Ala Ala Pro Ala
   1
<210> 109
   <211> 4
   <212> PRT
   <213> Artificial Sequence
<220>
   <223> C-terminus sequence for prion disease treatment metallopeptide library
<400> 109
   Ala Val Pro Val
   1
<210> 110
   <211> 6
   <212> PRT
   <213> Artificial Sequence
<220>
   <223> N-terminus sequence for prion disease treatment metallopeptide library
<220>
   <221> MISC_FEATURE
   <222> (6)..(6)
   <223> Pro, Gly or Ala
<400> 110
   Asp Ala Pro Ala Ala Xaa
   1 5
<210> 111
   <211> 5
   <212> PRT
   <213> Artificial Sequence
<220>
   <223> N-terminus sequence for prion disease treatment metallopeptide library
<220>
   <221> MISC_FEATURE
   <222> (5)..(5)
   <223> Pro, Gly or Ala
<400> 111
   Ala Pro Ala Ala Xaa
   1 5
<210> 112
   <211> 5
   <212> PRT
   <213> Artificial Sequence
<220>
   <223> N-terminus sequence for prion disease treatment metallopeptide library
<400> 112
   Asp Ala Pro Ala Ala
   1 5
<210> 113
   <211> 4
   <212> PRT
   <213> Artificial Sequence
<220>
   <223> N-terminus sequence for prion disease treatment metallopeptide library
<400> 113
   Ala Pro Ala Ala
   1
<210> 114
   <211> 6
   <212> PRT
   <213> Artificial Sequence
<220>
   <223> C-terminus sequence for prion disease treatment metallopeptide library
<400> 114
   Gly Pro Ala Val Pro Val
   1 5
<210> 115
   <211> 5
   <212> PRT
   <213> Artificial Sequence
<220>
   <223> C-terminus sequence for prion disease treatment metallopeptide library
<400> 115
   Gly Pro Ala Val Pro
   1 5
<210> 116
   <211> 4
   <212> PRT
   <213> Artificial Sequence
<220>
   <223> C-terminus sequence for prion disease treatment metallopeptide library
<400> 116
   Gly Pro Ala Val
   1
<210> 117
   <211> 4
   <212> PRT
   <213> Artificial Sequence
<220>
   <223> N-terminus sequence for prion disease treatment metallopeptide library
<400> 117
   Asp Ala Pro Ala
   1
<210> 118
   <211> 7
   <212> PRT
   <213> Artificial Sequence
<220>
   <223> C-terminus sequence for prion disease treatment metallopeptide library
<400> 118
   Ala Gly Pro Ala Val Pro Val
   1 5
<210> 119
   <211> 6
   <212> PRT
   <213> Artificial Sequence
<220>
   <223> C-terminus sequence for prion disease treatment metallopeptide library
<400> 119
   Ala Gly Pro Ala Val Pro
   1 5
<210> 120
   <211> 5
   <212> PRT
   <213> Artificial Sequence
<220>
   <223> C-terminus sequence for prion disease treatment metallopeptide library
<400> 120
   Ala Gly Pro Ala Val
   1 5
<210> 121
   <211> 4
   <212> PRT
   <213> Artificial Sequence
<220>
   <223> C-terminus sequence for prion disease treatment metallopeptide library
<400> 121
   Ala Gly Pro Ala
   1
<210> 122
   <211> 8
   <212> PRT
   <213> Artificial Sequence
<220>
   <223> C-terminus sequence for prion disease treatment metallopeptide library
<220>
   <221> MISC_FEATURE
   <222> (1)..(1)
   <223> Pro, Gly or Ala
<400> 122
   Xaa Ala Gly Pro Ala Val Pro Val
   1               5
<210> 123
   <211> 7
   <212> PRT
   <213> Artificial Sequence
<220>
   <223> C-terminus sequence for prion disease treatment metallopeptide library
<220>
   <221> MISC FEATURE
   <222> (1)..(1)
   <223> Pro, Gly or Ala
<400> 123
   Xaa Ala Gly Pro Ala Val Pro
   1               5
<210> 124
   <211> 6
   <212> PRT
   <213> Artificial Sequence
<220>
   <223> C-terminus sequence for prion disease treatment metallopeptide library
<220>
   <221> MISC FEATURE
   <222> (1)..(1)
   <223> Pro, Gly or Ala
<400> 124
   Xaa Ala Gly Pro Ala Val
   1               5
<210> 125
   <211> 5
   <212> PRT
   <213> Artificial Sequence
<220>
   <223> C-terminus sequence for prion disease treatment metallopeptide library
<220>
   <221> MISC_FEATURE
   <222> (1)..(1)
   <223> Pro, Gly or Ala
<400> 125
   Xaa Ala Gly Pro Ala
   1               5
<210> 126
   <211> 12
   <212> PRT
   <213> Artificial Sequence
<220>
   <223> Prion disease treatment metallopeptide library
<220>
   <221> MISC_FEATURE
   <222> (3)..(3)
   <223> Gly or Ala
<400> 126
   Asp Ala Xaa Ala Cys Ala Gly Pro Ala Val Pro Val
   1               5                   10
<210> 127
   <211> 9
   <212> PRT
   <213> Artificial Sequence
<220>
   <223> C-terminus sequence for prion disease treatment metallopeptide library
<400> 127
   Ala Pro Ala Gly Pro Ala Val Pro Val
   1               5
<210> 128
   <211> 8
   <212> PRT
   <213> Artificial Sequence
<220>
   <223> C-terminus sequence for prion disease treatment metallopeptide library
<400> 128
   Ala Pro Ala Gly Pro Ala Val Pro
   1               5
<210> 129
   <211> 7
   <212> PRT
   <213> Artificial Sequence
<220>
   <223> C-terminus sequence for prion disease treatment metallopeptide library
<400> 129
   Ala Pro Ala Gly Pro Ala Val
   1               5
<210> 130
   <211> 6
   <212> PRT
   <213> Artificial Sequence
<220>
   <223> C-terminus sequence for prion disease treatment metallopeptide library
<400> 130
   Ala Pro Ala Gly Pro Ala
   1               5
<210> 131
   <211> 5
   <212> PRT
   <213> Artificial Sequence
<220>
   <223> C-terminus sequence for prion disease treatment metallopeptide library
<400> 131
   Ala Pro Ala Gly Pro
   1               5
<210> 132
   <211> 4
   <212> PRT
   <213> Artificial Sequence
<220>
   <223> C-terminus sequence for prion disease treatment metallopeptide library
<400> 132
   Ala Pro Ala Gly
   1
<210> 133
   <211> 10
   <212> PRT
   <213> Artificial Sequence
<220>
   <223> C-terminus sequence for prion disease treatment metallopeptide library
<400> 133
   Ala Ala Pro Ala Gly Pro Ala Val Pro Val
   1 5 10
<210> 134
   <211> 9
   <212> PRT
   <213> Artificial Sequence
<220>
   <223> C-terminus sequence for prion disease treatment metallopeptide library
<400> 134
   Ala Ala Pro Ala Gly Pro Ala Val Pro
   1 5
<210> 135
   <211> 8
   <212> PRT
   <213> Artificial Sequence
<220>
   <223> C-terminus sequence for prion disease treatment metallopeptide library
<400> 135
   Ala Ala Pro Ala Gly Pro Ala Val
   1 5
<210> 136
   <211> 7
   <212> PRT
   <213> Artificial Sequence

<220>
   <223> C-terminus sequence for prion disease treatment metallopeptide library
<400> 136
   Ala Ala Pro Ala Gly Pro Ala
   1 5
<210> 137
   <211> 6
   <212> PRT
   <213> Artificial Sequence
<220>
   <223> C-terminus sequence for prion disease treatment metallopeptide library
<400> 137
   Ala Ala Pro Ala Gly Pro
   1 5
<210> 138
   <211> 5
   <212> PRT
   <213> Artificial Sequence
<220>
   <223> C-terminus sequence for prion disease treatment metallopeptide library
<400> 138
   Ala Ala Pro Ala Gly
   1 5
<210> 139
   <211> 4
   <212> PRT
   <213> Artificial Sequence
<220>
   <223> C-terminus sequence for prion disease treatment metallopeptide library
<400> 139
   Ala Ala Pro Ala
   1
<210> 140
   <211> 14
   <212> PRT
   <213> Artificial Sequence
<220>
   <223> Prion disease treatment metallopeptide library
<220>
   <221> MISC_FEATURE
   <222> (4)..(4)
   <223> Pro, Gly or Ala
<400> 140
   Asp Ala Cys Xaa Ala Ala Pro Ala Gly Pro Ala Val Pro Val
   1               5                   10
<210> 141
   <211> 11
   <212> PRT
   <213> Artificial Sequence
<220>
   <223> C-terminus sequence for prion disease treatment metallopeptide library
<220>
   <221> MISC FEATURE
   <222> (1)..(1)
   <223> Pro, Gly or Ala
<400> 141
   Xaa Ala Ala Pro Ala Gly Pro Ala Val Pro Val
   1               5                   10
<210> 142
   <211> 10
   <212> PRT
   <213> Artificial Sequence
<220>
   <223> C-terminus sequence for prion disease treatment metallopeptide library
<220>
   <221> MISC FEATURE
   <222> (1)..(1)
   <223> Pro, Gly or Ala
<400> 142
   Xaa Ala Ala Pro Ala Gly Pro Ala Val Pro
   1               5                   10
<210> 143
   <211> 9
   <212> PRT
   <213> Artificial Sequence
<220>
   <223> C-terminus sequence for prion disease treatment metallopeptide library
<220>
   <221> MISC_FEATURE
   <222> (1)..(1)
   <223> Pro, Gly or Ala
<400> 143
   Xaa Ala Ala Pro Ala Gly Pro Ala Val
   1               5
<210> 144
   <211> 8
   <212> PRT
   <213> Artificial Sequence
<220>
   <223> C-terminus sequence for prion disease treatment metallopeptide library
<220>
   <221> MISC_FEATURE
   <222> (1)..(1)
   <223> Pro, Gly or Ala
<400> 144
   Xaa Ala Ala Pro Ala Gly Pro Ala
   1               5
<210> 145
   <211> 7
   <212> PRT
   <213> Artificial Sequence
<220>
   <223> C-terminus sequence for prion disease treatment metallopeptide library
<220>
   <221> MISC FEATURE
   <222> (1)..(1)
   <223> Pro, Gly or Ala
<400> 145
   Xaa Ala Ala Pro Ala Gly Pro
   1 5
<210> 146
   <211> 6
   <212> PRT
   <213> Artificial Sequence
<220>
   <223> C-terminus sequence for prion disease treatment metallopeptide library
<220>
   <221> MISC_FEATURE
   <222> (1)..(1)
   <223> Pro, Gly or Ala
<400> 146
   Xaa Ala Ala Pro Ala Gly
   1 5
<210> 147
   <211> 5
   <212> PRT
   <213> Artificial Sequence
<220>
   <223> C-terminus sequence for prion disease treatment metallopeptide library
<220>
   <221> MISC_FEATURE
   <222> (1)..(1)
   <223> Pro, Gly or Ala
<400> 147
   Xaa Ala Ala Pro Ala
   1 5
<210> 148
   <211> 9
   <212> PRT
   <213> Homo sapiens
<220>
   <221> misc_feature
   <223> Oxytocin receptor ligand
<400> 148
   Cys Tyr Ile Gln Asn Cys Pro Leu Gly
   1 5
<210> 149
   <211> 10
   <212> PRT
   <213> Artificial Sequence
<220>
   <223> Oxytocin receptor metallopeptide library
<400> 149
   Ala Tyr Ile Gln Asn Ala Pro Leu Gly Cys
   1 5 10
<210> 150
   <211> 10
   <212> PRT
   <213> Artificial Sequence
<220>
   <223> Oxytocin receptor metallopeptide library
<400> 150
   Ala Tyr Ile Gln Asn Ala Ala Leu Gly Cys
   1 5 10
<210> 151
   <211> 10
   <212> PRT
   <213> Artificial Sequence
<220>
   <223> Oxytocin receptor metallopeptide library
<400> 151
   Ala Tyr Ile Gln Asn Ala Ala Leu Cys Gly
   1 5 10
<210> 152
   <211> 10
   <212> PRT
   <213> Artificial Sequence
<220>
   <223> Oxytocin receptor metallopeptide library
<400> 152
   Ala Tyr Ile Gln Asn Ala Ala Cys Leu Gly
   1 5 10
<210> 153
   <211> 10
   <212> PRT
   <213> Artificial Sequence
<220>
   <223> Oxytocin receptor metallopeptide library
<400> 153
   Ala Tyr Ile Gln Asn Ala Cys Pro Leu Gly
   1 5 10
<210> 154
   <211> 10
   <212> PRT
   <213> Artificial Sequence
<220>
   <223> Oxytocin receptor metallopeptide library
<400> 154
   Ala Tyr Ile Gln Asn Ala Cys Ala Leu Gly
   1 5 10
<210> 155
   <211> 10
   <212> PRT
   <213> Artificial Sequence
<220>
   <223> Oxytocin receptor metallopeptide library
<400> 155
   Ala Tyr Ile Gln Asn Cys Ala Pro Leu Gly
   1 5 10
<210> 156
   <211> 10
   <212> PRT
   <213> Artificial Sequence
<220>
   <223> Oxytocin receptor metallopeptide library
<400> 156
   Ala Tyr Ile Gln Cys Asn Ala Pro Leu Gly
   1 5 10
<210> 157
   <211> 10
   <212> PRT
   <213> Artificial Sequence
<220>
   <223> Oxytocin receptor metallopeptide library
<400> 157
   Ala Tyr Ile Cys Gln Asn Ala Pro Leu Gly
   1 5 10
<210> 158
   <211> 10
   <212> PRT
   <213> Artificial Sequence
<220>
   <223> Oxytocin receptor metallopeptide library
<400> 158
   Ala Tyr Cys Ile Gln Asn Ala Pro Leu Gly
   1 5 10
<210> 159
   <211> 8
   <212> PRT
   <213> Homo sapiens
<220>
   <221> MISC_FEATURE
   <222> (1)..(1)
   <223> Sarcosine
<220>
   <221> MISC_FEATURE
   <223> Angiotensin receptor ligand
<400> 159
   Xaa Arg Val Tyr Ile His Pro Thr
   1 5
<210> 160
   <211> 9
   <212> PRT
   <213> Artificial Sequence
<220>
   <223> Angiotensin receptor metallopeptide library
<220>
   <221> MISC_FEATURE
   <222> (1)..(1)
   <223> Sarcosine
<400> 160
   Xaa Arg Val Tyr Ile His Gly Cys Thr
   1               5
<210> 161
   <211> 9
   <212> PRT
   <213> Artificial Sequence
<220>
   <223> Angiotensin receptor metallopeptide library
<220>
   <221> MISC_FEATURE
   <222> (1)..(1)
   <223> Sarcosine
<400> 161
   Xaa Arg Val Tyr Ile His Cys Pro Thr
   1               5
<210> 162
   <211> 9
   <212> PRT
   <213> Artificial Sequence
<220>
   <223> Angiotensin receptor metallopeptide library
<220>
   <221> MISC_FEATURE
   <222> (1)..(1)
   <223> Sarcosine
<400> 162
   Xaa Arg Val Tyr Ile Cys His Pro Thr
   1 5
<210> 163
   <211> 9
   <212> PRT
   <213> Artificial Sequence
<220>
   <223> Angiotensin receptor metallopeptide library
<220>
   <221> MISC_FEATURE
   <222> (1)..(1)
   <223> Sarcosine
<400> 163
   Xaa Arg Val Tyr Cys Ile His Pro Thr
   1 5
<210> 164
   <211> 9
   <212> PRT
   <213> Artificial Sequence
<220>
   <223> Angiotensin receptor metallopeptide library
<220>
   <221> MISC_FEATURE
   <222> (1)..(1)
   <223> Sarcosine
<400> 164
   Xaa Arg Val Cys Tyr Ile His Pro Thr
   1 5
<210> 165
   <211> 9
   <212> PRT
   <213> Artificial Sequence
<220>
   <223> Angiotensin receptor metallopeptide library
<220>
   <221> MISC_FEATURE
   <222> (1)..(1)
   <223> Sarcosine
<400> 165
   Xaa Arg Cys Val Tyr Ile His Pro Thr
   1 5
<210> 166
   <211> 9
   <212> PRT
   <213> Artificial Sequence
<220>
   <223> Angiotensin receptor metallopeptide library
<220>
   <221> MISC_FEATURE
   <222> (1)..(1)
   <223> Sarcosine
<400> 166
   Xaa Arg Val Tyr Ile His Cys Gly Thr
   1 5
<210> 167
   <211> 6
   <212> PRT
   <213> Artificial Sequence
<220>
   <223> Amyloid beta-protein metallopeptide library
<400> 167
   Leu Pro Phe Phe Asp Cys
   1 5
<210> 168
   <211> 6
   <212> PRT
   <213> Artificial Sequence
<220>
   <223> Amyloid beta-protein metallopeptide library
<400> 168
   Leu Pro Phe Phe Cys Asp
   1 5
<210> 169
   <211> 6
   <212> PRT
   <213> Artificial Sequence
<220>
   <223> Amyloid beta-protein metallopeptide library
<400> 169
   Leu Ala Phe Phe Cys Asp
   1               5
<210> 170
   <211> 6
   <212> PRT
   <213> Artificial Sequence
<220>
   <223> Amyloid beta-protein metallopeptide library
<400> 170
   Leu Ala Phe Cys Phe Asp
   1               5
<210> 171
   <211> 6
   <212> PRT
   <213> Artificial Sequence
<220>
   <223> Amyloid beta-protein metallopeptide library
<400> 171
   Leu Ala Cys Phe Phe Asp
   1               5

## Description

### CROSS-REFERENCE TO RELATED APPLICATION

This application claims the benefit of the filing of U.S. Provisional Patent Application Serial No. 60/256,842, entitled *Iterative Deconvolution Of Target-Specific Folding Sites In Peptides,* filed on December 19, 2000; of U.S. Provisional Patent Application Serial No. 60/304,835, entitled *Metallopeptides for Treatment of Alzheimer's and Prion Disease,* filed on July 11, 2001; and of U.S. Provisional Patent Application Serial No. 60/327,835, entitled *Urokinase-Type Plasminogen Activator Receptor Specific Metallopeptides,* filed on October 4, 2001.

### BACKGROUND OF THE INVENTION

### Field of the Invention (Technical Field):

The present invention relates to methods for identification and determination of target-specific folding sites in peptides and proteins; methods to determine the specific sequence and local three-dimensional structure of that portion of peptides or proteins that bind to a receptor or target of interest, or mediate a biological activity of interest; methods to determine the pharmacophore of receptors or targets of interest; and directed libraries for identification and determination of target-specific folding sites in peptides and proteins and for identification and determination of pharmacophores of receptors or targets of interest.

### Background Art:

Note that the following discussion refers to a number of publications by author(s) and year of publication, and that due to recent publication dates certain publications are not to be considered as prior art vis-a-vis the present invention. Discussion of such publications herein is given for more complete background and is not to be construed as an admission that such publications are prior art for patentability determination purposes.

*Peptide and Protein Folding.* Determination of the biologically relevant structure of proteins and peptides, which can be characterized as a functional three-dimensional structure, is a difficult problem in the biological, biochemical and pharmaceutical sciences. Through use of any of a variety of methods the primary structure of relevant peptides or proteins may be ascertained. That is, the sequence of amino acid residues composing the peptide or protein is known, and it is known that the peptide or protein has a desired biological effect, such as binding a target molecule or receptor of interest, mediating a biological activity of interest, or the like. However, both the three-dimensional structure and sequence of the portion of the peptide or protein forming a ligand and thereby giving rise to the desired biological effect is unknown.

Peptides and proteins are highly flexible, due in large part to amino group and carboxyl group bonds of individual amino acid residues having a high rotational degree of freedom. In addition, some bonds in side chains of individual amino acid residues also have rotational degrees of freedom. The non-bonded steric interactions between amino acid residues force the peptide or protein along its degrees of freedom into some stable minimal free energy configuration. Local structures, also known as the "secondary structure," are common in peptides and proteins. These structures include α-helixes, β-bends, sheets, extended chains, loops and the like, and most often contribute to binding or receptor-specificity of peptides and proteins.

There are several types of α-helixes known, differing in torsion angles within the amino acid residues of the actual turn and by the patterns of intra- and inter-molecular hydrogen bonding. There are also a number of known different β-bends, differing in the dihedral torsion angles ψ (for the C*^{a}*-C bond) or Φ (for the C*^{a}*-N bond), or both.

A wide variety of mathematical, computational and others models have been developed for predicting the secondary structure of proteins and the secondary and tertiary structure of peptides, but no model gives satisfactory responses under other than the most limited circumstances. For example, software modeling programs (e.g., such as those distributed by Tripos, Inc., Pharmacopeia Inc. and the like), depend on various algorithms, statistical tools, assumed relationships between groups and the like, any or all of which may not be valid for any given protein or peptide. A number of methods are described in the art, such as those disclosed in International Publication No. WO 00/23564 to Xencor, Inc., International Publication Nos. WO 00/57309 and WO 01/35316, both to Structural Bioinformatics, Inc., International Publication No. WO 01/50355 to Structural Bioinformatics Advanced Technologies A/S, International Publication No. WO 01/59066 to Xencor, Inc., U.S. Patent No. 6,278,794 to Parekh et al., and U.S. Patent Application No. 2001/0000807 to Freire and Luque.

Generation of structure-based pharmacophores, utilizing experimental methods such as X-ray crystallography or NMR, optionally in conjunction with protein structure determination methods, such as homology modeling, is known in the art. However, in order for this approach to be employed it must be possible to obtain appropriate data from the ligand in the conformation specific for the receptor defining the pharmacophore. In many, if not most, instances this is not feasible.

It may be determined that a particular peptide or protein sequence, with a length between about five residues to about fifty or more residues, binds to a particular receptor. However, the specific residues actually participating in binding, and the local secondary structure of the sequence which contains these specific residues, is not known. Without this knowledge, it is impossible to devise a systematic rational approach to make peptide-based drugs, peptidomimetic drugs or other small molecule drugs. With knowledge of the specific residues and local secondary structure, it is possible to define the pharmacophore for the receptor. This definition may include, for example, the location in a three-dimensional construct of hydrogen bond donors and acceptors, positively and negatively charged centers, aromatic ring centers, hydrophobic centers and the like, preferably described in terms of the distances between the atoms in the pharmacophore.

U.S. Patent No. 5,834,250, to Wells et al., provides methods for the systematic analysis of the structure and function of polypeptides, specifically by identifying active domains by substituting a "scanning amino acid" for one of the amino acid residues within a suspected active domain of the parent polypeptide. These residue-substituted polypeptides are then assayed using a "target substance". In practice, a "scanning amino acid", such as alanine, is substituted for various residues in a polypeptide, and binding of the substituted polypeptide to a target substance compared to binding of the parent polypeptide. However, this method provides no direct information concerning the secondary structure of the active domain, nor information concerning the pharmacophore of the target substance. Similarly, U.S. Patent No. 6,084,066, to Evans and Kini, discloses homologues and analogs of naturally occurring polypeptides with "conformation-constraining moieties" flanking "interaction sites". However, this method requires that the "interaction site" or amino acid sequence be known. The "interaction site" sequence is then flanked on both termini with proline residues, which are asserted to stabilize interaction sites. This method similarly provides no direct information concerning the secondary structure of the "interaction site", nor information concerning the pharmacophore of the target substance.

There is thus a significant and substantial need to develop methods for identifying the specific residues in a peptide which are involved in binding to a receptor of interest, and to identify the specific secondary structure of the residues involved in binding.

*Metallopeptides*. It is known that linear peptides have high rotational degrees of freedom, such that for even small peptides with known primary structures the theoretically possible secondary and tertiary structures may number in the millions. In general cyclic peptides are more constrained, and at least small cyclic peptides have far fewer theoretically possible secondary and tertiary structures. However, even with cyclic peptides it is frequently impossible to predict with precision the actual secondary structures present in such peptide. By contrast, metallopeptides have well-defined and limited secondary structures, with the residues involved in metal ion complexation forming a turn structure about the metal ion. The atoms forming a part of the coordination sphere of the metal ion are fixed by the coordination geometry of the metal ion. This, coupled with the peptide bonds between residues and the side chain bonds, yields a conformationally fixed and predictable secondary structure for at least the residues involved in metal ion complexation. U.S. Patent No. 5,891,418, entitled *Peptide-Metal Ion Pharmaceutical Constructs and Applications,* U.S. Patent No. 6,027,711, entitled *Structurally Determined Metallo-Constructs and Applications,* and P.C.T. Patent Application Serial No. PCT/US99/29743, entitled *Metallopeptide Combinatorial Libraries and Applications,* each teach aspects of making and using metallopeptides and mimetics thereof, and each of the foregoing is Incorporated herein by reference. These patents and applications disclose receptor-specific metallopeptides and methods of making peptides and complexing the peptides to various metal ions.

There are methods for screening peptides for metal coordinating properties, such as disclosed in U.S. Patent No. 6,083,758 to Imperiali and Walkup. However, these methods, which employ monitoring the fluorescence to detect metal coordination, do not provide any information regarding binding of metal coordinated peptides to receptors or targets of interest.

WO 00/36136 A1 discloses protein fragments or analogues of said protein fragments which are complexed with metal ions.

WO 01/13112 A discloses metallopeptides and metallopeptide combinatorial libraries specific for melanocortin receptors, their use in biological, pharmaceutical and related applications.

US-A-6,027,711 discloses metallopeptides comprising the sequence of a peptide of interest such as a ligand of a receptor, wherein the biological function domain specific for the receptor is conformationally constrained upon complexing the metal ion backbone with a metal ion.

### SUMMARY OF THE INVENTION (DISCLOSURE OF THE INVENTION)

In accordance with one aspect of the present invention there is provided a method of determining a secondary structure binding to a target of interest within a known parent polypeptide that binds to the target of interest. The parent polypeptide may be a peptide, a polypeptide or a protein. Such method includes (a) providing a known parent polypeptide that binds to a target of interest with a known primary structure, such primary structure consisting of n residues; (b) constructing a first peptide of the formula R₁-C-R₂; (c) complexing the first peptide of the formula R₁-C-R₂ to a metal ion, thereby forming a first R₁-C-R₂ metallopeptide; (d) screening the first R₁-C-R₂ metallopeptide for binding to the target of interest; (e) repeating steps (b) through (d) as required, wherein the resulting R₁-C-R₂ metallopeptide differs in at least either R₁ or R₂; and (f) selecting the R₁-C-R₂ metallopeptide exhibiting binding to the target of interest, whereby such R₁-C-R₂ metallopeptide comprises the secondary structure binding to the target of interest. In the formula R₁-C-R₂, R₁ includes from 2 to n residues, which residues are the same as or homologues of residues in the parent polypeptide and in the same order as residues in the parent polypeptide primary structure. C is a residue or mimetic thereof providing both an N and an S for metal ion complexation. R₂ includes from 0 to n-2 residues, which residues are the same as or homologues of residues in the parent polypeptide and in the same order as residues in the parent polypeptide primary structure. R₁ and R₂ together form a sequence in the same order as in the parent polypeptide primary structure with C either inserted between two adjacent residues corresponding to two adjacent residues in the primary structure or substituting for a single residue corresponding to a single residue in the primary structure. C may be an L- or D-3-mercapto amino acid, including L- or D-cysteine, L- or D-penicillamine, 3-mercapto phenylalanine, or a homologue of any of the foregoing. In a preferred embodiment, n is at least 3.

The number of residues included in R₁ and R₂ together can be less than n, equal to n or greater than n. For polypeptides where n is at least 15, the method can further include the step of dividing the primary structure into at least three divided primary structures, each such divided primary structure overlapping the primary structure of each adjacent divided primary structure by at least two residues, and thereafter following steps (b) through (f) with respect to each such divided primary structure. The peptides of the formula R₁-C-R₂ can include an N-terminus free amino group or acetyl group and can independently include a C-terminus free carboxylate or amide group.

The metal ion in this and in other methods and constructs of this invention may be an ion of V, Mn, Fe, Co, Ni, Cu, Zn, Ga, As, Se, Y, Mo, Tc, Ru, Rh, Pd, Ag, Cd, In, Sn, W, Re, Os, Ir, Pt, Au, Hg, TI, Pb, Bi, Po, At, Sm, Eu or Gd. The resulting R₁-C-R₂ metallopeptides, and other metallopeptides of this invention, are stable in solution. For some metal ions, the R₁-C-R₂ metallopeptides, and other metallopeptides of this invention, form a stable solid when not in solution. Re is a particularly preferred ion, and forms a stable solid metallopeptide when not in solution.

The target of interest in this method and other methods of this invention may be a receptor, antibody, toxin, enzyme, hormone, nucleic acid, intracellular protein domain of biological relevance or extracellular protein domain of biological relevance.

Any method of screening for binding to the target of interest may be employed. In one embodiment, the method of screening for binding includes competing a known binding partner for binding to the target of interest with the R₁-C-R₂ metallopeptide, such as in a competitive inhibition assay. In such an assay, the parent polypeptide may be utilized as the known binding partner. Alternatively, a peptide derived from the parent polypeptide, which derived peptide binds to the target of interest, may be employed. The method of screening for binding to the target of interest may also include a functional assay. In one embodiment, employed where the target of interest is a biological receptor capable of transmitting a signal, the method of screening includes determining whether the R₁-C-R₂ metallopeptide induces transmission of the signal, and is thus an agonist. In a related embodiment, the method of screening includes determining whether the R₁-C-R₂ metallopeptide inhibits transmission of the signal in the presence of a binding partner to the target of interest known to induce transmission of the signal, and is thus an antagonist.

In this method, R₁ and R₂ can each contain residues that are the same as residues in the parent polypeptide and in the same order as residues in the parent polypeptide primary structure. In an alternative embodiment, one or more residues are substituted with homologues. Thus any cysteine residue in R₁ or R₂ can be substituted with a homologue that does not contain a free sulfhydryl group. Suitable homologues that can be substituted for a cysteine include glycine, alanine, serine, aminoisobutyric acid or dehydroalanine residues. Alternatively, the cysteine can be substituted with an S-protected cysteine, such that the sulfur atom in the cysteine cannot form a complex with the metal ion. In general, the cysteine can be substituted with a neutral mimetic of an amino acid residue of less than about 150 MW. Any proline residue in the two residues immediately adjacent the amino-terminus side of C are preferably substituted, and may be substituted with a glycine, alanine, serine, aminoisobutyric acid or dehydroalanine residue. In general, any proline residue can be substituted with a neutral mimetic of an amino acid of less than about 150 MW that provides an N for metal ion complexation.

The peptides of the formula R₁-C-R₂ are preferably constructed by a chemical method of peptide synthesis. Such methods include solid phase synthesis and solution phase synthesis. In one advantageous embodiment, C can include an orthogonal S-protecting group compatible with the chemical method of peptide synthesis, which orthogonal S-protecting group is characterized by being cleavable at or prior to metal ion complexation. In yet another embodiment the peptides of the formula R₁-C-R₂ are constructed by expression in biological systems, in which embodiment the method can include use of a recombinant vector.

In accordance with another aspect of the present invention there is provided a related but different method of determining a secondary structure binding to a target of interest within a known parent polypeptide that binds to the target of interest. In this method, a parent polypeptide with a known primary structure that binds to a target of interest comprising n amino acid residues is provided, wherein n is at least 3. At least one construct is then made, the construct including at least three elements. One element is an N₁S₁ element with an α-amino group that provides both an N and an S for complexation to a metal ion. The remaining at least two elements each include an α-amino group and an α-carboxyl group and provide an N for complexation to a metal ion. These at least two elements are the same as or homologous with and in the same order as residues in the parent polypeptide with a known primary structure. The at least three elements are joined by peptide bonds and ordered such that the N₁S₁ element is on the carboxyl terminus end of the at least two elements, thereby forming an N₁S₁ element-containing construct. The resulting N₁S₁ element-containing construct is then complexed to a metal ion, thereby forming a metalloconstruct. The metalloconstruct is screened for binding to the target of interest. The foregoing steps are repeated as required, in each instance with the remaining at least two elements including at least one different residue in the parent polypeptide with a known primary structure. The metalloconstruct exhibiting the highest binding to the target of interest is then selected. In this method, the N₁S₁ element can optionally be the carboxyl terminal end element of the construct. In one embodiment, the N₁S₁ element-containing construct includes at least four elements, the four elements consisting of an N₁S₁ element with an a-amino group and the remaining at least three elements including an α-amino group and an α-carboxyl group, the remaining at least three elements being the same as or homologous with and in the same order as residues in the parent polypeptide with a known primary structure. In this embodiment, the N₁S₁ element is on the carboxyl terminus end of any two of the at least three elements, and the at least four elements are joined by peptide bonds. In yet another embodiment of this method, the at least two elements are amino acid residues, and optionally such amino acid residues are alanine, aspartic acid, glutamic acid, phenylalanine, glycine, histidine, isoleucine, lysine, leucine, methionine, asparagine, methionine, proline, glutamine, arginine, serine, threonine, valine, tryptophan or tyrosine. The amino acid residues may be L-amino acid residues, D-amino acid residues, a combination of L-amino acid residues and D-amino acid residues, or any modified protein amino acid residues, non-protein amino acid residues, mimetics of non-protein amino acid residues, mimetics of protein amino acid residues, post-translationally modified amino acid residues, or enzymatically modified amino acid residues. The number of elements in a construct of this method may be less than n, equal to n, equal to n + 1 or greater.

In accordance with another aspect of this invention there is provided a method of determining a metallopeptide that binds to a target of interest. In this method a known amino acid sequence with a known primary structure of n residues, where n is at least 3, is selected, which known amino acid sequence binds to the target of interest. A library of amino acid sequences is then designed by selecting at least two consecutive residues from a stretch of consecutive residues in the known primary structure and inserting a residue providing both an N and S for metal ion complexation on the carboxy terminal end of two of the at least two selected consecutive residues. Each such designed sequence constitutes a library member. Each library member differs by at least one residue or the location of the insertion of the residue providing both an N and S for metal ion complexation. The library of designed amino acid sequences is then constructed, using any method of peptide synthesis, and each library member of designed amino acid sequences is complexed to a metal ion, thereby forming a library of metallopeptides. Each member of the library of metallopeptides is then screened for binding to the target of interest, and a metallopeptide exhibiting binding to the target of interest is selected. In a related embodiment, at least one residue of the selected at least two consecutive residues is a homologue of the corresponding residue in the stretch of consecutive residues in the known primary structure.

In accordance with another aspect of this invention another method of determining a metallopeptide that binds to a target of interest is provided. In this method a known amino acid sequence with a known primary structure of n residues, where n is at least 4, is selected, which known amino acid sequence binds to the target of interest. A library of amino acid sequences is then designed by selecting at least three consecutive residues from a stretch of consecutive residues in the known primary structure and substituting a residue providing both an N and S for metal ion complexation for the carboxy terminal residue of any consecutive stretch of three of the at least three selected consecutive residues. If the selected at least three consecutive residues are more than three residues, then the residue providing both an N and S for metal ion complexation need not be the carboxy terminal residue of resulting amino acid sequence, so long as the residue is substituted for the carboxy terminal residue in any group of three consecutive residues. Each sequence constitutes a library member, the library members being characterized in that each differs by at least one residue from any other library member. The library is then constructed and the library members complexed to a metal ion, thereby forming a library of metallopeptides. Each member of the library is then screened for binding to the target of interest and a metallopeptide exhibiting binding to the target of interest is selected. In a related embodiment, at least one residue of the selected at least two consecutive residues is a homologue of the corresponding residue in the stretch of consecutive residues in the known primary structure.

In accordance with yet another embodiment of this invention a method of determining a target-specific binding pharmacophore for a target of interest is provided. In this method, a metallopeptide binding to a target of interest is selected by means of any of the forgoing methods. Utilizing the selected metallopeptide, the spatial position of amino acid side chains in and immediately adjacent the metal ion coordination site is determined by building a molecular model based on the coordination geometry of the metal ion. This thus defines a target-specific binding pharmacophore for the target of interest. This method can optionally further include optimizing binding of the selected metallopeptide to the target of interest by changing the chirality of one or more of the amino acid residues complexed to the metal ion, or amino acid residues adjacent to the amino acid residues complexed to the metal ion. This method can also optionally further include optimizing binding of the selected metallopeptide to the target of interest by substituting a natural or synthetic homologue for at least one amino acid residue complexed to the metal ion, or at least one amino acid residue adjacent to the amino acid residues complexed to the metal ion. After such optimization, if the optimized metallopeptide provides improved binding to the target of interest, then the optimized metallopeptide is utilized for building a molecular model based on the coordination geometry of the metal ion. In this method, computer-based modeling can be employed to build the molecular model. This molecular model, and hence the pharmacophore, may include the location in a three-dimensional model of hydrogen bond donors and acceptors, positively and negatively charged centers, aromatic ring centers, hydrophobic centers and the like. In a preferred embodiment, the resulting pharmacophore is described in terms of spatial location of atoms in the pharmacophore and the distances between the atoms in the pharmacophore

In accordance with yet another embodiment of this invention a target-specific binding pharmacophore for a target of interest is provided. The pharmacophore is defined by a metallopeptide, selected such that the metallopeptide binds to the target of interest. The metallopeptide includes a residue providing both an N and an S for metal ion complexation and, joined by a peptide bond to the amino-terminus side of such residue, at least two consecutive residues that are the same as or homologues of the same number of consecutive residues of the primary structure of a known sequence of amino acid residues that binds to the target of interest. A metal ion is complexed to the residues. Any proline residue in the two residues immediately adjacent the amino-terminus side of the residue providing both an N and an S for metal ion complexation is substituted with a residue providing an N for metal ion complexation. Any residue with a free sulfhydryl group, other than the residue providing both an N and an S for metal ion complexation, is substituted with a homologue not containing a free sulfhydryl group. The pharmacophore thus provided may be further defined by the spatial position of amino acid side chains in and immediately adjacent the metal ion coordination site, such as by use of a molecular model based on the coordination geometry of the metal ion.

In accordance with yet another embodiment of this invention a library of metallopeptides targeted to a target of interest is provided. Each constituent library member includes an amino acid sequence of the formula R₁-C-R₂, defined as set forth above, with a metal ion complexed to each library member. Representative libraries, as set forth in the examples contained herein, include libraries of metallopeptides targeted to the urokinase-type plasminogen activator receptor, melanocortin receptors, vasopressin receptor, oxytocin receptor or angiotensin receptor, or libraries constituting amyloid beta-protein related peptides for treatment of Alzheimer's disease or peptides for treatment of prion disease.

It is a primary object of this invention to provide conformationally-constrained metallopeptides as surrogates for naturally-occurring structural motifs, such as those motifs commonly found in naturally-occurring peptides and proteins, including reverse turn structures, type I, II and III beta turns, gamma turns, inverse gamma turns, and short helical, sheet and extended chain structures. A secondary structural motif is necessarily defined by a conformationally-constrained metallopeptide, which secondary structural motif mimics, or can be made to mimic, the topologies found in naturally occurring structural motifs. The secondary structural motif formed as a consequence of metal ion complexation in the metallopeptide is more stable than the naturally occurring secondary structural motifs, which are generally stabilized only by weaker interactions such as van der Waals' interactions and hydrogen bonds.

Another object of this invention is to provide backbone structures of turns formed upon complexation of a metal ion to an amino acid sequence including an N₁S₁ residue, forming a secondary structural motif with substantial topological similarities to classical protein turn structures. Amino acid side chains associated with the metal ion-induced turn can be topographically positioned such that they occupy the same chemical space as the corresponding side chains in classical turn structures.

Another object of this invention is to provide libraries of metallopeptides based upon a known amino acid sequence that exhibits binding to a target or receptor of interest, wherein the peptides include a metal ion-complexing domain, such that a specific conformational structure providing a secondary structural motif is obtained upon metal complexation.

Another object of this invention is to provide metallopeptide sequences, wherein the metallopeptides include a metal ion-complexing domain, such that a specific conformational secondary structural motif is obtained upon metal complexation.

Another object of this invention is to provide metallopeptide sequences, wherein the metallopeptides include a metal ion-complexing domain in a distinct and known location within the sequence, wherein the metallopeptides may be exposed to a substance and one or more metallopeptides will exhibit specificity and affinity for the substance.

Another object of this invention is to provide a method for identifying the specific residues within a known peptide that are involved in binding to a known target of interest.

Another object of this invention is to provide methods for synthesis of peptides wherein the peptides contain a single reactive -SH group forming a part of a metal ion-complexing domain, whereby the reactive -SH group is protected during synthesis, and is deprotected only upon complexing the peptide with a metal ion.

Another object of this invention is to provide a method for making metallopeptides as models for the active binding site in a known parent polypeptide, wherein each endogenous cysteine residue is substituted or, alternatively, wherein each endogenous cysteine residue further includes an S-protecting group, such that the sulfur of such endogenous cysteine does not form a part of a metal ion-complexing domain.

Another object of this invention is to provide libraries of peptides wherein each of the peptides forming the library contains a secondary structural motif upon complexation with metal ion, thereby forming a metallopeptide.

Another object of this invention is to provide libraries containing metallopeptides with high specificity and affinity for a target molecule of interest, such high specificity and affinity resulting from each of the metallopeptides forming the library containing a secondary structural motif as a consequence of metal ion complexation.

Another object of this invention is to provide a method for rapid and efficient complexation of a pool of diverse peptides with a metal ion, including a rhenium metal ion.

Other objects, advantages and novel features, and further scope of applicability of the present invention will be set forth in part in the detailed description to follow, taken in conjunction with the accompanying drawings, and in part will become apparent to those skilled in the art upon examination of the following, or may be learned by practice of the invention. The objects and advantages of the invention may be realized and attained by means of the instrumentalities and combinations particularly pointed out in the appended claims.

### BRIEF DESCRIPTION OF THE DRAWINGS

The accompanying drawings, which are incorporated into and form a part of the specification, illustrate one or more embodiments of the present invention and, together with the description, serve to explain the principles of the invention. The drawings are only for the purpose of illustrating one or more preferred embodiments of the invention and are not to be construed as limiting the invention. In the drawings:

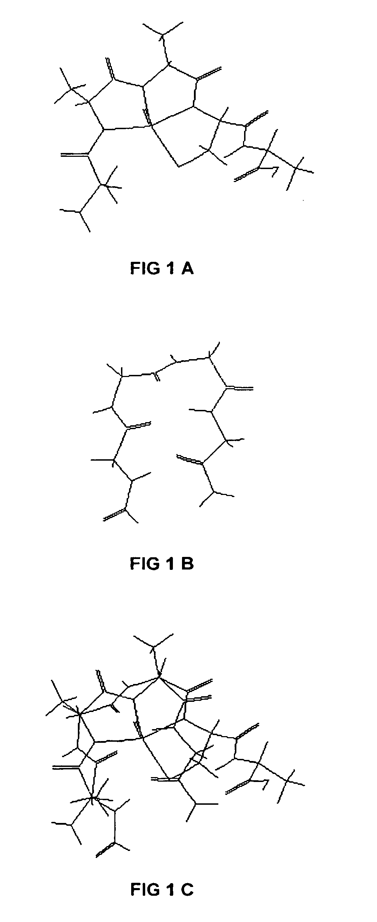

**Fig. 1 A** is backbone diagram of the peptide sequence Ala-Ala-Ala-Cys-Ala (SEQ ID NO:1) complexed to a rhenium metal ion, with the Ala-Ala-Cys sequence forming the metal ion binding domain, thereby forming a metallopeptide;
**Fig. 1 B** is a backbone peptide coordinate diagram of a classical beta-II' turn;
**Fig. 1 C** is the diagram of **Fig. 1 A** superimposed on the diagram of **Fig. 1 B**, aligned at the respective C-α carbon atoms of the three consecutive N-terminal amino acid residues. Comparison of the superimposed structures demonstrates excellent overlap at the three C-α carbon atom positions, with a calculated root mean square deviation (RMSD) per atom of < 0.05 Å. The metal ion located in the center of the turn of the diagram of **Fig. 1 A** corresponds to the hydrogen bond that stabilizes the natural beta turn structure of **Fig. 1 C**. In this representation, three C-β carbon atoms of the metallopeptide are pointed in directions other than those in natural beta-turn structure, thereby providing access to additional chemical space. The C-terminal end of the metallopeptide further provides access to new chemical space;

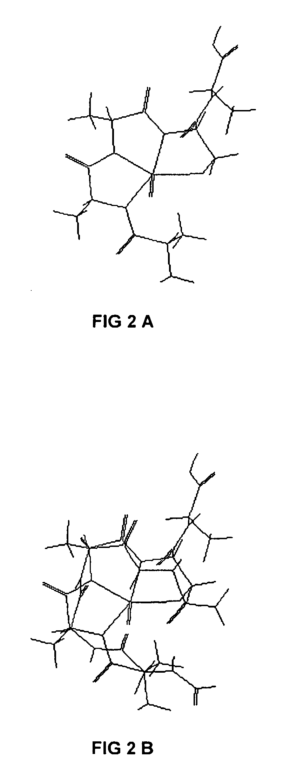

**Fig. 2 A** is a backbone diagram of the peptide sequence Ala-Ala-Ala-D-Cys-Ala complexed to a rhenium metal ion, with the Ala-Ala-D-Cys sequence forming the metal ion binding domain, thereby forming a metallopeptide;
**Fig. 2 B** is the diagram of **Fig. 2** **A** superimposed on the diagram of **Fig. 1** **B** in a manner similar to that depicted in **Fig. 1** **C**. A comparison of these structures reveals excellent overlap at three C-α carbon atoms of the three consecutive N-terminal amino acid residues, similarly with an overlap of RMSD < 0.05 Å. The metal ion located in the center of the turn similarly corresponds to the hydrogen bond that stabilizes the natural beta turn structure. In this representation, C-β carbon atoms of the metallopeptide are pointed in directions other than those in natural beta-turn structure, thereby providing access to additional chemical space. The C-terminal end of the metallopeptide further provides access to a new chemical space, which space is different then that addressable by metallopeptide in **Fig. 1** **A**;

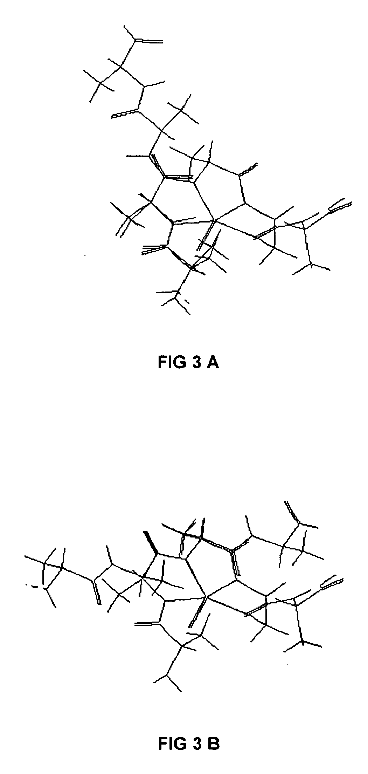

**Fig. 3** **A** is a backbone diagram of the peptide sequence Ala-Ala-Ala-D-Cys-Ala complexed to a rhenium metal ion superimposed on an extended chain peptide structure. In this depiction, C-α atoms of two consecutive amino acid residues of extended chain structure are overlapped onto the C₁-α and C₂-α atoms of the metallopeptide sequence. The superimposition further illustrates positioning of these two amino acid residues, including their C-β carbon atoms, in approximately similar chemical juxtaposition;
**Fig. 3** **B** is a backbone diagram of the peptide sequence Ala-Ala-Ala-D-Cys-Ala complexed to a rhenium metal ion superimposed on an extended chain peptide structure. In this depiction, C-α atoms of two consecutive amino acid residues of the extended chain structure are overlapped onto C₂-α and C₃-α atoms of the metallopeptide sequence. The superimposition illustrates exact positioning of C-α carbon atoms of these two amino acid residues as well as the C₃-β carbon atom, while allowing access to a different chemical space at the C₂-β carbon atom;

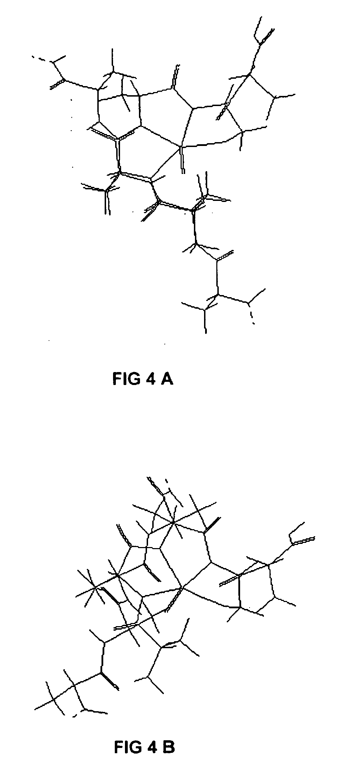

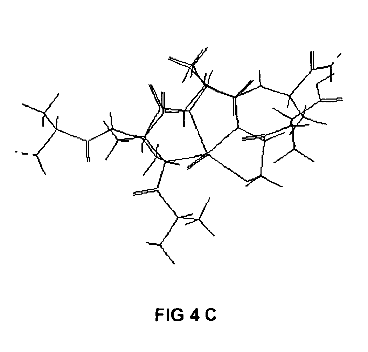

**Fig. 4** **A** is a backbone diagram of the peptide sequence Ala-Ala-Ala-D-Cys-Ala complexed to a rhenium metal ion superimposed on a β-sheet peptide structure. In this depiction C-α atoms of two consecutive amino acid residues of the β-sheet structure are overlapped onto the C₁-α and C₂-α atoms of the metallopeptide sequence. The superimposition illustrates these two amino acid residues along with their C-β carbon atoms in similar chemical juxtaposition;
**Fig. 4** **B** is a backbone diagram of the peptide sequence Ala-Ala-Ala-D-Cys-Ala complexed to a rhenium metal ion superimposed on a β-sheet peptide structure. In this depiction C-α atoms of two consecutive amino acid residues of the β-sheet structure are overlapped onto the C₂-α and C₃-α atoms of the metallopeptide sequence. The superimposition illustrates exact positioning of C-α carbon atoms of these two amino acid residues while allowing access to a different chemical space at the C₂-β and C₃-β carbon atoms;
**Fig. 4** **C** is a backbone diagram of the peptide sequence Ala-Ala-Ala-D-Cys-Ala complexed to a rhenium metal ion superimposed on a β-sheet peptide structure. In this depiction C-α atoms of two consecutive amino acid residues of the β-sheet structure are overlapped onto C₂-α and C₃-α atoms of the metallopeptide sequence in a manner different then that in **Fig. 4** **B**. This superimposition orientation suggests positioning of these two amino acid residues along with C₃-β carbon atoms in similar chemical juxtaposition and allowing for accessing alternate chemical space at C₂-β atom;

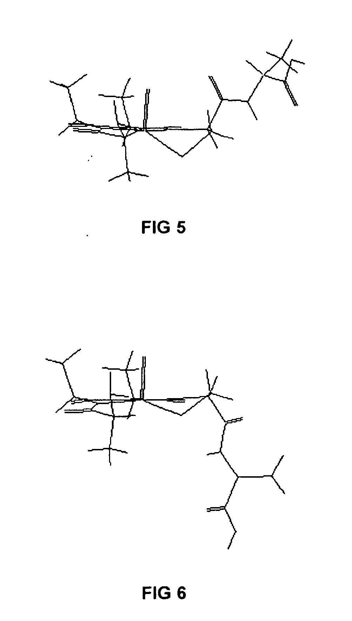

**Fig. 5** is a backbone diagram of the peptide sequence Ala-Ala-Ala-D-Cys-Ala complexed to a rhenium metal ion when viewed along the plane passing through the C-α carbons of amino acid residues 1, 2, 3, and D-Cys. The diagram depicts helicity in the metallopeptide with respect to amino acid positions 1 and 5. This i to l + 5 residue pitch of the metallopeptide can be matched topographically to the i and l + 4 residues chemical space in an a-helix;
**Fig. 6** is a backbone diagram of the peptide sequence Ala-Ala-Ala-Cys-Ala (SEQ ID NO:1) complexed to a rhenium metal ion when viewed along the plane passing through the C-α carbons of amino acid residues 1, 2, 3, and Cys. The diagram similarly depicts helicity in the metallopeptide with respect to amino acid positions 1 and 5.

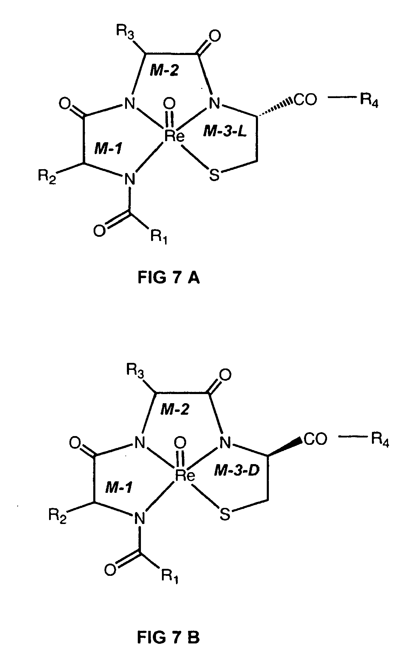

**Fig. 7** **A** illustrates the structure of a conceptualized L-Cys metallopeptide complexed to Re, wherein M-1 and M-2 are two amino acid residues involved in metal complexation along with the L-Cys (M-3-L) residue;
**Fig. 7** **B** illustrates the structure of a conceptualized D-Cys metallopeptide complexed to Re, wherein M-1 and M-2 are two amino acid residues involved in metal complexation along with the D-Cys (M-3-D) residue;

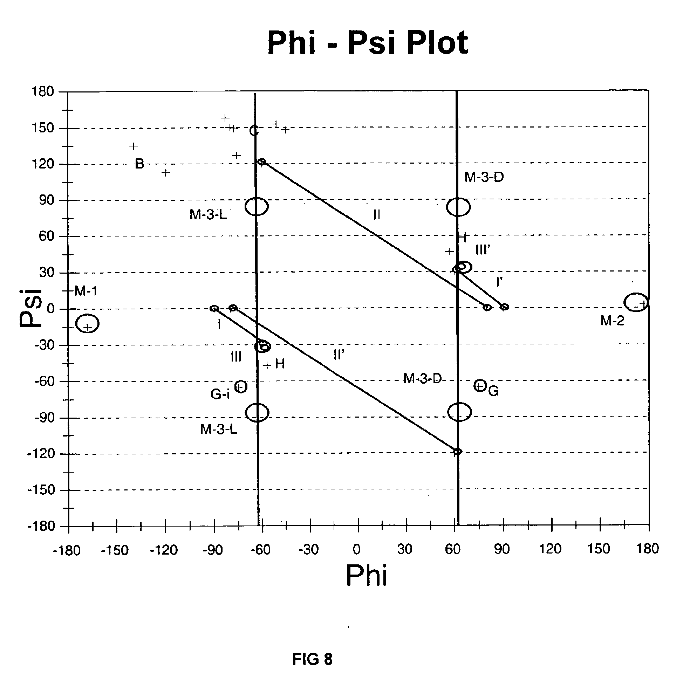

**Fig. 8** is a phi-psi (Ramachandran) plot of the metallopeptides of **Fig. 7** **A** and **7 B** showing coordinates (structural propensity) for M-1, M-2, M-3-L, and M-3-D residues. Also included in the plot are the regions of natural protein structures such as an α-helix (H), β-sheet (B), collagen helix (C), gamma turn (G), inverse gamma turn (G-i), type-1-beta turn (I), type-1'-beta-turn (I'), type-II-beta-turn (II), type-II'-beta-turn (II'), type-III-beta-turn (III) and type-III'-beta-turn (III'). A dashed line defining an amino acid pair (i+1 and i+2 residues) of a turn structure is shown. The H with negative phi and psi values is for a natural right handed helix, while the other H with positive phi, psi values represents a left handed helix. The M-1 and M-2 residues reside near the 0°, 180° or 0°, -180° coordinates. Both positions indicate that these amino acid residues in these metallopeptides represent a structure different then any of the natural protein structures. The phi angle in L-Cys (M-3-L) or D-Cys (M-3-D) is fixed at approximately -63° and +63° respectively (two solid vertical lines). Based on the psi value of Cys residues, M-3-L and M-3-D would lie somewhere on these two vertical lines. However, due to the restricted orientation of the carbonyl (CO) group of either Cys residue, the psi angle would range from 60° to 90° or -60° to -90° for L- and D-Cys, respectively. Under these conditions it is evident from the Ramachandran plot that the conformational characteristics at Cys fall close to a right-handed helix region for L-Cys and a left-handed helix region for D-Cys. This conclusion accords with the depictions of **Fig. 5** and **6**;
**Fig. 9** A illustrates the structure of an L-Cys metallopeptide complexed to Re of the primary structure des-aminoPhe-D-Asp-L-HomoSer-L-Cys-Trp-amide;

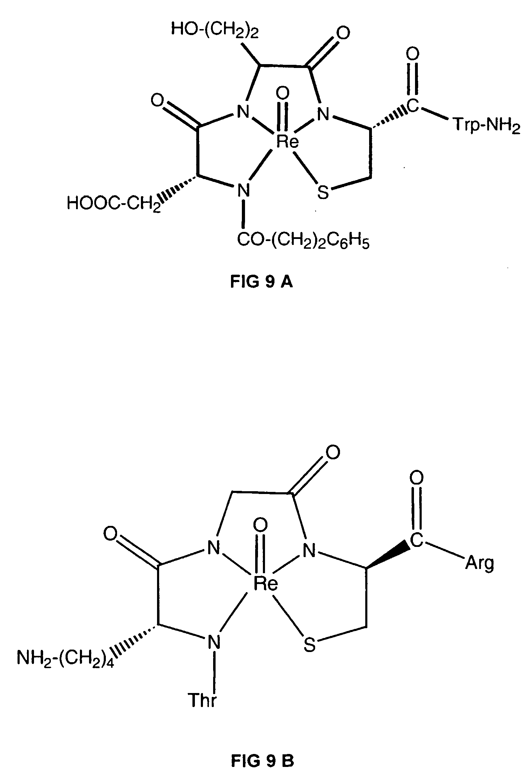

**Fig. 9** **B** illustrates the structure of a D-Cys metallopeptide complexed to Re of the primary Thr-D-Lys-Gly-D-Cys-Arg;

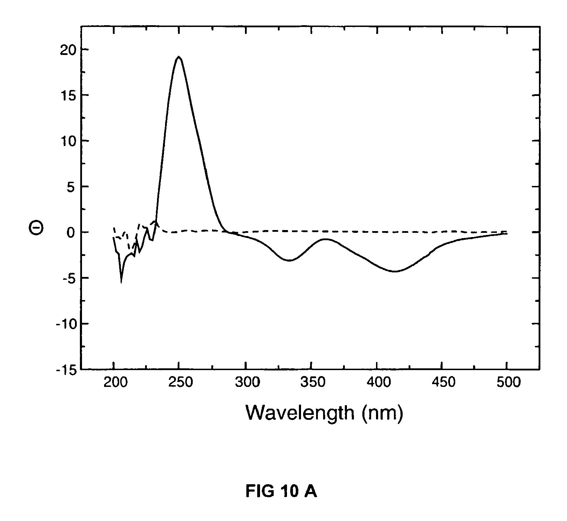

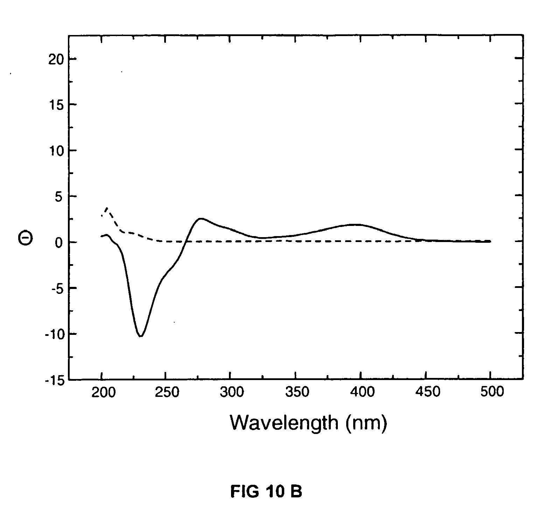

**Fig. 10** **A** is a circular dichroism (CD) spectra plot of the metallopeptide of **Fig. 9** **A** (shown as the solid line) compared to the peptide of the structure of **Fig. 9** **A** when not complexed to a metal ion (shown as the dashed line), wherein the x-plot is wavelength (nm) and the y-plot is the mean molar ellipticity Θ of the sample per residue x 10⁻³ (degrees • cm²/decimol). The CD spectrum of the linear peptide (dashed line) shows no organized structure (zero ellipticity), whereas the CD spectrum for the Re-complexed peptide (solid line) is characteristic of ordered structure;
**Fig. 10** **B** is a circular dichroism (CD) spectra plot of the metallopeptide of **Fig. 9** **B** (shown as the solid line) compared to the peptide of the structure of **Fig. 9** **B** when not complexed to a metal ion (shown as the dashed line), wherein the x-plot is wavelength (nm) and the y-plot is the mean molar ellipticity Θ of the sample per residue x 10⁻³ (degrees • cm²/decimol). The CD spectrum of the linear peptide (dashed line) shows no organized structure (zero ellipticity), whereas the CD spectrum for the Re-complexed peptide (solid line) is characteristic of ordered structure; and

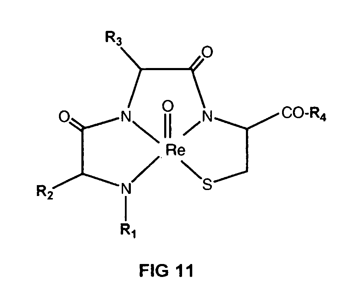

**Fig. 11** is a generic structure of both the urokinase-type tissue plasminogen activator metallopeptide template of Example 1 and the melanocortin metallopeptide template of Example 2.

### DESCRIPTION OF THE PREFERRED EMBODIMENTS

### (BEST MODES FOR CARRYING OUT THE INVENTION)

Certain terms as used throughout the specification and claims are defined as follows:

The terms "bind," "binding," "label," "labeling," "complex," and "complexing," as used throughout the specification and claims are generally intended to cover all types of physical and chemical binding, reactions, complexing, attraction, chelating and the like.

The "polypeptides" and "peptides" of this invention can be a) naturally-occurring, b) produced by chemical synthesis, c) produced by recombinant DNA technology, d) produced by biochemical or enzymatic fragmentation of larger molecules, e) produced by methods resulting from a combination of methods a through d listed above, or f) produced by any other means for producing polypeptides or peptides.

The term "polypeptide" as used throughout the specification and claims is intended to include any structure comprised of two or more amino acid residues, including chemical modifications and derivatives of amino acid residues. The term "polypeptides" thus includes a conventional "peptide" containing from two to about 20 amino acid residues, a conventional polypeptide with from about 20 to about 50 amino acid residues, and a conventional "protein" with a minimum of about fifty 50 amino acid residues. For the most part, the polypeptides made according to this invention and utilized as metallopeptides comprise fewer than 100 amino acid residues, and preferably fewer than 60 amino acid residues, and most preferably ranging from about 3 to 20 amino acid residues. The amino acid residues forming all or a part of a polypeptide may be naturally occurring amino acid residues, stereoisomers and modifications of such amino acid residues, non-protein amino acid residues, post-translationally modified amino acid residues, enzymatically modified amino acid residues, constructs or structures designed to mimic amino acid residues, and the like, so that the term "polypeptide" includes pseudopeptides and peptidomimetics, including structures which have a non-peptidic backbone. A "manufactured" peptide or polypeptide includes a peptide or polypeptide produced by chemical synthesis, recombinant DNA technology, biochemical or enzymatic fragmentation of larger molecules, combinations of the foregoing or, in general, made by any other method.

The "amino acid residues" used in this invention, and the term as used in the specification and claims, include the known naturally occurring coded protein amino acid residues, which are referred to by both their common three letter abbreviation and single letter abbreviation. See *generally* Synthetic Peptides: A User's Guide, GA Grant, editor, W.H. Freeman & Co., New York, 1992, the teachings of which are incorporated herein by reference, including the text and table set forth at pages 11 through 24. As set forth above, the term "amino acid residue" also includes stereoisomers and modifications of naturally occurring protein amino acid residues, non-protein amino acid residues, post-translationally modified amino acid residues, enzymatically synthesized amino acid residues, derivatized amino acid residues, constructs or structures designed to mimic amino acid residues, and the like. Modified and unusual amino acid residues are described generally in Synthetic Peptides: A User's Guide, cited above; Hruby VJ, Al-obeidi F and Kazmierski W: Biochem J 268:249-262, 1990; and Toniolo C: Int J Peptide Protein Res 35:287-300, 1990; the teachings of all of which are incorporated herein by reference. A single amino acid residue, or a derivative thereof, is sometimes referred to herein as a "residue" or as an "amino acid."

The constructs of this invention also include a meta! ion, which may be an ionic form of any element in metallic form, including but not limited to metals and metalloids. The metal ion may, but need not, be radioactive, paramagnetic or superparamagnetic. The metal ion can be of any oxidation state of any metal, including oxidation states of vanadium (V), manganese (Mn), iron (Fe), cobalt (Co), nickel (Ni), copper (Cu), zinc (Zn), gallium (Ga), arsenic (As), selenium (Se), yttrium (Y), molybdenum (Mo), technetium (Tc), ruthenium (Ru), rhodium (Rh), palladium (Pd), silver (Ag), cadmium (Cd), indium (In), tin (Sn), tungsten (W), rhenium (Re), osmium (Os), iridium (Ir), platinum (Pt), gold (Au), mercury (Hg), thallium (TI), lead (Pb), bismuth (Bi), polonium (Po), astatine (At), samarium (Sm), europium (Eu), and gadolinium (Gd). The metal ion can also be a radionuclide of any of the foregoing, including In, Au, Ag, Hg, Tc, Re, Sn, At, Y and Cu. A preferred metal ion with a tetradentate coordination sphere is Re. For applications wherein a radioisotope is desirable for screening or in assay systems, an alpha-, gamma- or beta-emitting radionuclide may be employed.

In one embodiment, the method of the invention provides for the systematic analysis of a parent polypeptide to determine at least one active sequence or domain in the parent polypeptide that is involved in the interaction, such as binding, of the parent polypeptide with a target substance. As used herein, "parent polypeptide" refers to any sequence of amino acid residues that exhibits interaction, such as binding, to a target substance, and which may thus constitute a peptide, a polypeptide or a protein. The parent polypeptide is generally a polypeptide as defined herein, with from about 3 to about 100 amino acid residues, but the term parent polypeptide can also include larger constructs, generally considered in the art to be large polypeptides or proteins. To employ the method of the invention, the primary structure, which is to say the sequence, of at least part, and preferably of all, of the parent polypeptide must be known. However, it is not necessary to have any information concerning the secondary or tertiary structure of the parent polypeptide in order to practice the method of the invention.

The parent polypeptide may be any sequence that exhibits binding to a receptor found on, for example, cells, tissues, organs or other biological materials. Examples of parent polypeptides include, without limitation, biologically active peptides, hormones, neurotransmitters, enzymes, antibodies and the like. Such parent polypeptides may transmit signals, directly or indirectly, as a result of binding to a receptor, and thus a parent polypeptide may be an agonist, an antagonist, or a mixed agonist-antagonist. Examples of suitable parent polypeptides of the invention include melanocortin-receptor specific peptides, urokinase-type tissue plasminogen activator protein, amyloid beta-protein related peptides, prion disease related peptides, vasopressin peptides, oxytocin peptides, angiotensin peptides, calcitonin, calcitonin gene related peptide, opioid peptides, human growth hormone, human prolactin receptor ligands, various interferons, such as alpha-interferon, epidermal growth factor, tumor necrosis factor, and various hypotensive peptides, fibrinolytic peptides, chemotactic peptides, growth promoter peptides, mitogens, immunomodulators and the like.

In general, in order to employ the invention at least one assay or test to determine binding of the constructs of the invention to a receptor of interest, and preferably to also determine binding of the parent polypeptide to a receptor of interest, must be known. In a preferred embodiment of the invention, a competitive inhibition or similar assay is employed, whereby the binding or functional activity of a construct of the invention can be directly compared to the parent polypeptide, and relative binding or functional activity thus directly determined. In other embodiment other assays or tests may be employed. These assays may, but need not, be functional assays. Examples of assays include any of a variety of competitive inhibition assays, direct binding assays, functional assays, and the like. It is also possible and contemplated to employ assays that determine, for example, whether a construct of the invention is an agonist, antagonist or mixed agonist-antagonist, and further where binding and function can separately be determined, to independently determine both receptor affinity and specificity as well as functional activity. Examples of such assays and tests are well known and well documented in the art, and in general one or more such assays or tests are known for any parent polypeptide.

In a method of the invention, the parent polypeptide is employed as the template for generation of one or more, and preferably of a series, of peptides that are then complexed to a metal ion. In general, but not necessarily, the generated peptides are of shorter length than the parent polypeptide. However, it is possible and contemplated for the generated peptide to have a primary structure either as long as or longer than that of the parent polypeptide. The generated peptide, of whatever length, is complexed to a metal ion, thereby forming a metallopeptide. The metallopeptide is then employed in any of a variety of known or new assays or tests, and the binding or function, or both, of the metallopeptide compared to that of the parent polypeptide.

The coordination sphere of various common metal ions, in general, is tetradentate to hexadentate. In one embodiment according to this invention, residues are included within each generated peptide such that the peptide contains the desired number of groups (4 to 6 in most cases) for complexing with the metal. As a result, upon complexing with a metal, the resulting metallopeptide forms a secondary structural motif about the site of metal complexation. A metal with coordination number 4, 5 or 6, and complexing respectively with an amino acid sequence forming a tetra, penta, or hexadentate ligand, will fold and constrain the ligand. The amino acid or amino acid mimetic sequence forming a ligand is defined as the metal ion-complexing domain ("MCD") of the peptide or peptidomimetic. A highly flexible molecule like a peptide, in other words, is folded to form a secondary structural motif upon its complexation with a metal ion. This resulting motif is a highly constrained structure in the conformational sense.

A binding domain ("BD") of the metallopeptide is defined in the specification and claims as a sequence of two or more amino acid residues which constitute a biologically active sequence, exhibiting binding to a receptor found on cells, tissues, organs and other biological materials, thereby constituting the metallopeptide as a member of a specific binding pair. In preferred embodiments of this invention, the BD of a metallopeptide of this invention includes at least a portion of the MCD, and may, but need not, be co-extensive with the MCD. In preferred embodiments of this invention the sequence of amino acid residues constituting the BD are thus also all or a part of the sequence of amino acid residues constituting, together with the metal ion, a secondary structural motif. The BD also includes any sequence, which may be consecutive amino acid residues or mimetics (sychnological) or non-consecutive amino acid residues or mimetics (rhegnylogical) which forms a ligand, which ligand is capable of forming a specific interaction with its acceptor or receptor. The term "receptor" is intended to include both acceptors and receptors. The receptor may be a biological receptor. The sequence or BD may transmit a signal to the cells, tissues or other materials associated with the biological receptor after binding, but such is not required. Examples include, but are not limited to, BDs specific for hormone receptors, neurotransmitter receptors, cell surface receptors, enzyme receptors and antibody-antigen systems. The BD may be either an agonist or antagonist, or a mixed agonist-antagonist. The BD may also include any ligand for site-specific RNA or DNA binding, such as sequences that may be employed as mimics of transcription and other gene regulatory proteins. The BD may also include any sequence of one or more amino acid residues or mimetics, or other constrained molecular regions, which exhibit binding to a biological receptor found on other peptides, on enzymes, antibodies, or other compositions, including proteinaceous compositions, which may themselves exhibit binding to another biological receptor. A peptide or peptidomimetic complexed to a metal ion with such a BD constitutes a member of a "specific binding pair," which specific binding pair is made up of at least two different molecules, where one molecule has an area on the surface or in a cavity which specifically binds to a particular spatial and polar organization of the other molecule. Frequently, the members of a specific binding pair are referred to as ligand and receptor or anti-ligand. Examples of specific binding pairs include antibody-antigen pairs, hormone-receptor pairs, peptide-receptor pairs, enzyme-receptor pairs, carbohydrate-protein pairs (glycoproteins), carbohydrate-fat pairs (glycolipids), lectin-carbohydrate pairs and the like.

Conformational constraint refers to the stability and preferred conformation of the three-dimensional shape assumed by a peptide or other construct. Conformational constraints include local constraints, involving restricting the conformational mobility of a single residue in a peptide; regional constraints, involving restricting the conformational mobility of a group of residues, which residues may form some secondary structural unit; and global constraints, involving the entire peptide structure. *See generally* Synthetic Peptides: A User's Guide, cited above.

The primary structure of a peptide is its amino acid sequence. The secondary structure deals with the conformation of the peptide backbone and the folding up of the segments of the peptide into regular structures such as α-helices, β-bends, turns, extended chains and the like. For example, the local three-dimensional shape assumed by an amino acid sequence complexed to a metal ion forms a secondary structure, here called a secondary structural motif. *See generally* Synthetic Peptides: A User's Guide, cited above, including the text, figures and tables set forth at pages 24-33, 39-41 and 58-67. A global or tertiary structure refers to a peptide structure that exhibits a preference for adopting a conformationally constrained three-dimensional shape.

The products resulting from the methods set forth herein can be used for both medical applications and veterinary applications. Typically, the product is used in humans, but may also be used in other mammals. The term "patient" is intended to denote a mammalian individual, and is so used throughout the specification and in the claims. The primary applications of this invention involve human patients, but this invention may be applied to laboratory, farm, zoo, wildlife, pet, sport or other animals. The products of this invention may optionally employ radionuclide ions, which may be used for diagnostic imaging purposes or for radiotherapeutic purposes.

In one preferred embodiment of the invention, the regional secondary structure of that portion of a peptide, polypeptide, or protein, and in general any molecule or molecular structure incorporating amino acid residues or mimetics thereof, binding to any receptor or target of interest is defined by means of the methods and constructs hereafter provided. In a related preferred embodiment, the pharmacophore of a receptor or target of interest, for which there is a known peptide, polypeptide, or protein, or in general any molecule or molecular structure incorporating amino acid residues or mimetics thereof that binds thereto, is defined by means of the methods and constructs hereafter provided.

The present invention employs to advantage the unique structures and characteristics of metallopeptides and similar metalloconstructs formed by complexing a metal ion to two or more amino acid residues, and in a preferred embodiment, to three amino acid residues. For most metal ions, including for example ions of Re, Tc, Cu, Ni, Au, Ag, Sn and Hg, a complex to an MCD including an available sulfur atom (S) is preferred. That is, metal ions, provided that such ions are in the appropriate and desired oxidation state for complexing, will preferably complex to a tri-peptide MCD sequence including a residue with an S available for complexing, and most preferably a residue including both an S and a nitrogen atom (N) available for complexing, in preference to tri-peptide sequences wherein no S is available for complexing.

It may thus be seen that in any amino acid sequence of length n, where n is at least 3, metal ions in the appropriate and desired oxidation state will preferentially complex to a tri-peptide sequence X-X-Cys, forming the MCD, where each X is independently any natural amino acid residue other than Pro or Cys, and further provided that the only Cys present in the amino acid sequence of length n is the Cys in X-Y-Cys. That is, the dynamics of the metal complexation reaction is such that the preferred resulting metallopeptide includes, for a tetradenate metal ion, an N₃S₁ ligand, formed of the tri-peptide sequence X-Y-Cys. With more than one Cys residue, or mimetic or variation of a Cys residue providing both an N and S, the structure of the resulting metallopeptide is difficult to predict, and a variety of species of metallopeptides may result from complexing with a metal ion. For example, an amino acid sequence of length n containing two Cys residues may cross-link, dimerize, polymerize, form internal disulfide bridges and the like. In terms of metal complexation, there may, depending on the primary structure of the sequence, be two different MCDs, such that some molecules will have a metal ion bound to the first MCD, others to the second MCD and still others to both MCDs. Thus the structure of a resulting metallopeptide cannot be predicted, and must be empirically determined. Similarly, it is also possible, again depending on the primary structure of the sequence, that one or more MCDs in the sequence will provide N₃S₁ ligands, while at least one other MCD in the sequence will provide an N₂S₂ ligand. Here too the structure of the resulting metallopeptide cannot be predicted, and must be empirically determined.

The present invention encompasses a method for defining the secondary structure of a region of a peptide, polypeptide, or protein, or in general any molecule or molecular structure incorporating amino acid residues or mimetics thereof, that binds to any receptor or target of interest. This is accomplished by substitution or insertion of a Cys residue, or other residue, mimetic, or homologue providing both an N and S for complexation to the coordination sphere of a metal ion (an "N₁S₁ residue"), at various positions along the molecule, complexing a metal ion thereto to form a metallopeptide, and testing the resulting metallopeptide for binding to the receptor or target of interest. In one embodiment of the invention, the primary structure of a parent polypeptide, such as a peptide, polypeptide or protein binding to a receptor or target of interest, is known. Such parent polypeptide is composed of some specific number of residues, referred to as "n" residues. A series of peptides of the formula R₁-C-R₂ is made, wherein R₁ is from 2 to n residues that are the same as or homologues of residues in the parent polypeptide and in the same order as in the parent polypeptide. C is any N₁S₁ residue, including but not limited to L-Cys, D-Cys, L-Pen, D-Pen or 3-mercapto phenylalanine. R₂ is from 0 to n-2 different residues that are the same as, or homologues of, residues in the known primary structure in the same order as in the parent polypeptide. Further, R₁ and R₂ together constitute at least two residues, and together form a sequence in the same order as in the parent sequence where C is either inserted between two adjacent residues or substitutes for a single residue. Any Cys in R₁ or R₂ may be conservatively substituted with Gly, Ala or Ser (among naturally occurring coded protein amino acid residues), and preferably Gly or Ala. Alternatively, a Cys with an S-protecting group (as hereafter described) may be employed. In a further embodiment, any synthetic or unnatural relatively small, neutral amino acid may be employed, for example amino isobutyric acid (Aib) or dehydroalanine (ΔAla). Any Pro in the two residues on the immediately adjacent amino-terminus side of C is located in a position that forms a part of the putative MCD, and is similarly conservatively substituted. Such substitution is required because there is no available N in Pro to complex to the coordination sphere of a metal ion, and therefore Pro cannot form a part of the MCD. Accordingly, any such Pro may be substituted with Gly, Ala or Ser (among naturally occurring coded protein amino acid residues), and preferably Gly or Ala. In a further embodiment, any synthetic or unnatural relatively small, neutral amino acid may be employed, for example Aib or ΔAla.

In the specification and the claims, the term "homologue" includes, in the case of a Cys to be substituted as set forth above, a conservative substitution with Gly, Ala or Ser, and preferably Gly or Ala. The term "homologue" further includes a Cys with an S-protecting group, wherein because of the S-protecting group the sulfur in the Cys residue is no longer available for binding to a metal ion. The terms "homologue" further includes, in the case of a Cys to be substituted, any synthetic or unnatural relatively small, neutral amino acid, for example Aib or ΔAla. In the case of a Pro to be substituted as set forth above, the term "homologue" includes a conservative substitution with Gly, Ala or Ser, and preferably Gly or Ala. The terms "homologue" further includes, in the case of a Pro to be substituted, any synthetic or unnatural relatively small, neutral amino acid, for example Aib or ΔAla. In the case of residues in either R₁ or R₂, other than Pro in the two residues on the immediately adjacent amino-terminus side of C or Cys, a "homologue" of such residue includes (a) a D-amino acid residue substituted for an L-amino acid residue, (b) a post-translationally modified residue, (c) a non-protein amino acid or other modified amino acid residue based on such residue, such as phenylglycine, homophenylalanine, ring-substituted halogenated, and alkylated or arylated phenylalanines for a phenylalanine residue, diamino proionic acid, diamino butyric acid, ornithine, lysine and homoarginine for an arginine residue, and the like, and (d) any amino acid residue, coded or otherwise, or a construct or structure that mimics an amino acid residue, which has a similarly charged side chain (neutral, positive or negative), preferably a similar hydrophobicity or hydrophilicity, and preferably a similar side chain in terms of being a saturated aliphatic side chain, a functionalized aliphatic side chain, an aromatic side chain or a heteroaromatic side chain.

Assume, for example, a parent polypeptide of six amino acid residues or residues that binds to a specified and known receptor. The parent polypeptide may be described as:
X¹-X²-X³-X⁴-X⁵-X⁶
Employing the formula R₁-C-R₂ as defined above, and assuming, for example, that in the first instance a Cys is used for C and is inserted between the X⁴ and X⁵ positions, it can be seen that the following peptides are contemplated by the invention with respect to insertion of Cys between the X⁴ and the X⁵ positions:
X¹-X²-X³-X⁴-Cys-X⁵-X⁶
X²-X³-X⁴-Cys-X⁵-X⁶
X³-X⁴-Cys-X⁵-X⁶
X¹-X²-X³-X⁴-Cys-X⁵
X¹-X²-X³-X⁴-Cys
X²-X³-X⁴-Cys-X⁵
X²-X³-X⁴-Cys
X³-X⁴-Cys-X⁵
X³-X⁴-Cys
Similar series of peptides can be generated assuming that the Cys is inserted between the X² and X³ positions, between the X³ and X⁴ positions, between the X⁵ and X⁶ positions, or following the X⁶ position. For example, assuming that Cys is inserted between the X² and X³ positions, the following peptides result:
X¹-X²-Cys-X³-X⁴-X⁵-X⁶
X¹-X²-Cys-X³-X⁴-X⁵
X¹-X²-Cys-X³-X⁴
X¹-X²-Cys-X³
X¹-X²-Cys
Assuming that Cys is inserted following the X⁶ position the following peptides result:
X¹-X²-X³-X⁴-X⁵-X⁶-Cys
X²-X³-X⁴-X⁵-X⁶-Cys
X³-X⁴-X⁵-X⁶-Cys
X⁴-X⁵-X⁶-Cys
X⁵-X⁶-Cys

In the practice of the invention, it is also possible and contemplated that the Cys may be employed to replace a residue in the parent polypeptide rather than be inserted between two adjacent residues. Assume again a parent polypeptide of six amino acid residues or residues that binds to a specified and known receptor described as:
X¹-X²-X³-X⁴-X⁵-X⁶
Employing the formula R₁-C-R₂, and assuming, for example, that the C is Cys and replaces, in the first instance, the X⁴ residue, it can be seen that the following peptides are contemplated by the invention with respect to replacement of X⁴ by Cys:
X¹-X²-X³-Cys-X⁵-X⁶
X²-X³-Cys-X⁵-X⁶
X¹-X²-X³-Cys-X⁵
X¹-X²-X³-Cys
X²-X³-Cys-X⁵
X²-X³-Cys
Similar series of peptides can be generated assuming that the Cys replaces the X³ residue, X⁵ residue or the X⁶ residue. For example, assuming that Cys replaces the X³ residue, the following peptides result:
X¹-X²-Cys-X⁴-X⁵-X⁶
X¹-X²-Cys-X⁴-X⁵
X¹-X²-Cys-X⁴
X¹-X²-Cys
Assuming that Cys replaces the X⁶ residue the following peptides result:
X¹-X²-X³-X⁴-X⁵-Cys
X²-X³-X⁴-X⁵-Cys
X³-X⁴-X⁵-Cys
X⁴-X⁵-Cys

Of course, in each of the preceding examples if any of the residues in the parent polypeptide X¹-X²-X³-X⁴-X⁵-X⁶ are a Cys, then a conservative substitution with Gly, Ala or Ser, and preferably Gly or Ala, may be employed. Alternatively, a Cys with an S-protecting group, as hereafter described, may be employed. In a further embodiment, any synthetic or unnatural relatively small, neutral amino acid may be employed in lieu of the Cys. Assume, for example, the parent polypeptide may be described as:
X¹-X²-Cys-X⁴-X⁵-X⁶
Employing the formula R₁-C-R₂, and assuming for example that in the first instance C is a Cys inserted between the X⁴ and X⁵ positions, and that Ala is employed to substitute for the endogenous Cys in the parent polypeptide in the X³ position, it can be seen that the following peptides are contemplated by the invention:
X¹-X²-Ala-X⁴-Cys-X⁵-X⁶
X²-Ala-X⁴-Cys-X⁵-X⁶
Ala-X⁴-Cys-X⁵-X⁶
X¹-X²-Ala-X⁴-Cys-X⁵
X¹-X²-Ala-X⁴-Cys
X²-Ala-X⁴-Cys-X⁵
X²-Ala-X⁴-Cys
Ala-X⁴-Cys-X⁵
Ala-X⁴-Cys
Similar substitutions are employed in the event that the C of the formula R₁-C-R₂ is employed by replacement rather than insertion.

Similarly, if in any of the preceding examples there is a Pro in the parent polypeptide, then when that Pro is located in the sequence wherein it would be within the sequence forming a part of the MCD (i.e., the C of the formula R₁-C-R₂ and the two residues immediately adjacent the amino-terminus side of C and comprising at least a part of R₁), then a conservative substitution with Gly, Ala or Ser, and preferably Gly or Ala may be employed in lieu of Pro. Alternatively, any synthetic or unnatural relatively small, neutral amino acid or mimetic (preferably but not necessarily also hydrophobic) may be employed in lieu of Pro, on the proviso that an N is available for complexing the metal ion. Assume, for example, the parent polypeptide may be described as:
X¹-X²-Pro-X⁴-X⁵-X⁶
Employing the formula R₁-C-R₂, and assuming for example that in the first instance C is Cys and the Cys is inserted between the X⁴ and X⁵ positions, and that Gly is employed to substitute for the endogenous Pro in the parent polypeptide in the X³ position, it can be seen that the following peptides are contemplated by the invention:
X¹-X²-Gly-X⁴-Cys-X⁵-X⁶
X²-Gly-X⁴-Cys-X⁵-X⁶
Gly-X⁴-Cys-X⁵-X⁶
X¹-X²-Gly-X⁴-Cys-X⁵
X¹-X²-Gly-X⁴-Cys
X²-Gly-X⁴-Cys-X⁵
X²-Gly-X⁴-Cys
Gly-X⁴-Cys-X⁵
Gly-X⁴-Cys
However, in the parent polypeptide X¹-X²-Pro-X⁴-X⁵-X⁶ and assuming that Cys is inserted following the X⁶ position, no substitution of Pro is required, such that the following peptides result:
X¹-X²-Pro-X⁴-X⁵-X⁶-Cys
X²-Pro-X⁴-X⁵-X⁶-Cys
Pro-X⁴-X⁵-X⁶-Cys
X⁴-X⁵-X⁶-Cys
X⁵-X⁶-Cys
Similar substitutions are employed in the event that the C of the formula R₁-C-R₂ is employed by replacement rather than insertion.

In yet another embodiment the parent polypeptide may be treated as a single unit. Assume a peptide of fifteen amino acid residues or residues binds to a specified known receptor. The peptide may be described as:
NH₂-X¹-X²-X³-X⁴-X⁵-X⁶-X⁷-X⁸-X⁹-X¹⁰-X¹¹-X¹²-X¹³-X¹⁴-X¹⁵-COOH

In this parent polypeptide, X may be any residue, which residue may repeat multiple times in any order or sequence. Thus the residue in position X¹ may be different from or the same as the residue in position X², which may be different from or the same as the residues in position X¹ or X³, and so on. Here too when a Cys is present in the parent polypeptide, substitution may be made. Similarly, where a Pro is present that comprises a part of the putative MCD, such as when a Pro falls within the two residues in R₁ immediately adjacent the amino-terminus side of the N₁S₁ residue hereafter provided, substitution may be made.

In the practice of this invention, an N₁S₁ residue, providing both an N and an S for complexing to a metal ion, is employed, such as L- or D-cysteine, or any other natural, unnatural or synthetic amino acid or mimetic providing both an N and S for complexing to a metal ion. For the following examples, "Cys" is employed, it being understood that any N₁S₁ residue could be similarly employed, and that this example and those that follow are not limited to Cys as the N₁S₁ residue. Peptides are constructed using standard peptide synthesis techniques, in which the cysteine is inserted after the 2nd position (X²) through the 16th (n+1) position (following X¹⁵), such that the following peptides result:
NH₂-X¹-X²-Cys-X³-X⁴-X⁵-X⁶-X⁷-X⁸-X⁹-X¹⁰-X¹¹X¹²-X¹³-X¹⁴-X¹⁵-COOH
NH₂-X¹-X²-X³-Cys-X⁴-X⁵-X⁶-X⁷-X⁸-X⁹-X¹⁰-X¹¹-X¹²-X¹³-X¹⁴-X¹⁵-COOH
NH₂-X¹-X²-X³-X⁴-Cys-X⁵-X⁶-X⁷-X⁸-X⁹-X¹⁰-X¹¹-X¹²-X¹³-X¹⁴-X¹⁵-COOH
NH₂-X¹-X²-X³-X⁴-X⁵-Cys-X⁶-X⁷-X⁸-X⁹-X¹⁰-X¹¹-X¹²-X¹³-X¹⁴-X¹⁵-COOH
NH₂-X¹-X²-X³-X⁴-X⁵-X⁶-Cys-X⁷-X⁸-X⁹-X¹⁰-X¹¹-X¹²-X¹³-X¹⁴-X¹⁵-COOH
NH₂-X¹-X²-X³-X⁴-X⁵-X⁶-X⁷-Cys-X⁸-X⁹-X¹⁰-X¹¹-X¹²-X¹³-X¹⁴-X¹⁵-COOH
NH₂-X¹-X²-X³-X⁴-X⁵-X⁶-X⁷-X⁸-Cys-X⁹-X¹⁰-X¹¹-X¹²-X¹³-X¹⁴-X¹⁵-COOH
NH₂-X¹-X²-X³-X⁴-X⁵-X⁶-X⁷-X⁸-X⁹-Cys-X¹⁰-X¹¹-X¹²-X¹³-X¹⁴-X¹⁵-COOH
NH₂-X¹-X²-X³-X⁴-X⁵-X⁶-X⁷-X⁸-X⁹-X¹⁰-Cys-X¹¹-X¹²-X¹³-X¹⁴-X¹⁵-COOH
NH₂-X¹-X²-X³-X⁴-X⁵-X⁶-X⁷-X⁸-X⁹-X¹⁰-X¹¹-Cys-X¹²-X¹³-X¹⁴-X¹⁵-COOH
NH₂-X¹-X²-X³-X⁴-X⁵-X⁶-X⁷-X⁸-X⁹-X¹⁰-X¹¹-X¹²-Cys-X¹³-X¹⁴-X¹⁵-COOH
NH₂-X¹-X²-X³-X⁴-X⁵-X⁶-X⁷-X⁸-X⁹-X¹⁰-X¹¹-X¹²-X¹³-Cys-X¹⁴-X¹⁵-COOH
NH₂-X¹-X²-X³-X⁴-X⁵-X⁶-X⁷-X⁸-X⁹-X¹⁰-X¹¹-X¹²-X¹³-X¹⁴-Cys-X¹⁵-COOH
NH₂-X¹-X²-X³-X⁴-X⁵-X⁶-X⁷-X⁸-X⁹-X¹⁰-X¹¹-X¹²-X¹³-X¹⁴-X¹⁵-Cys-COOH
In this way each potential insertion point along the parent polypeptide is "scanned" to determine if creation of a metal ion-stabilized secondary structural motif at each insertion point results in a metallopeptide with biological activity, however defined, and preferably biological activity at least equal or approximately equal to that of the parent polypeptide.

During synthesis the -SH group of C of the formula R₁-C-R₂ may be protected using an orthogonal protecting agent as set forth below. The resulting orthogonally-protected Cys-containing peptide is then deprotected, and subsequently complexed with a metal ion, such as a rhenium ion, thereby forming a metallopeptide, using in the case of a rhenium ion a suitable pre-formed metal-oxo transfer agent, such as Re(O)Cl₃(PPh₃)₂. Through use of suitable assays or tests, such as competitive inhibition assays, the binding of each of the resulting metallopeptides is compared against the parent polypeptide, and those metallopeptides with enhanced or increased binding are identified as involving a reverse turn structure about the metal ion complex forming all or a part of the BD of the metallopeptide.

In a related approach, sequences of defined length but less than that of the parent polypeptide are synthesized. These sequences are based on, for example, the hypothetical known parent polypeptide of 15 residues as set forth above. In one embodiment, a Cys is inserted into the sequence of defined length. Thus it is possible and contemplated that a series of sequences of four amino acid residues is synthesized and screened as set forth above. The four amino acid residues consist of one residue on the carboxyl terminus side of the Cys and two residues on the amino terminus side of the Cys, as follows:
NH₂-X¹-X²-Cys-X³-COOH
NH₂-X²-X³-Cys-X⁴-COOH
NH₂-X³-X⁴-Cys-X⁵-COOH
NH₂-X⁴-X⁵-Cys-X⁶-COOH
NH₂-X⁵-X⁶-Cys-X⁷-COOH
NH₂-X⁶-X⁷-Cys-X⁸-COOH
NH₂-X⁷-X⁸-Cys-X⁹-COOH
NH₂-X⁸-X⁹-Cys-X¹⁰-COOH
NH₂-X⁹-X¹⁰-Cys-X¹¹-COOH
NH₂-X¹⁰-X¹¹-Cys-X¹²-COOH
NH₂-X¹¹-X¹²-Cys-X¹³-COOH
NH₂-X¹²-X¹³-Cys-X¹⁴-COOH
NH₂-X¹³-X¹⁴-Cys-X¹⁵-COOH

In yet another related approach, sequences of defined length but less than that of the parent polypeptide are synthesized, with the Cys employed as substitute for an amino acid residue in the parent polypeptide. Thus it is possible and contemplated that a sequence of four amino acid residues is synthesized and screened as set forth above, the four amino acid residues comprising one residue on the carboxyl terminus side of the Cys, and two residues on the amino terminus side of the Cys, with the Cys substituted for a residue in the parent polypeptide, as follows:
NH₂-X¹-X²-Cys-X⁴-COOH
NH₂-X²-X³-Cys-X⁵-COOH
NH₂-X³-X⁴-Cys-X⁶-COOH
NH₂-X⁴-X⁵-Cys-X⁷-COOH
NH₂-X⁵-X⁶-Cys-X⁸-COOH
NH₂-X⁶-X⁷-Cys-X⁹-COOH
NH₂-X⁷-X⁸-Cys-X¹⁰-COOH
NH₂-X⁸-X⁹-Cys-X¹¹-COOH
NH₂-X⁹-X¹⁰-Cys-X¹²-COOH
NH₂-X¹⁰-X¹¹-Cys-X¹³-COOH
NH₂-X¹¹-X¹²-Cys-X¹⁴-COOH
NH₂-X¹²-X¹³-Cys-X¹⁵-COOH

In yet another related approach, alternate sequences of defined length but less than that of the parent polypeptide are synthesized. Thus it is possible and contemplated that yet another sequence of four amino acid residues is synthesized and screened as set forth above, the four amino acid residues including Cys as the carboxyl-terminus residue of a tetrapeptide sequence including three ordered residues from the parent polypeptide, as follows:
NH₂-X¹-X²-X³-Cys-COOH
NH₂-X²-X³-X⁴-Cys-COOH
NH₂-X³-X⁴-X⁵-Cys-COOH
NH₂-X⁴-X⁵-X⁶-Cys-COOH
NH₂-X⁵-X⁶-X⁷-Cys-COOH
NH₂-X⁶-X⁷-X⁸-Cys-COOH
NH₂-X⁷-X⁸-X⁹-Cys-COOH
NH₂-X⁸-X⁹-X¹⁰-Cys-COOH
NH₂-X⁹-X¹⁰-X¹¹-Cys-COOH
NH₂-X¹⁰-X¹¹-X¹²-Cys-COOH
NH₂-X¹¹-X¹²-X¹³-Cys-COOH
NH₂-X¹²-X¹³-X¹⁴-Cys-COOH
NH₂-X¹³-X¹⁴-X¹⁵-Cys-COOH

In each of the foregoing examples a tetrapeptide sequence is employed, wherein one of the residues is Cys. However, it is to be understood that the sequence may be of any length from a tripeptide (e.g., X-X-Cys) to a peptide of length n+1, where n is the length of the parent polypeptide. In alternative embodiments, other residues, mimics, terminal groups can be added, such that the length of the sequence is in excess of n+1. Similarly, it is to be understood that Cys may be any residue, natural or unnatural, or mimetic thereof, or different construct, provided only that it comprises an N₁S₁ residue. In each such case, the resulting Cys-containing peptides are complexed with a metal ion, such as a rhenium ion, forming a metallopeptide, such as by using a suitable pre-formed metal-oxo transfer agent such as Re(O)Cl₃(PPh₃)₂.

In yet another embodiment, it is contemplated and to be understood that a parent polypeptide may be divided into overlapping sequences, and that each such sequence is then effectively considered and treated as an independent parent polypeptide, according to the methods and constructs of this invention. For example, assume a parent polypeptide of length n where n is 30. Such parent polypeptide may be suspected of containing more than one discrete BD. Accordingly, in one embodiment the primary sequence is divided into constructs, such as three constructs. For example, one construct may consist of the residues from the 1 to 15 positions, a second the residues from the 7 to 21 positions, and a third the residues from the 16 to 30 positions. In this way, all possible endogenous and contiguous BDs are included in at least one of the three constructs. Each construct is thereafter treated as an independent parent polypeptide, according to the methods of this invention. In a preferred embodiment, this method is employed with parent polypeptides of at least a length where n is 15, with three divided constructs employed, each such divided construct overlapping the adjacent divided construct by at least two residues.

Through use of suitable screen assays, such as competitive inhibition assays, the binding of each of the resulting metallopeptides is compared against the parent polypeptide, and those with enhanced or increased binding are identified as involving a secondary structural motif about the metal ion complex forming at least a part of the BD. Once one or more metallopeptides with enhanced or increased binding are identified, amino acid residues on either the amino or carbonyl ends may be added, subtracted, and the like, side chains modified, and similar changes made to obtain a metallopeptide with optimal binding or other desired characteristics, including agonist, antagonist or mixed agonist/antagonist activity.

In the event that the parent polypeptide contains one or more endogenous Cys residues, it is possible to protect the intrinsic Cys residues with a non-orthogonal -SH protecting agent, to protect the introduced N₁S₁ residue with an orthogonal -SH protecting agent, to thereafter selectively deprotect the orthogonal -SH protecting agent, to then complex the deprotected N₁S₁ residue with a metal ion, and thereafter to deprotect the Cys residue with the non-orthogonal -SH protecting agent. Examples of common non-orthogonal -SH protecting groups include, but are not limited to, trityl, benzyl, p-methoxy benzyl, and ^{t}Bu.

It may further been seen from the foregoing that in another embodiment of the invention the pharmacophore of a receptor or other target of interest may be defined. Assume that a known parent polypeptide (which may be a peptide, polypeptide or protein), binds to a receptor for which definition of the pharmacophore is desired. While the primary structure of the parent polypeptide is known, the specific residues involved in binding to the receptor, and the secondary structure involved in such binding to the receptor, is not known. Thus definition of the pharmacophore cannot be derived solely from knowledge of the primary structure of the parent polypeptide. Knowledge of the pharmacophore may, for example, permit design and construction of any of a wide variety of small molecules, including peptidomimetics and non-peptide small molecules, which bind to the receptor, optionally acting as either an agonist or antagonist. Based on the primary structure of the known parent polypeptide, a series of metallopeptides is constructed as set forth above. The metallopeptide with optimal binding and other desired characteristics with respect to the receptor and the parent polypeptide is selected. The selected metallopeptide may be optimized as desired, such as by determining the fewest residues yielding acceptable binding, for example such that in the formula R₁-Cys-R₂, R₁ and R₂ together constitute no more than three, and optionally preferably only two, residues. Similarly, modifications to optimize the selected metallopeptide may optionally be made with respect to side chains, such that the resulting metallopeptide has desired hydrogen bond donors and acceptors, charged centers, aromatic ring centers, hydrophobic centers and the like, thereby providing optimal binding to the receptor.

When a metallopeptide is selected that provides optimal binding to the desired receptor compared to the parent polypeptide, as determined by the methods of this invention, then the metallopeptide so selected may be modeled. In a typical peptide (i.e. a parent polypeptide), there are a wide variety of torsion angles that determine a diverse range of probabilistically-determined secondary and tertiary structures of the peptide. Thus with a typical peptide, knowledge of the primary structure does not necessary imply that the secondary or tertiary structure can be determined absent empirical evidence. However, with a metallopeptide of this invention, employing the formula R₁-Cys-R₂, the metal ion and MCD of the metallopeptide are conformationally constrained, with a fixed and determined secondary structure. Because of the metal ion complexation, the torsion angles within and between the residues of the MCD are fixed and may be determined based upon the type of metal ion employed, including its oxidation state, coordination geometries and the like.

As a result, any metallopeptide, including specifically the portion thereof the MCD and, to a significant extent, residues adjacent to the MCD, may be modeled, thereby determining the secondary structure. By this means the pharmacophore can be modeled as the complement to the metallopeptide. For example, the location in a three-dimensional construct of hydrogen bond donors and acceptors, positively and negatively charged centers, aromatic ring centers, hydrophobic centers and the like may be determined (including determination of the distance between atoms constituting a part of the pharmacophore). Any of a wide variety of software programs may be employed for such modeling, including programs such as SYBYL (Tripos, Inc.), Alchemy (Tripos, Inc.), Align/Pharmacophore (Accelrys Inc.), Catalyst (Accelrys Inc.), MacroModel (Schrödinger, Inc.), PC-Model (Serena Software), CS ChemOffice (CambridgeSoft Corporation) and other programs known in the field. Techniques for pharmacophore modeling are taught in any number of articles and texts, including Pharmacophore Perception, Development and Use in Drug Design, Osman F. Güner, Ed., Int'l University Line, La Jolla, CA, 2000; and Guidebook on Molecular Modeling in Drug Design, N. Claude Cohen, Ed., Academic Press, San Diego, 1996.

It may further be seen that using the methods and constructs of this invention libraries of metallopeptides may be designed and made wherein each constituent series member includes an MCD sequence necessary for providing a coordination site for complexation with a metal. These libraries may be made using any method, including specifically solution and solid phase synthesis techniques.

Upon complexing the MCD with a metal, a specific structure results which forms a secondary structural motif. The specific stereochemical features of this complex are due to the stereochemistry of the coordination sphere of the complexing metal ion. The preferred geometry of the coordination sphere of the metal dictates and defines the nature and extent of conformational restriction. In general, most of the metals that may prove useful in this invention have a coordination number of 4 to 6 (and sometimes, but rarely, as high as 8), which implies that the putative MCD must be made of residues with reactive groups located in a stereocompatible manner establishing a bond with a metal ion of given geometry and coordination sphere. Coordinating groups in the peptide chain include nitrogen atoms of amine, amide, Imidazole, or guanidino functionalities; sulfur atoms of thiols or disulfides; and oxygen atoms of hydroxy, phenolic, carbonyl, or carboxyl functionalities. In addition, the peptide chain or individual amino acid residues can be chemically altered to include a coordinating group, such as oxime, hydrazino, sulfhydryl, phosphate, cyano, pyridino, piperidino, or morpholino groups. For a metal with a coordination number of 4, a preferred MCD is a three amino acid sequence in which one of the amino acid residues has a side chain with a sulfur-based coordinating group (such as Cys), such residue constituting an N₁S₁ ligand. Thus, a three amino acid sequence can provide an N₃S, N₂SO or similar ligand, yielding tetradentate coordination of a metal ion utilizing nitrogen and sulfur and, optionally, oxygen atoms.

The choice of metal ion partially determines the structure of the resulting turning structure. For example, use of an Re ion results in a square pyramidal coordination geometry. Tc (which has substantially similar coordination requirements and chemistries and generally may be substituted for Re in any example herein) similarly results in a square pyramidal coordination geometry. Use of other metal ions, such as Cu, Ni or Zn, results in square planar coordination geometries. Thus while the atomic radius of Re is on the order of 1.37 A and that of Cu is smaller, on the order of 1.28 A, the resulting dimensions of the metal coordination group is determined, in large part, by the coordination geometry, and not just by the atomic radius of the metal ion. With metal ions such as Cu, Ni or Zn employing square planar coordination tetradentate geometries, the metal ion and each of the four coordinating atoms (such as S, N or O) are co-planar. However, when employing metal ions such as Re or Tc (which result in square pyramidal coordination tetradentate geometries), the four coordinating atoms (such as S, N or O) are co-planar, but the metal ion is, in the case of Re, about 65 Å removed from the plane of the coordinating atoms.

In this invention any of a wide range of metal ions may be employed, but Re and Tc are particularly preferred. Both metals form similar complexes with Cys-containing peptides yielding similar square pyramidal complexes. Re-complexed peptides, however, are chemically more stable than the corresponding Tc-containing peptides. The square planar complexes of Zn and Cu, with the metal ion as well as the four coordinating atoms of the peptide all in one plane, results in a near identical complexation geometry as is obtained with Tc or Re, where the metal ion is projected upwards from the plane of four coordinating atoms of the peptide, notwithstanding the differences in the atomic radius of the metal ions. The net result are metallopeptides that each afford topographic similarities, whether for example Re, Tc, Zn or Cu is employed. The Re-complexed metallopeptides, however, are unique in that the metallopeptides are air and moisture stable, without any need for special or exotic excipients or protecting agents. The Re-complexes can routinely be isolated as solid compounds and are stable as solids and in solutions over a wide pH range, thereby facilitating both analytical characterization and, more importantly, use in both in vitro and in vivo biological experiments over a wide range of conditions. Other metal types, such as Zn-complexes and Cu-complexes, are utilized in experiments in a solution form. However, Zn-complexes and Cu-complexes are extremely easy to form, and essentially are formed in the presence of 1 micromolar to 1 millimolar concentration of the metal ion in an appropriately buffered solution.

The Re- and Tc-complexes are metaloxo complexes, generally and in a preferred embodiment in an oxidation state [V]. The metaloxo core M=O in the metallopeptides may give rise to an isomerism in the core structure. The metal-oxo group may be *syn* or *anti* with respect to a chiral amino acid side chain. Since the orientation of the oxo group does not alter the topographic surface created by the amino acid side chains, this isomerism has no effect on the biological activity of the metallopeptides. It can be well appreciated from **Figs. 1** **A, 1 C, 2 A, 2 B, 3 A, 3 B, 4 A, 4 B, 4 C, 5** and **6** that the oxo group of a metal ion does not sterically hinder the conformationally constrained amino acid side chain presentations. In fact, the metal ion is situated at a location spatially similar to that where turns are stabilized by a hydrogen bond in natural turn structures; thus the oxo group falls within a space not addressable in natural turn structures. The computer modeling of individual syn- and anti-isomers of metallopeptides have shown that these two structures are completely indistinguishable with respect to each amino acid location, with orientation of the oxo group being the only difference.

The utility of an embodiment of the invention, resulting in a structure that mimics topologies of naturally occurring peptide and protein structures, may be perceived with reference to certain of the figures of the invention. Protein structure is discussed and explain extensively in Introduction to Protein Structure, Carl Branden and John Tooze, 1991, Garland Publishing Inc. New York and London, and the discussion therein is incorporated here by reference. **Fig. 1** **B** depicts the backbone structure of a classical beta-II' turn. **Fig. 1** **A** depicts the structure, of an Re metal-coordinated pentapeptide of SEQ ID NO:1, wherein an L-Cys is employed. **Fig. 1** **C** is the diagram of **Fig. 1** **A** superimposed on the diagram of **Fig. 1** **B** at their respective C-α carbon atoms of the three consecutive N-terminal amino acid residues (C₁-α, C₂-α and C₃-α atoms). It can be seen by examination of **Fig. 1** **C** that there is excellent overlap at these three carbon atoms, with an RMSD < 0.05 Å, demonstrating that the turn structure of **Fig. 1** **A** forms a close mimic of the classical beta-II' turn of **Fig. 1** **B**. Thus the topology and relative relationship of, for example, side chains of these amino acid residues of **Fig. 1** **A** and **Fig. 1** **B** would be very similar. It should be noted that the sequence employed for **Fig. 1** **A**, Ala-Ala-Ala-Cys-Ala (SEQ ID NO:1), was employed only as a model, and that any pentapeptide wherein Cys is the 4 position and the remainder of the residues are any residue other than Cys or Pro would result in a similar backbone diagram, with the same overlap of the C₁-α, C₂-α and C₃-α atoms.

**Fig. 2** **A** depicts the structure, similarly by way of a backbone diagram, of a Re metal-coordinated Ala-Ala-Ala-D-Cys-Ala pentapeptide. **Fig. 2** **B** is a diagram of **Fig. 2** **A** superimposed at the respective C- α carbon atoms of the three consecutive N-terminal amino acid residues (C₁-α, C₂-α and C₃-α atoms). It can be seen by examination of **Fig. 1** **C** that there is here also excellent overlap at these three carbon atoms, similarly with an RMSD < 0.05 Å, demonstrating that the turn structure of **Fig. 2** **A** also forms a close mimic of the classical beta-II' turn of **Fig. 1** **B.** Thus the topology and relative relationship of, for example, side chains of amino acid residues of **Fig. 2** **A** and **Fig. 1** **B** would be very similar. Here too any pentapeptide sequence employing a D-Cys in the 4 position and any residues other than Cys or Pro in the remaining positions would result in a similar backbone diagram, with the same overlap of the C₁-α, C₂-α and C₃-α atoms. It is also evident from a comparison of Re-peptide structures in **Fig. 1** **A** and **2 A** that the C-terminal 5th amino acid extensions in these templates effectively allow for accessing additional and distinct chemical space for establishing a specific receptor contact, thereby adding to enhanced diversity in these structures.

**Fig. 3** **A** is a backbone diagram of the peptide sequence Ala-Ala-Ala-D-Cys-Ala complexed to a rhenium metal ion superimposed on an extended chain peptide structure. In this depiction C-α atoms of two consecutive amino acid residues of extended chain structure are overlapped onto C₁-α and C₂-α atoms of the metallopeptide sequence. The superimposition suggests positioning of these two amino acid residues along with their C-beta carbon atoms in approximately similar chemical juxtaposition. **Fig. 3** **B** similarly depicts C-α atoms of two consecutive amino acid residues of an extended chain structure overlapped onto the C₂-α and C₃-α atoms of the sequence Ala-Ala-Ala-D-Cys-Ala. The superimposition suggests exact positioning of C₂-α and C₃-α atoms as well as the C₃-β carbon atom, while allowing access to a different chemical space at the C₂-β carbon atom;

**Figs. 4** **A, 4 B** and **4 C** illustrate the rhenium complexed peptide sequence Ala-Ala-Ala-D-Cys-Ala superimposed on a β-sheet peptide structure. Three separate superimpositions are shown: that of **Fig. 4** **A** with C-α atoms of two consecutive amino acid residues of the β-sheet structure overlapped onto C₁-α and C₂-α atoms of the metallopeptide sequence; that of **Fig. 4** **B** with C-α atoms of two consecutive amino acid residues of the β-sheet structure overlapped onto C₂-α and C₃-α atoms of the metallopeptide; and that of **Fig. 4** **C** with C-α atoms of two consecutive amino acid residues of the β-sheet structure overlapped onto C₂-α and C₃-α atoms of the metallopeptide in a different orientation that as shown in **Fig. 4** **B**. Each illustrates either similar or exaction positioning of C-α carbon atoms, while allowing the metallopeptides to access additional or different chemical space, such as at the C₂-β and C₃-β carbon atoms in the case of **Fig. 4** **B** or at the at C₂-β atom in the case of **Fig. 4** **C**.

**Fig. 5** illustrates that the topology of side chains in a metallopeptide can be organized and selected similar to that observed in natural turn structures, such as helixes. Thus there is a functional helicity in the metallopeptide with respect to amino acid residues 1 and 5, which i to l + 5 residue pitch on the metallopeptide can be matched topographically to the i and i + 4 residues chemical space in an α-helix. Similarly, utilizing a natural Cys, a similar topology results, as shown in **Fig. 6**.

The Ramachandran plot of **Fig. 8** shows the coordinates, and thus corresponding structural propensity, of the M-1, M-2, M-3-L, and M-3-D residues of the metallopeptides of **Fig. 7** **A** and **7 B**. It can thus be seen that a metallopeptide with an L-Cys forms a mimic of a short right hand turn of helix, while a metallopeptide with a D-Cys forms a mimic of a short left hand turn of a helix. It is well know that natural helix turns are right handed only. The metallopeptide approach, therefore, offers the advantage that both right and left handed structures can be constructed. These structure can be utilized to topographically position the side chains of i and l + 5 residues in a L-Cys containing metallopeptide in the same chemical space as that for the side chains of i and I + 4 residues in a right handed helix. Alternatively, a D-Cys containing metallopeptide allows creation of a topographic mimic for i and i + 4 residues of a putative un-natural left helix.

It is also to be appreciated that while the natural and linear peptide and analogues are subjected to the confines of the Chou-Fasman type of rules (P.Y. Chou and G.D. Fasman: Prediction of protein structure, Biochemistry 13:222-245, 1974) that preclude inclusion of certain amino acid residues in particular types of secondary structures, the methods and constructs of this invention are completely independent of these rules. This invention allows incorporation of any natural or synthetic amino acid residues in the structure without Chou-Fasman rules limitations, and with virtually no other limitations.

It is to be appreciated here that while the structures shown in **Figs. 1 - 7** have backbones that are very distinct from those in natural protein structures, the objective of this invention is to utilize the similarities in terms of positioning C-α carbon atoms, as well as C-β carbon atoms in certain cases, of various amino acid residues in the same chemical spaces as in corresponding native protein structures, and further to derivatize these positions so as to achieve a chemical topology or surface similar to that in a natural bioactive structural motif. Utilizing these metallopeptide structures a biologically active molecule can therefore be identified that represents and defines the sites of folding or conformational constraints in a parent polypeptide, such as a peptide or protein. For a polypeptide with an unknown structure-function relationship, this information is generated by synthesizing a combination of all the metallopeptides corresponding to the parent polypeptide designed by inserting or substituting a Cys residue at some or all positions in the parent polypeptide. The structure of the biologically active metallopeptide in this series then elucidates the folding site in the polypeptide. This metallopeptide also provides information on key constrained amino acid residues, including but not limited to their relationship, including spatial relationship, to one another and their chirality. This information is then utilized to generate a molecular model, such as a computer-based molecular model, that defines a minimal structure pharmacophore model for further optimization. In the practice of this invention it is possible to utilize structural motifs thus identified by further modification of the defined topology to accentuate a desired biological effect, such as by substituting homologous amino acid side chains in place of naturally-occurring side chains in the parent polypeptide. Examples of homologous side chains include, but are not limited to, substituting D-amino acid residues for an L-amino acid or utilizing homologues of an amino acid, such as for example the series phenylglycine, homophenylalanine, ring-substituted halogenated, and alkylated or arylated phenylalanines for a phenylalanine residue, diamino proionic acid, diamino butyric acid, ornithine, lysine and homoarginine for an arginine residue, and the like.

It may be seen that in the practice of the invention a free thiol or sulfhydryl (-SH) group of a residue is utilized for complexation of metal ions. Peptides and other organic molecules with free - SH groups, however, are easily oxidized in air and in solution, and can often form a disulfide-linked dimer. If more than one free -SH group is present in a molecule, oxidation may lead to a complex polymer. In addition, with more than one free -SH group when the metal ion is complexed to the peptide, it is possible to have metal ion complexation at more than one MCD in the peptide. This results in mixed species of metallopeptides, thereby complicating determination of the specific metallopeptide responsible for binding to a target of interest, as well as determination of the relevant secondary structure. Similarly, if a mixture of different peptides or organic molecules with free -SH groups are prepared, oxidation generally leads to a complex mixture of polymers of unknown composition. This is of serious concern in preparing libraries of metallopeptides or other organic molecules where one or more -SH group is intended for use in metal complexation.

In order to construct metallopeptides of this invention which incorporate an -SH group, and most particularly in order to construct libraries, it is desirable to employ S-protected derivatives. The S-protecting group is chosen such that (a) the synthesis of peptides with the S-protecting group is compatible with methods of solution and solid phase peptide synthesis, so that the S-protecting group is stable during synthetic procedures, and (b) the S-protecting group can be deprotected in situ, without cleavage from the resin in the case of solid phase synthesis, during the metal complexation step. An S-protecting group meeting the forgoing criteria is defined herein as an orthogonal S-protected group (OSPG). Many prior art methods meet at most only one of the two criteria specified above, and thus do not constitute an OSPG as defined herein.

Use of orthogonally S-protected thiol groups permits synthesis of metallo-compounds in a single vessel. A mixture of compounds, each compound containing an OSPG, is used for complexation with a metal ion, and it is only during metal ion complexation that the S-protected group is deprotected, and accordingly polymerization and cross-linking is avoided. This procedure thus provides homogenous libraries of metallopeptides.

One OSPG meeting the criteria specified above, and which can be advantageously used in this invention, employs an S'Bu (S-thio-butyl or S-t-butyl) group to protect the -SH group. The S^{t}Bu group is stable under both the acidic and basic conditions typically employed in peptide synthesis. Further, the S^{t}Bu group may be cleaved by reduction using a suitable phosphine reagent, which reduction step may be employed immediately prior to, or in conjunction with, complexing of a metal ion to the peptide. Such OSPG cleavage does not cleave the peptide from the resin, or otherwise alter the structure of the peptide.

Another OSPG meeting the criteria specified above and suitable for this invention employs an S-Acm (S-acetamidomethyl) group to protect the -SH group. The Acm group is also stable under the acid and base conditions usually employed during peptide synthesis. The S-Acm group may be removed by treatment of S-Acm-protected peptide or peptide resin with mercury (II) acetate or silver (I) tertrafluoroborate, which liberates the thiol peptide in its mercury or silver ion-complexed state. If a mercury or silver ion metallopeptide is desired, the resulting metallopeptide may be kept in solution and employed in assays as described herein. Alternatively, free thiol-containing peptide can be recovered by treating the mercury or silver ion and thiol complexed salts with an excess of a thiol-containing reagent, such as beta-mercaptoethanol or dithiothreitol. The resulting peptide is then used for metal complexation to a metal such as Re or Tc. Alternatively, the mercury or silver ion and thiol complexed peptide may be directly treated with a metal ion complexing reagent, such as an Re complexing reagent, to form a desired metallopeptide, such as an Re metallopeptide.

Other examples of OSPGs for metallopeptides include 4-methoxytrityl (Mmt), 3-nitro-2-pyridinesulfenyl (Npys) and S-sulfonate (SO₃H). Mmt is selectively removed upon treatment with 1% TFA in dichloromethane. Npys and S-sulfonate are selectively removed by treatment with a thiol-containing reagent such as beta-mercaptoethanol or dithiothreitol or a phosphine reagent such as tributyl phosphine. The Npys group (R.G. Simmonds RG et al: Int J Peptide Protein Res, 43:363,1994) is compatible with Boc chemistry for peptide synthesis and the S-sulfonate (Maugras I et al: Int J Peptide Protein Res, 45:152, 1995) is compatible with both Fmoc and Boc chemistries. Similar OSPGs derived from homologueous series of S-alkyl, or S-aryl, or S-aralkyl may also be used in this invention. A primary characterization of the OSPG is that its use results in the formation of a disulfide (S-S) bond utilizing one sulfur atom each from the thiol-containing amino acid and the protecting group. In addition, the resulting disulfide bond is cleavable by the use of any of a variety of disulfide cleaving agents, including but not limited to phosphine- and thiol-containing reagents.

The method employing S^{t}Bu protected -SH groups, or other OSPGs, may be employed for the generation of either solid phase or soluble libraries. For solid phase libraries, peptides may be synthesized by use of conventional Fmoc chemistry. In the case of conventional Fmoc chemistry, Fmoc-L-Cys-(S^{t}Bu) is coupled to an appropriate resin, via one or more intermediate amino acid residues, and additional amino acid residues are thereafter coupled to the L-Cys-(S^{t}Bu) residue. S^{t}Bu may be employed with either L- or D-Cys, and any of a variety of other amino acid residues, including designer or unnatural amino acid residues and mimics thereof, characterized by an -SH group available for complexation to a metal ion, including, but not limited to, 3-mercapto phenylananine and other related 3-mercapto amino acid residues such as 3-mercapto valine (penicillamine), all of the foregoing of which constitute an N₁S₁ residue. In all these cases, S-protection can be by S-Bu^{t}, S-Acm, Mmt, Npys, S-sulfonate and related groups, as described above.

The complexation of metal ions to the peptides, including peptides in a library, and specifically to the MCD of peptides, is achieved by mixing the peptides with the metal ion. This is conveniently done in solution, with the solution including an appropriate buffer. In one approach, the metal ion is, when mixed with the peptide or peptidomimetic constituents, already in the oxidation state most preferred for complexing to the MCD. Some metal ions are complexed in their most stable oxidation state, such as calcium (II), potassium (I), indium (III), manganese (II), copper (II), zinc (II) and other metals. In other instances, the metal must be reduced to a lower oxidation state in order to be complexed to the MCD. This is true of ferrous, ferric, stannous, stannic, technetiumoxo[V], pertechnetate, rheniumoxo[V], perrhenate and other similar metal ions. Reduction may be performed prior to mixing with the sequences, simultaneously with mixing with the sequences, or subsequent to mixing with the sequences. Any means of reduction of metal ions to the desired oxidation state known to the art may be employed.

Re and Tc are preferred metal ions to employ, particularly in that the resulting metallopeptides may be purified and removed from solution, such as by lyophilization, and remain stable. Other metallopeptides, as for example metallopeptides utilizing Zn, Cu, Ni, Co, Fe and Mn are stable in solution, but are prone to oxidation and loss of the metal ion if removed from solution. Thus these metallopeptides must be kept in solution, and optimally at the appropriate pH and with appropriate buffers, at all times, including during conduct of assays and other tests. This imparts some limitations on the utility of these metal ions; however, metallopeptides utilizing metal ions other than Re or Tc may be employed as discussed herein.

For tetradentate coordination with a metal ion, rhenium or technietum are preferred ions. Because of its ready availability and the stability of the coordination complex, Re is a particularly preferred metal ion. Solid phase resin bound peptide or peptidomimetic sequences may be labeled with rhenium ion by treatment with the rhenium transfer agent ReOCl₃(PPh₃)₂ in the presence of a base, such as 1,8-Diazabicyclo[5,4,0] undec-7-ene (DBU). The sequences may then be cleaved from the resin. Peptide or peptidomimetic sequences in solution may similarly be labeled by treatment with the rhenium transfer agent ReOCl₃(PPh₃)₂ in the presence of a base, such as triethyl amine, disopropylethylamine, N-methylmopholine or DBU. Metal complexation in the presence of DBU as a base can conveniently be accomplished at ambient room temperature.

In an alternative method of metal complexation a mild base, such as sodium acetate, can be used. In this case the thiol-containing sequence, either in solution or bound to solid phase, is taken in a suitable solvent, such as dimethylformamide (DMF), dichloromethane (DCM), N-methylpyrrolidinone (NMP), methanol (MeOH) or a mixture thereof, and heated to 60-70° C with the rhenium transfer agent ReOCl₃(PPh₃)₂ in the presence of sodium acetate for 15 minutes. Similarly, other bases such as triethylamine, ammonium hydroxide and so on, may be employed. According to this invention, MeOH is a preferred choice of solvent for rhenium complexation in the case of S-deprotected peptides in solution. The solvent choice for S-deprotected peptides still attached to the solid phase is guided mainly by considerations of superior solvation (swelling) of the solid phase. DMF and NMP may be employed. Various mixtures of these solvents, also in combination with MeOH, and DCM, CHCl₃ and so on, may also be employed to yield optimized complexation results.

In one embodiment of this invention, an S'Bu protected peptide is treated in situ with rhenium transfer agent in the presence of DBU and tributylphosphine to effect S-deprotection and rhenium complexation in one vessel. Alternately, complexing of rhenium to the S'Bu protected peptide in the presence of rhenium perrhenate may be accomplished by treatment with Sn[II]Cl₂. This reagent effects S-deprotection as well as conversion of the ReO₄ state to an ReO state in situ to thereby effect complexation of the rhenium to the S-deprotected peptide. A preferred procedure in this invention is the use of S-Bu^{t} protected peptide with S-deprotection by treatment with tributylphosphine, and metal complexation of the resulting peptide utilizing ReOCl₃(PPh₃)₂ in the presence of DBU at room temperature.

It is possible and contemplated to prepare libraries of peptides of this invention, and to then complex the resulting peptides to a metal ion, such as rhenium, resulting in a metallopeptide. Such a library may be a solid phase library, or may be a solution phase library.

A peptide library is first assembled based on the parent polypeptide, as described above, by well-known methods of peptide synthesis. Both solid-phase and soluble libraries can be obtained in this manner. The entire library is then reacted with an appropriate metal-complexing agent to obtain the corresponding metal-coordinated library, comprising a similar class of predetermined structures. For example, to complex a peptide library with rheniumoxo metal ion, the peptide library can be treated with Re(O)Cl₃(PPh₃)₂ in the presence of sodium acetate. This procedure results in quantitative complexation of ReO with the peptide. In order to complex Zn, Ni, Co, Mn, Fe or Cu ions, the peptide library is treated with chloride or other suitable salts of these metal ions to yield the library of corresponding metal ions. Essentially, a variety of metal ions can be used to construct different metallopeptide libraries. One limiting factor in selection of the appropriate metal ion is the relative stability of a particular metal-peptide complex, related in large part to the metal-peptide complex binding constant or constants. It is well known in the art that some metal-peptide constructs are stable only within specified pH or other special conditions, or are easily oxidized in air. Other peptide-metal ion complexes, such as those with ReO, are stable in pure form and can be isolated and stored under normal storage conditions for a long period of time.

In a preferred embodiment a solid-phase methodology is employed for the synthesis of metallopeptides, in which the metal ion complexation is also achieved while the peptide is on the solid phase. Using Fmoc chemistry a linear peptide is fully assembled on rink amide resin using a S^{t}Bu protected Cys derivative. Following synthesis of the peptide, the S^{t}Bu group is removed by treatment with Bu₃P in DMF. The resulting free -SH containing peptide-resin is treated with the rhenium transfer reagent ReO[V]Cl₃(PPh₃)₂ in presence of DBU as base. Complete metal-ion complexation is achieved within 2 hours at room temperature. The resulting metallopeptide resin is washed, dried and then treated with TFA to cleave the metallopeptide from the resin and remove all side chain protecting groups. The metallopeptide is purified by HPLC and characterized by mass spectrometry and amino acid analysis.

The invention is further illustrated by the following non-limiting example:

### Example 1

The amino terminal fragment (ATF) of urokinase-type tissue plasminogen activator (uPA) protein is sufficient for binding to the uPA receptor. In particular, the binding capability has been demonstrated to be within the omega loop composed of the 21-30 amino acid sequence of ATF that is encased within a Cys-Cys disulfide bridge. An N- and C-terminally capped 11-amino acid peptide corresponding to this omega loop sequence was selected for making a series of Re-complexed metallopeptides to determine the structure and location of the biologically relevant reversed turn structure within this sequence. The parent polypeptide, here a parent peptide, with the sequence Ac-Val-Ser-Asn-Lys-Tyr-Phe-Ser-Asn-Ile-His-Trp-NH₂ (SEQ ID NO:2) was subjected to a series of systematic Cys insertions starting after the 2 position and to the n+1 position, where n was the number of residues in the parent peptide. A series of ten peptides were synthesized by standard methods of solid-phase peptide synthesis. The -SH group of Cys was protected with an orthogonal S-^{t}Bu group. After the compete assembly of each individual peptide on resin the S-^{t}Bu group was removed by treatment with tributylphosphine and the peptide resin then treated with the Re-oxo transfer agent Re(O)Cl₃(PPh₃)₂ in the presence of DBU to form a metallopeptide. The peptide resin was then treated with TFA to cleave the resulting metallopeptide from the resin. The metallopeptides were purified by high precision liquid chromatography (HPLC) and assayed in receptor-binding assay using U937 cells and ATF as the competitive receptor binding ligand. The data presented in **Table 1** shows that the peptide Ac-Val-Ser-Asn-Lys-Tyr-Phe-Ser-Asn-Ile-His-Cys-Trp-NH₂ (SEQ ID NO:3) bound to a rhenium ion to form a metallopeptide was the most potent of all these molecules, and signified location of BD around the Ile-His-Cys-Trp fragment of the peptide. Other compounds in the table presented turn structures that were not associated with the pharmacophore involved with the uPA receptor binding. This series of ten systematically synthesized molecules was therefore sufficient to delineate the location of the turn segment in this peptide fragment. In **Table 1**, the assignments of R₁, R₂, R₃ and R₄ are as shown in the template of **Fig. 11**.

| **Table 1** | | | | | |
|---|---|---|---|---|---|
| **uPA Receptor Binding Data Showing Percentage Inhibition Of Binding of Metallopeptides with the Defined Amino Sequence** | | | | | |
| **R₁** | **R₂** | **R₃** | **Cys** | **R₄** | **% Inhibition at 1 µM** |
| Ac- | Val | Ser- | Cys- | Asn-Lys-Tyr-Phe-Ser-Asn-Ile-His-Trp-NH₂ (SEQ ID NO:4) | **0.0** |
| Ac-Val- | Ser- | Asn | Cys- | Lys-Tyr-Phe-Ser-Asn-Ile-His-Trp-NH₂ (SEQ ID NO:5) | **0.0** |
| Ac-Val-Ser- | Asn- | Lys- | Cys- | Tyr-Phe-Ser-Asn-Ile-His-Trp-NH₂ (SEQ ID NO:6) | **13.0** |
| Ac-Val-Ser-Asn- | Lys- | Tyr- | Cys- | Phe-Ser-Asn-Ile-His-Trp-NH₂ (SEQ ID NO:7) | **18.0** |
| Ac-Val-Ser-Asn-Lys- | Tyr- | Phe | Cys- | Ser-Asn-Ile-His-Trp-NH₂ (SEQ ID NO:8) | **0.0** |
| Ac-Val-Ser-Asn-Lys-Tyr- | Phe- | Ser- | Cys- | Asn-Ile-His-Trp-NH₂ (SEQ ID NO:9) | **35.0** |
| Ac-Val-Ser-Asn-Lys-Tyr-Phe- | Ser- | Asn- | Cys- | Ile-His-Trp-NH₂ (SEQ ID NO:10) | **32.0** |
| Ac-Val-Ser-Asn-Lys-Tyr-Phe-Ser- | Asn- | Ile- | Cys- | His-Trp-NH₂ (SEQ ID NO:11) | **0.0** |
| Ac-Val-Ser-Asn-Lys-Tyr-Phe-Ser-Asn- | Ile- | His- | Cys- | Trp-NH₂ (SEQ ID NO:3) | **106.0** |
| Ac-Val-Ser-Asn-Lys-Tyr-Phe-Ser-Asn-lie- | His- | Trp- | Cys-NH₂ | (SEQ ID NO:12) | **0.0** |

### Example 2

Ac-Nle-Ala-His-D-Phe-Arg-Trp-NH₂ is a known receptor-binding sequence for melanotropin receptors. The K, values for binding to MCR-1 and -4 were measured to be 0.1 µM and 2 µM respectively. A series of metallopeptides based on this sequence were synthesized by inserting a Cys residue after the 2 position and through the n+1 position and complexing the resulting Cys-containing peptide with an Re-oxo metal ion core as described Example 1. The resulting metallopeptides were screened for inhibiting the binding of 125-I-NDP-alpha-MSH radioligand using B-16 mouse melanoma cells for MCR-1 and cloned human MCR-2, -3 and -4 receptor transfected 293 cells.

The competitive inhibition binding assay was conducted using membranes prepared from hMC3-R, hMC4-R, hMC5-R, and B-16 mouse melanoma cells (containing MC1-R) using 0.4 nM ¹²⁵I-NDP-alpha-MSH (New England Nuclear, Boston, MA, USA) in 50 mM HEPES buffer containing 1 mM MgCl₂, 2 mM CaCl₂, and 5 mM KCI, at pH 7.2. The assay tube also contained a chosen concentration of the test peptide of this invention, complexed to a rhenium metal ion as indicated, for determining its efficacy in inhibiting the binding of ¹²⁵I-NDP-alpha-MSH to its receptor. Non-specific binding was measured by complete inhibition of binding of ¹²⁵I-NDP-alpha-MSH in the assay with the presence of 1 µM alpha-MSH. Incubation was for 90 minutes at room temperature, after which the assay mixture was filtered and the membranes washed three times with ice cold buffer. The filter was dried and counted in a gamma counter for remaining radioactivity bound to the membranes. 100% specific binding was defined as the difference in radioactivity (cpm) bound to cell membranes in the absence and presence of 1 µM alpha-MSH. The cpm obtained in presence of test compounds were normalized with respect to 100% specific binding to determine the percent inhibition of ¹²⁵I-NDP-alpha MSH binding. Each assay was conducted in triplicate and the actual mean valves are described in **Table 2**.

The data is presented in **Table 2**. It was evident that the metallopeptide Ac-Nle-Ala-His-D-Phe-Arg-Cys-Trp-NH₂ presented a conformationally constrained structure obtained by the complexation of the rheniumoxo metal ion, which structure was a BD specific for the MCR-1 receptor but not the MCR-4 receptor. The locus of this structural motif was included in the D-Phe-Arg-Cys-Trp sequence. This turn motif therefore led to the development of a potent MCR-1 specific ligand. The constrained structural motif with the D-Phe-Arg-Trp-Cys sequence locus, Ac-Nle-Ala-His-D-Phe-Arg-Trp-Cys-NH₂, presented a pharmacophore for binding both the MCR-1 and MCR-4 receptors. In **Table 1**, the assignments of R₁, R₂, R₃ and R₄ are as shown in the template of **Fig. 11**.

| **Table 2** | | | | | | | | |
|---|---|---|---|---|---|---|---|---|
| **Melanocortin Receptor Binding Data Showing Percentage Inhibition Of Binding of Metallopeptides with the Defined Amino Sequence** | | | | | | | | |
| **R₁** | **R₂** | **R₃** | **Cys** | **R₄** | **% Inhibition at 1 µM** | | | |
| | | | | | **MC-1** | **MC-3** | **MC-4** | **MC-5** |
| Ac- | Nle- | Ala- | Cys- | His-D-Phe-Arg-Trp-NH₂ | 96 | 47 | 71 | 64 |
| Ac-Nle- | Ala- | His- | Cys- | D-Phe-Arg-Trp-NH₂ | 84 | 9 | 75 | 58 |
| Ac-Nle-Ala- | His- | D-Phe- | Cys- | Arg-Trp-NH₂ | 93 | 15 | 66 | 57 |
| Ac-Nle-Ala-His- | D-Phe- | Arg- | Cys- | Trp-NH₂ | 96 | 0 | 17 | 0 |
| Ac-Nle-Ala-His-D-Phe- | Arg- | Trp- | Cys- | NH₂ | 91 | 70 | 98 | 93 |

### Example 3

*Alzheimer's and Prion Diseases.* Alzheimer's and prion diseases, such as Creutzfeldt-Jakob disease and related prion-driven diseases, are disorders of protein conformation. These are neurodegenerative diseases that lead to dementia. In most cases the disease is due to a set of conformational changes in the respective disease associated protein, amyloid-β in the case of Alzheimer's disease, and glycoprotein PrP^{SC} in the case of prion disease, which results in high level of beta-sheet structural motif. It has been shown that a peptide related to a specific sequence of respective protein with the additional ability to destabilize the formation of the beta sheet and capable of binding to the disease state protein conformer may serve as a useful therapeutic to halt progression of the disease, and may even effect its reversal. Other researchers have developed a series of linear peptides that have shown specific binding to the disease state protein and show promise of their therapeutic potential. See, for example, Soto C: Plaque busters: Strategies to inhibit amyloid formation in Alzheimer's Disease. Mol. Medicine Today, 5: 343-350 (1999); Soto C. et al.: Beta-sheet breaker peptides inhibit fibrillogenesis in a rat brain model of amyloidosis: Implications for Alzheimer's therapy. Nature Medicine, 4: 822-826 (1998); Soto C: Alzheimer's and prion disease as disorders of protein conformation: Implications for the design of novel therapeutic approaches. J. Mol. Med., 77: 412-418 (1999); and Soto C et al.: Reversion of prion protein conformational changes by synthetic beta-sheet breaker peptides. The Lancet, 355: 192-197 (2000).

The metallopeptides of this invention may used to conformationally restrict a portion of the parent polypeptide based on the co-ordination of Re metal ion to at least a portion of the amino acid sequence thereof. The resulting metallopeptide is proteolytically stable and is generally relatively more hydrophobic than the corresponding parent peptide. A base metallopeptide template may also be decorated with appropriate side chain functionalities to generate topographies that mimic the bioactive topography of a natural peptide, for example, peptides related to Alzheimer's and prion disease.

*Representative Alzheimer's Disease Peptides of the Invention*. The 17-20 hydrophobic region peptide (LVFF) serves in part as a template for developing specific beta-sheet breaker peptides. The linear peptide sequences of **Table 3** are used as a starting template for rational design of peptide sequences which, when bound to a metal ion such as rhenium, form a metallopeptide.

**Table 3**

| **Amyloid Beta-Protein Related Peptides for Treatment of AD** |
|---|
| His-Gln-Lys-Leu-Val-Phe-Phe-Ala-Glu-Asp-Val (SEQ ID NO:13) |
| Ac-Leu-Ala-Phe-Phe-Asp-NH₂ (SEQ ID NO:14) |
| Ac-Leu-Pro-Phe-Phe-Asp-NH₂ (SEQ ID NO: 15) |

The parent polypeptides or peptides described in **Table 3** can be employed as the template basis for synthesizing a series of metallopeptides, using the methods and constructs of this invention, with either L-Cys or D-Cys. In the practice of this invention, an N₁S₁ residue is employed, such as cysteine, which may be either L-Cys or D-Cys. Peptides are constructed using standard peptide synthesis techniques, in which the cysteine is inserted at selected points. The -SH group of Cys may be protected using an orthogonal protecting agent as set forth above. The resulting Cys-containing peptides are then deprotected, and subsequently complexed with a rhenium ion, forming a metallopeptide, using a suitable pre-formed metal-oxo transfer agent such as Re(O)Cl₃(PPh₃)₂. Through use of competitive inhibition assays, the binding of each of the resulting metallopeptides is compared against the parent peptide, and those with enhanced or increased binding are identified.

Utilizing this approach, a series of initial and precursor metallopeptides are defined as set forth in **Table 4**.

**Table 4**

| **Precursor Metallopeptides for AD** |
|---|
| R₁-His-Gln-Lys-Leu-Aaa-Phe-Phe-Ala-Glu-Asp-Val-Cys-R₂ (SEQ ID NO:16) |
| R₁-His-Gln-Lys-Leu-Aaa-Phe-Phe-Ala-Glu-Asp-Cys-Val-R₂ (SEQ ID NO:17) |
| R₁-His-Gln-Lys-Leu-Aaa-Phe-Phe-Ala-Glu-Cys-Asp-Val-R₂ (SEQ ID NO:18) |
| R₁-His-Gln-Lys-Leu-Aaa-Phe-Phe-Ala-Cys-Glu-Asp-Val-R₂ (SEQ ID NO:19) |
| R₁-His-Gln-Lys-Leu-Aaa-Phe-Phe-Cys-Ala-Glu-Asp-Val-R₂ (SEQ ID NO:20) |
| R₁-His-Gln-Lys-Leu-Bbb-Phe-Phe-Cys-Ala-Glu-Asp-Val-R₂ (SEQ ID NO:21) |
| R₁-His-Gln-Lys-Leu-Bbb-Phe-Cys-Phe-Ala-Glu-Asp-Val-R₂ (SEQ ID NO:22) |
| R₁-His-Gln-Lys-Leu-Bbb-Cys-Phe-Phe-Ala-Glu-Asp-Val-R₂ (SEQ ID NO:23) |
| R₁-His-Gln-Lys-Leu-Cys-Aaa-Phe-Phe-Ala-Glu-Asp-Val-R₂ (SEQ ID NO:24) |
| R₁-His-Gln-Lys-Cys-Leu-Aaa-Phe-Phe-Ala-Glu-Asp-Val-R₂ (SEQ ID NO:25) |
| R₁-His-Gln-Cys-Lys-Leu-Aaa-Phe-Phe-Ala-Glu-Asp-Val-R₂ (SEQ ID NO:26) |

Where:
R₁ is H (N-terminus is free amino group) or Ac (Acetyl group at N-terminus);
R₂ is OH (free carboxylate at C-terminus) or NH₂ (C-terminal is amide group);
Aaa is Val, Pro, Gly or Ala;
Bbb is Val, Gly or Ala;
Cys is either L-Cys or D-Cys; and
the three amino acid residues preceding Cys and the one amino acid immediately following Cys are either L-amino acid residues or D-amino acid residues, or any combination thereof.

The following series of peptides are derived from the series of peptides of **Table 4**. In each of these series the length of peptide is shortened successively either from the N- or the C-termini, or both. In the following series (**Table 5** through **Table 14**), R₁, R₂, Aaa, Bbb and Cys are as defined, with the three amino acid residues preceding Cys and the one amino acid immediately following Cys either L-amino acid residues or D-amino acid residues, or any combination thereof.

**Table 5**

| | |
|---|---|
| | R₃-Asp-Val-Cys-R₂ |
| where R₃ is | R₁-Gln-Lys-Leu-Aaa-Phe-Phe-Ala-Glu, (SEQ ID NO:27) |
| | R₁-Lys-Leu-Aaa-Phe-Phe-Ala-Glu, (SEQ ID NO:28) |
| | R₁-Leu-Aaa-Phe-Phe-Ala-Glu, (SEQ ID NO:29) |
| | R₁-Aaa-Phe-Phe-Ala-Glu, (SEQ ID NO:30) |
| | R₁-Phe-Phe-Ala-Glu, (SEQ ID NO:31) |
| | R₁-Phe-Ala-Glu, |
| | R₁-Ala-Glu, |
| | R₁-Glu, or |
| | R₁. |

**Table 6**

| | |
|---|---|
| | R₄-Glu-Asp-Cys-R₅ |
| where R₄ is | R₁-His-Gln-Lys-Leu-Aaa-Phe-Phe-Ala, (SEQ ID NO:32) |
| | R₁-Gln-Lys-Leu-Aaa-Phe-Phe-Ala, (SEQ ID NO:33) |
| | R₁-Lys-Leu-Aaa-Phe-Phe-Ala, (SEQ ID NO:34) |
| | R₁-Leu-Aaa-Phe-Phe-Ala, (SEQ ID NO:35) |
| | R₁-Aaa-Phe-Phe-Ala, |
| | R₁-Phe-Phe-Ala, |
| | R₁-Phe-Ala, |
| | R₁-Ala, or |
| | R₁; |
| and R₅ is | Val-R₂, or |
| | R₂. |

**Table 7**

| | |
|---|---|
| | R₆-Ala-Glu-Cys-R₇ |
| where R₆ is | R₁-His-Gln-Lys-Leu-Aaa-Phe-Phe, (SEQ ID NO:36) |
| | R₁-Gln-Lys-Leu-Aaa-Phe-Phe, (SEQ ID NO:37) |
| | R,-Lys-Leu-Aaa-Phe-Phe, (SEQ ID NO:38) |
| | R₁-Leu-Aaa-Phe-Phe, |
| | R₁-Aaa-Phe-Phe, |
| | R₁-Phe-Phe, |
| | R₁-Phe, or |
| | R₁; |
| and R₇ is | Asp-Val-R₂, |
| | Asp-R_{2,} or |
| | R₂. |

**Table 8**

| | |
|---|---|
| | R₈-Phe-Ala-Cys-R₉ |
| where R₈ is | R₁-His-Gln-Lys-Leu-Aaa-Phe, (SEQ ID NO:39) |
| | R₁-Gln-Lys-Leu-Aaa-Phe, (SEQ ID NO:40) |
| | R₁-Lys-Leu-Aaa-Phe, |
| | R₁-Leu-Aaa-Phe, |
| | R₁-Aaa-Phe, |
| | R₁-Phe, or |
| | R₁; |
| and R₉ is | Glu-Asp-Val-R₂, |
| | Glu-Asp-R₂, |
| | Glu-R₂, or |
| | R₂. |

**Table 9**

| | |
|---|---|
| | R₉-Phe-Phe-Cys-R₁₀ |
| where R₉ is | R₁-His-Gln-Lys-Leu-Aaa, (SEQ ID NO:41) |
| | R₁-Gln-Lys-Leu-Aaa, |
| | R₁-Lys-Leu-Aaa, |
| | R,-Leu-Aaa, |
| | R₁-Aaa, |
| | R₁, |
| | R₁-His-Gln-Lys-Leu-Bbb, (SEQ ID NO:42) |
| | R₁-Gln-Lys-Leu-Bbb, |
| | R₁-Lys-Leu-Bbb, |
| | R₁-Leu-Bbb, or |
| | R₁-Bbb; |
| and R₁₀ is | Ala-Glu-Asp-Val-R₂, (SEQ ID NO:43) |
| | Ala-Glu-Asp-R₂, |
| | Ala-Glu-R₂, |
| | Glu-R₂, or |
| | R₂. |

**Table 10**

| | |
|---|---|
| | R₁₁-Bbb-Phe-Cys-R₁₂ |
| where R₁₁ is | R₁-His-Gln-Lys-Leu, (SEQ ID NO:44) |
| | R₁-Gln-Lys-Leu, |
| | R₁-Lys-Leu, |
| | R₁-Leu, or |
| | R₁; |
| and R₁₂ is | Phe-Ala-Glu-Asp-Val-R₂, (SEQ ID NO:45) |
| | Phe-Ala-Glu-Asp-R₂, (SEQ ID NO:46) |
| | Phe-Ala-Glu-R₂, |
| | Phe-Glu-R₂, |
| | Phe-R₂, or |
| | R₂. |

**Table 11**

| | |
|---|---|
| | R₁₃-Leu-Bbb-Cys-R₁₄ |
| where R₁₃ is | R₁-His-Gln-Lys, |
| | R₁-Gln-Lys, |
| | R₁-Lys, or |
| | R₁; |
| and R₁₄ is | Phe-Phe-Ala-Glu-Asp-Val-R₂, (SEQ ID NO:47) |
| | Phe-Phe-Ala-Glu-Asp-R₂, (SEQ ID NO:48) |
| | Phe-Phe-Ala-Glu-R₂, (SEQ ID NO:49) |
| | Phe-Phe-Glu-R₂, |
| | Phe-Phe-R₂, |
| | Phe-R₂, or |
| | R₂. |

**Table 12**

| | |
|---|---|
| | R₁₅-Lys-Leu-Cys-R₁₆ |
| where R₁₅ is | R₁-His-Gln, |
| | R₁-Gln, or |
| | R₁; |
| and R₁₆ is | Aaa-Phe-Phe-Ala-Glu-Asp-Val-R₂, (SEQ ID NO:50) |
| | Aaa-Phe-Phe-Ala-Glu-Asp-R₂, (SEQ ID NO:51) |
| | Aaa-Phe-Phe-Ala-Glu-R₂, (SEQ ID NO:52) |
| | Aaa-Phe-Phe-Glu-R₂, |
| | Aaa-Phe-Phe-R₂, |
| | Aaa-Phe-R₂, |
| | Aaa-R₂, |
| | R₂, |
| | Bbb-Phe-Phe-Ala-Glu-Asp-Val-R₂, (SEQ ID NO:53) |
| | Bbb-Phe-Phe-Ala-Glu-Asp-R₂, (SEQ ID NO:54) |
| | Bbb-Phe-Phe-Ala-Glu-R₂, (SEQ ID NO:55) |
| | Bbb-Phe-Phe-Glu-R₂, |
| | Bbb-Phe-Phe-R₂, |
| | Bbb-Phe-R₂, or |
| | Bbb-R₂. |

**Table 13**

| | |
|---|---|
| | R₁₇-His-Gln-Lys-Cys-R₁₈ (SEQ ID NO:56) |
| where R₁₇ is | R₁-His or |
| | R₁; |
| and R₁₈ is | Leu-Aaa-Phe-Phe-Ala-Glu-Asp-Val-R₂, (SEQ ID NO:57) |
| | Leu-Aaa-Phe-Phe-Ala-Glu-Asp-R₂, (SEQ ID NO:58) |
| | Leu-Aaa-Phe-Phe-Ala-Glu-R₂, (SEQ ID NO:59) |
| | Leu-Aaa-Phe-Phe-Glu-R₂, (SEQ ID NO:60) |
| | Leu-Aaa-Phe-Phe-R₂, |
| | Leu-Aaa-Phe-R₂, |
| | Leu-Aaa-R₂, |
| | Leu-R₂, or |
| | R₂. |

**Table 14**

| | |
|---|---|
| | R₁-His-Gln-Cys-R₁₉ |
| where R₁₉ is | Lys-Leu-Aaa-Phe-Phe-Ala-Glu-Asp-Val-R₂, (SEQ ID NO:61) |
| | Lys-Leu-Aaa-Phe-Phe-Ala-Glu-Asp-R₂, (SEQ ID NO:62) |
| | Lys-Leu-Aaa-Phe-Phe-Ala-Glu-R₂, (SEQ ID NO:63) |
| | Lys-Leu-Aaa-Phe-Phe-Glu-R₂, (SEQ ID NO:64) |
| | Lys-Leu-Aaa-Phe-Phe-R₂, (SEQ ID NO:65) |
| | Lys-Leu-Aaa-Phe-R₂, |
| | Lys-Leu-Aaa-R₂, |
| | Lys-Leu-R₂, |
| | Lys-R₂, or |
| | R₂. |

*Representative Prion Disease Peptides of the Invention.* In another embodiment of this invention, peptides are provided for use in treatment of prion disease, including but not limited to Creutzfeldt-Jakob disease, variant Creutzfeldt-Jakob disease and related prion driven disorders. The linear peptide sequence of **Table 15** is used as a parent peptide for the rational design of peptide sequences which, when bound to a metal ion such as rhenium, form a metallopeptide.

**Table 15**

| |
|---|
| Asp-Ala-Pro-Ala-Ala-Pro-Ala-Gly-Pro-Ala-Val-Pro-Val (SEQ ID NO:66) |

The parent peptide described in **Table 15** can be employed as the template basis for synthesizing a series of metallopeptides, using the methods and constructs of this invention, with either L-Cys or D-Cys. In the practice of this invention, an N₁S₁ residue is employed, such as cysteine, which may be either L-Cys or D-Cys. Peptides are constructed using standard peptide synthesis techniques, in which the cysteine is inserted at selected points. The -SH group of Cys may be protected using an orthogonal protecting agent as set forth above. The resulting Cys-containing peptides are then deprotected, and subsequently complexed with a rhenium ion, forming a metallopeptide, using a suitable pre-formed metal-oxo transfer agent such as Re(O)Cl₃(PPh₃)₂. Through use of competitive inhibition assays, the binding of each of the resulting metallopeptides is compared against the parent peptide, and those with enhanced or increased binding are identified.

Utilizing this approach, a series of precursor molecules are defined as set forth in **Table 16**.

**Table 16**

| **Precursor Metallopeptides for Prion Disease** |
|---|
| S₁-Asp-Ala-Pro-Ala-Ala-Pro-Ala-Gly-Pro-Ala-Val-Aaa-Val-Cys-S₂ (SEQ ID NO:67) |
| S₁-Asp-Ala-Pro-Ala-Ala-Pro-Ala-Gly-Pro-Ala-Val-Aaa-Cys-Val-S₂ (SEQ ID NO:68) |
| S₁-Asp-Ala-Pro-Ala-Ala-Pro-Ala-Gly-Bbb-Ala-Val-Cys-Bbb-Val-S₂ (SEQ ID NO:69) |
| S₁-Asp-Ala-Pro-Ala-Ala-Pro-Ala-Gly-Aaa-Ala-Cys-Val-Pro-Val-S₂ (SEQ ID NO:70) |
| S₁-Asp-Ala-Pro-Ala-Ala-Pro-Ala-Gly-Aaa-Cys-Ala-Val-Pro-Val-S₂ (SEQ ID NO:71) |
| S₁-Asp-Ala-Pro-Ala-Ala-Bbb-Ala-Gly-Cys-Bbb-Ala-Val-Pro-Val-S₂ (SEQ ID NO:72) |
| S₁-Asp-Ala-Pro-Ala-Ala-Aaa-Ala-Cys-Gly-Pro-Ala-Val-Pro-Val-S₂ (SEQ ID NO:73) |
| S₁-Asp-Ala-Pro-Ala-Ala-Aaa-Cys-Ala-Gly-Pro-Ala-Val-Pro-Val-S₂ (SEQ ID NO:74) |
| S₁-Asp-Ala-Bbb-Ala-Ala-Cys-Bbb-Ala-Gly-Pro-AJa-Val-Pro-Val-S₂ (SEQ ID NO:75) |
| S₁-Asp-Ala-Aaa-Ala-Cys-Ala-Pro-Ala-Gly-Pro-Ala-Val-Pro-Val-S₂ (SEQ ID NO:76) |
| S₁-Asp-Ala-Aaa-Cys-Ala-Ala-Pro-Ala-Gly-Pro-Ala-Val-Pro-Val-S₂ (SEQ ID NO:77) |
| S₁-Asp-Ala-Cys-Bbb-Ala-Ala-Pro-Ala-Gly-Pro-Ala-Val-Pro-Val-S₂ (SEQ ID NO:78) |

where:
S₁ is H (N-terminus is free amino group) or Ac (Acetyl group at N-terminus);
S₂ is OH (free carboxylate at C-terminus) or NH₂ (C-terminal is amide group);
Aaa is Gly or Ala;
Bbb is Pro, Gly or Ala; and
the three amino acid residues preceding Cys and the one amino acid immediately following Cys are either L-amino acid residues or D-amino acid residues, or any combination thereof.

The following series of peptides are derived from the series of peptides of **Table 16**. In each of these series the length of peptide is shortened successively either from the N- or the C-termini, or both. In the following series (**Table 17** through **Table 28**), S₁, S₂, Aaa, Bbb and Cys are as defined, with the three amino acid residues preceding Cys and the one amino acid immediately following Cys either L-amino acid residues or D-amino acid residues, or any combination thereof.

**Table 17**

| | |
|---|---|
| | S₃-Aaa-Val-Cys-S₂ |
| where S₃ is | S,-Asp-Ala-Pro-Ala-Ala-Pro-Ala-Gly-Pro-Ala-Val, (SEQ ID NO:79) |
| | S₁-Ala-Pro-Ala-Ala-Pro-Ala-Gly-Pro-Ala-Val, (SEQ ID NO:80) |
| | S₁-Pro-Ala-Ala-Pro-Ala-Gly-Pro-Ala-Val, (SEQ ID NO:81) |
| | S₁-Ala-Ala-Pro-Ala-Gly-Pro-Ala-Val, (SEQ ID NO:82) |
| | S₁-Ala-Pro-Ala-Gly-Pro-Ala-Val, (SEQ ID NO:83) |
| | S₁-Pro-Ala-Gly-Pro-Ala-Val, (SEQ ID NO:84) |
| | S₁-Ala-Gly-Pro-Ala-Val, (SEQ ID NO:85) |
| | S₁-Gly-Pro-Ala-Val, (SEQ ID NO:86) |
| | S₁-Pro-Ala-Val, |
| | S₁-Ala-Val, |
| | S,-Val, or |
| | S₁. |

**Table 18**

| | |
|---|---|
| | S₄-Val-Aaa-Cys-S₅ |
| where S₄ is | S₁-Asp-Ala-Pro-Ala-Ala-Pro-Ala-Gly-Pro-Ala, (SEQ ID NO:87) |
| | S₁-Ala-Pro-Ala-Ala-Pro-Ala-Gly-Pro-Ala, (SEQ ID NO:88) |
| | S₁-Pro-Ala-Ala-Pro-Ala-Gly-Pro-Ala, (SEQ ID NO:89) |
| | S₁-Ala-Ala-Pro-Ala-Gly-Pro-Ala, (SEQ ID NO:90) |
| | S₁-Ala-Pro-Ala-Gly-Pro-Ala, (SEQ ID NO:91) |
| | S₁-Pro-Ala-Gly-Pro-Ala, (SEQ ID NO:92) |
| | S₁-Ala-Gly-Pro-Ala, (SEQ ID NO:93) |
| | S₁-Gly-Pro-Ala, |
| | S₁-Pro-Ala, |
| | S₁-Ala, or |
| | S₁; |
| and S₅ is | Val-S₂ or |
| | S₂. |

**Table 19**

| | |
|---|---|
| | S₆-Ala-Val-Cys-S₇ |
| where S₆ is | S₁-Asp-Ala-Pro-Ala-Ala-Pro-Ala-Gly-Bbb, (SEQ ID NO:94) |
| | S₁-Ala-Pro-Ala-Ala-Pro-Ala-Gly-Bbb, (SEQ ID NO:95) |
| | S₁-Pro-Ala-Ala-Pro-Ala-Gly-Bbb, (SEQ ID NO:96) |
| | S₁-Ala-Ala-Pro-Ala-Gly-Bbb, (SEQ ID NO:97) |
| | S₁-Ala-Pro-Ala-Gly-Bbb, (SEQ ID NO:98) |
| | S₁-Pro-Ala-Gly-Bbb, |
| | S₁-Ala-Gly-Bbb, |
| | S₁-Gly-Bbb, |
| | S₁-Bbb, or |
| | S₁; |
| and S₇ is | Bbb-Val-S₂, |
| | Val-S₂, or |
| | S₂. |

**Table 20**

| | |
|---|---|
| | S₈-Aaa-Ala-Cys-S₉ |
| where S₈ is | S,-Asp-Ala-Pro-Ala-Ala-Pro-Ala-Gly, (SEQ ID NO:99) |
| | S₁-Ala-Pro-Ala-Ala-Pro-Ala-Gly, (SEQ ID NO:100) |
| | S₁-Pro-Ala-Ala-Pro-Ala-Gly, (SEQ ID NO:101) |
| | S₁-Ala-Ala-Pro-Ala-Gly, (SEQ ID NO:102) |
| | S₁-Ala-Pro-Ala-Gly, (SEQ ID NO:103) |
| | S₁-Pro-Ala-Gly, |
| | S,-Ala-Gly, |
| | S₁-Gly, or |
| | S₁; |
| and Sg is | Val-Pro-Val-S₂, |
| | Val-Pro-S₂, |
| | Val-S₂, or |
| | S₂. |

**Table 21**

| | |
|---|---|
| | S₁₀-Gly-Aaa-Cys-Ala-Val-Pro-Val-S₁₁, (SEQ ID NO:104) |
| where S₁₀ is | S₁-Asp-Ala-Pro-Ala-Ala-Pro-Ala, (SEQ ID NO:105) |
| | S₁-Ala-Pro-Ala-Ala-Pro-Ala, (SEQ ID NO:106) |
| | S₁-Pro-Ala-Ala-Pro-Ala, (SEQ ID NO:107) |
| | S₁-Ala-Ala-Pro-Ala, (SEQ ID NO:108) |
| | S₁-Ala-Pro-Ala, |
| | S₁-Pro-Ala, |
| | S₁-Ala, or |
| | S₁; |
| and S₁₁ is | Ala-Val-Pro-Val-S₂, (SEQ ID NO:109) |
| | Ala-Val-Pro-S₂, |
| | Ala-Val-S₂, |
| | Ala-S₂, or |
| | S₂. |

**Table 22**

| | |
|---|---|
| | S₁₂-Ala-Gly-Cys-Bbb-S₁₃ |
| where S₁₂ is | S₁-Asp-Ala-Pro-Ala-Ala-Bbb, (SEQ ID NO:110) |
| | S₁-Ala-Pro-Ala-Ala-Bbb, (SEQ ID NO:111) |
| | S₁-Pro-Ala-Ala-Bbb, |
| | S₁-Ala-Ala-Bbb, |
| | S₁-Ala-Bbb, |
| | S₁-Bbb, or |
| | S₁; |
| and S₁₃ is | Bbb-Ala-Val-Pro-Val-S₂, |
| | Bbb-Ala-Val-Pro-S₂, |
| | Bbb-Ala-Val-S₂, |
| | Bbb-Ala-S₂, |
| | Bbb-S₂, or |
| | S₂. |

**Table 23**

| | |
|---|---|
| | S₁₄-Aaa-Ala-Cys-S₁₅ |
| where S₁₄ is | S₁-Asp-Ala-Pro-Ala-Ala, (SEQ ID NO:112) |
| | S₁-Ala-Pro-Ala-Ala, (SEQ ID NO:113) |
| | S₁-Pro-Ala-Ala, |
| | S₁-Ala-Ala, |
| | S₁-Ala, or |
| | S₁; |
| and S₁₅ is | Gly-Pro-Ala-Val-Pro-Val-S₂, (SEQ ID NO:114) |
| | Gly-Pro-Ala-Val-Pro-S₂, (SEQ ID NO:115) |
| | Gly-Pro-Ala-Val-S₂, (SEQ ID NO:116) |
| | Gly-Pro-Ala-S₂, |
| | Gly-Pro-S₂, |
| | Gly-S₂, or |
| | S₂. |

**Table 24**

| | |
|---|---|
| | S₁₆-Ala-Aaa-Cys-S₁₇ |
| where S₁₆ is | S₁-Asp-Ala-Pro-Ala, (SEQ ID NO:117) |
| | S₁-Ala-Pro-Ala, |
| | S₁-Pro-Ala, |
| | S₁-Ala, or |
| | S₁; |
| and S₁₇ is | Ala-Gly-Pro-Ala-Val-Pro-Val-S₂, (SEQ ID NO:118) |
| | Ala-Gly-Pro-Ala-Val-Pro-S₂, (SEQ ID NO:119) |
| | Ala-Gly-Pro-Ala-Val-S₂, (SEQ ID NO:120) |
| | Ala-Gly-Pro-Ala-S₂, (SEQ ID NO:121) |
| | Ala-Gly-Pro-S₂, |
| | Ala-Gly-S₂, |
| | Ala-S₂, or |
| | S₂. |

**Table 25**

| | |
|---|---|
| | S₁₈-Ala-Ala-Cys-Bbb-S₁₉ |
| where S₁₈ is | S₁-Asp-Ala-Bbb, |
| | S₁-Ala-Bbb, |
| | S₁-Bbb, or |
| | S₁; |
| and S₁₉ is | Bbb-Ala-Gly-Pro-Ala-Val-Pro-Val-S₂, (SEQ ID NO:122) |
| | Bbb-Ala-Gly-Pro-Ala-Val-Pro-S₂, (SEQ ID NO:123) |
| | Bbb-Ala-Gly-Pro-Ala-Val-S₂, (SEQ ID NO:124) |
| | Bbb-Ala-Gly-Pro-Ala-S₂, (SEQ ID NO:125) |
| | Bbb-Ala-Gly-Pro-S₂, |
| | Bbb-Ala-Gly-S₂, |
| | Bbb-Ala-S₂, |
| | Bbb-S₂, or |
| | S₂. |

**Table 26**

| | |
|---|---|
| | S₂₀-Asp-Ala-Aaa-Ala-Cys- Ala-Gly-Pro-AJa-Val-Pro-Val-S₂₁ (SEQ ID NO:126) |
| where S₂₀ is | S₁-Asp-Ala, |
| | S₁-Ala, or |
| | S₁; |
| and S₂₁ is | Ala-Pro-Ala-Gly-Pro-Ala-Val-Pro-Val-S₂, (SEQ ID NO:127) |
| | Ala-Pro-Ala-Gly-Pro-Ala-Val-Pro-S₂, (SEQ ID NO:128) |
| | Ala-Pro-Ala-Gly-Pro-Ala-Val-S₂, (SEQ ID NO:129) |
| | Ala-Pro-Ala-Gly-Pro-Ala-S₂, (SEQ ID NO:130) |
| | Ala-Pro-Ala-Gly-PrO-S₂, (SEQ ID NO:131) |
| | Ala-Pro-Ala-Gly-S₂, (SEQ ID NO:132) |
| | Ala-Pro-Ala-S₂, |
| | Ala-Pro-S₂, |
| | Ala-S₂, or |
| | S₂. |

**Table 27**

| | |
|---|---|
| | S₂₂-Ala-Aaa-Cys-S₂₃ |
| where S₂₂ is | S₁-Asp or |
| | S₁; |
| and S₂₃ is | Ala-Ala-Pro-Ala-Gly-Pro-Ala-Val-Pro-Val-S₂, (SEQ ID NO:133) |
| | Ala-Ala-Pro-Ala-Gly-Pro-Ala-Val-Pro-S₂, (SEQ ID NO:134) |
| | Ala-Ala-Pro-Ala-Gly-Pro-Ala-Val-S₂, (SEQ ID NO:135) |
| | Ala-Ala-Pro-Ala-Gly-Pro-Ala-S₂, (SEQ ID NO:136) |
| | Ala-Ala-Pro-Ala-Gly-Pro-S₂, (SEQ ID NO:137) |
| | Ala-Ala-Pro-Ala-Gly-S₂, (SEQ ID NO:138) |
| | Ala-Ala-Pro-Ala-S₂, (SEQ ID NO:139) |
| | Ala-Ala-Pro-S₂, |
| | Ala-Ala-S₂, |
| | Ala-S₂, or |
| | S₂. |

**Table 28**

| | |
|---|---|
| | S₁-Asp-Ala-Cys-Bbb-Ala-Ala-Pro-Ala-Gly-Pro-Ala-Val-Pro-Val-S₂₄ (SEQ ID NO:140) |
| where S₂₄ is | Bbb-Ala-Ala-Pro-Ala-Gly-Pro-Ala-Val-Pro-Val-S₂, (SEQ ID NO:141) |
| | Bbb-Ala-Ala-Pro-Ala-Gly-Pro-Ala-Val-Pro-S₂, (SEQ ID NO:142) |
| | Bbb-Ala-Ala-Pro-Ala-Gly-Pro-Ala-Val-S₂, (SEQ ID NO:143) |
| | Bbb-Ala-Ala-Pro-Ala-Gly-Pro-Ala-S₂, (SEQ ID NO:144) |
| | Bbb-Ala-Ala-Pro-Ala-Gly-Pro-S₂, (SEQ ID NO:145) |
| | Bbb-Ala-Ala-Pro-Ala-Gly-S₂, (SEQ ID NO:146) |
| | Bbb-Ala-Ala-Pro-Ala-S₂, (SEQ ID NO:147) |
| | Bbb-Ala-Ala-Pro-S₂, |
| | Bbb-Ala-Ala-S₂, |
| | Bbb-Ala-S₂, |
| | Bbb-S₂, or |
| | S₂. |

### Example 4

A discrete library of peptides was developed based on the known vasopressin ligand Pmp-D-Trp-Ile-Thr-Dap-Cys-Pro-Orn, wherein Pmp is β-mercapto-β, β-cyclopentamethylenepropionyl and Dap is diaminopropionic acid (Chan WY et al.: Discovery and design of novel and selective vasopressin and oxytocin agonists and antagonists: the role of bioassays, Exp Physiol 85: Spec No:7S-18S, 2000). This ligand contains a disulfide bridge between the 1 and 6 residues. The endogenous Cys group was replaced by an Ala, like Cys a relatively small, neutral amino acid. By making such a substitution, the need to protect and the endogenous Cys in the n = 6 position was eliminated. Similarly, cyclohexylacetic acid was substituted in place of the N-terminal β-mercapto-β, β-cyclopentamethylenepropionyl group. By making such substitutions, the need to protect and subsequently deprotect the endogenous -SH groups of the two residues at the 1 and 6 position was eliminated. In addition, some compounds were made with different neutral N-terminal residues, such as cyclohexglycine (Chg), Pmp or D-Chg. The peptides were made as described generally in Example 1, and were complexed with rhenium as described therein. The resulting metallopeptides, shown in **Table 29** below, were then screened for activity.

The screening of metallopeptides for binding to oxytocin receptor was done using cell membranes prepared from rat uterus. A Millipore Multi-Screen System was used for the assay, and was performed in 96-well Millipore filter plates (Durapore, 0.45 µm porosity) freshly blocked with 0.5% bovine serum albumin in phosphate buffered saline (PBS). The membrane preparations (10 - 50 µg/well) were incubated with 412-800 pM ³H-oxytocin in HEPES Buffer containing 0.2% bovine serum albumin along with a test compound (1 µM final assay concentration) for 2 hours at 4° C. Non-specific binding was determined by addition of 10⁻⁶ M oxytocin instead of the test compound. After incubation, the membranes were filtered and washed three times with ice-cold PBS. The membranes were air-dried and punched directly into scintillation vials. After addition of the scintillation cocktail, the vials were capped and gently shaken for 12 hours to dissolve the radioactivity contained in the filters. The vials were then read for tritium counts in a scintillation counter. Specific binding was determined as the radioactivity in wells containing ³H-oxytocin alone minus the radioactivity in wells containing 10⁻⁶ M oxytocin. The assay was performed in triplicates. The activity profile for the test compounds were generated by their ability to inhibit specific binding of the radiotracer to its receptor.

The screening of compounds for vasopressin-1 receptor was performed using cell membranes prepared from rat liver. The assay was essentially performed as described above for the oxytocin receptor assay. In this assay 2-4 nM ³H-vasopressin-1 antagonist (obtained from Perkin Elemer- NEN Life Sciences) was used as the radiotracer and Arg⁸-vasopressin (1 µM final concentration in the assay) was used to determine non-specific binding. The assay was performed in triplicates. Activity profile for the test compounds were generated by their ability to inhibit specific binding of the radiotracer to its receptor.

| **Table 29** | | |
|---|---|---|
| **Peptide Sequences for Re Complexation to Form Metallopeptides and Percent Inhibit of Binding to Oxytocin and Vasopressin Receptors** | | |
| **Re Complexed Sequence** | **% Inhibition at 1 µM** | |
| | **Oxytocin Receptor** | **Vasopressin-1 Receptor** |
| Caca-D-Trp-Ile-Thr-Dap-Ala-*Ala-Orn-Cys*-NH₂ | 0 | 23 |
| Caca-D-Trp-Ile-Thr-Dap-*Ala-Ala-Cys*-Orn-NH₂ | 0 | 0 |
| Caca-D-Trp-Ile-Thr-*Dap-Ala-Cys*-Ala-Orn-NH₂ | 0 | 0 |
| Caca-D-Trp-Ile-Thr-*Dap-Ala-Cys*-Pro-Orn-NH₂ | 0 | 0 |
| Caca-D-Trp-Ile-*Thr-Dap-Cys*-Ala-Pro-Orn-NH₂ | 9 | 22 |
| Caca-D-Trp-*Ile-Thr-Cys*-Dap-Ala-Pro-Orn-NH₂ | 0 | 0 |
| Caca-D-*Trp-Ile-Cys*-Thr-Dap-Ala-Pro-Orn-NH₂ | 0 | 52 |
| *Pmp-D-Trp-Cys*-Ile-Thr-Dap-Ala-Pro-Orn-NH₂ | 0 | 23 |
| *Chg-D-Trp-Cys*-Ile-Thr-Dap-Ala-Pro-Orn-NH₂ | 0 | 0 |
| *D-Chg-D-Trp-Cys*-Ile-Thr-Dap-Ala-Pro-Orn-NH₂ | 0 | 8 |

In the foregoing table the MCD is in italics. "Caca" is cyclohexylacetic acid. It can be seen in **Table 29** that in the sequence Caca-D-Trp-Ile-Thr-Dap-Ala-Ala-Orn-Cys-NH₂ the Pro in the n = 7 position was substituted with an Ala, as is the case in the two succeeding sequences. Thereafter, Pro was utilized. This was done to investigate if the presence of a Pro next to constrained metal-peptide core caused any conformational perturbance. The data on this set of compounds clearly demonstrates that the compounds are selective for the vasopressin receptor and that one of these peptides has the maximal activity. It was remarkable to observe that this peptide is also constrained by metal complexation in the same region as the parent disulfide bridge-constrained peptide. However, in this case, the metal ion induced constraint identified a more specified pair of amino acid residues, D-Trp-lle, as the main residues structurally organized in a bioactive disposition. In the parent peptide, four amino acid residues, D-Trp-lle-Thr-Dap, are within the disulfide constraint. The metallopeptide approach, therefore, defined a more precise pharmacophore model.

### Example 5

A discrete library of peptides was developed based on the known natural oxytocin ligand Cys-Tyr-Ile-Gln-Asn-Cys-Pro-Leu-Gly-NH₂ (SEQ ID NO:148). This ligand contains an endogenous Cys at both the 1 and 6 positions. Both endogenous Cys groups were replaced by an Ala, like Cys a relatively small, neutral amino acid. By making such a substitution, the need to protect and subsequently deprotect the endogenous Cys in the 1 and 6 positions was eliminated. The peptides were made as described generally in Example 1, and were complexed with rhenium as described therein. The resulting metallopeptides, shown in **Table 30** below, were then screened for activity as described in Example 4.

| **Table 30** | | |
|---|---|---|
| **Peptide Sequences for Re Complexation to Form Metallopeptides and Percent Inhibit of Binding to Oxytocin and Vasopressin Receptors** | | |
| **Re Complexed Sequence** | **% Inhibition at 1 µM** | |
| | **Oxytocin Receptor** | **Vasopressin-1 Receptor** |
| Ala-Tyr-Ile-Gln-Asn-Ala-Pro-*Leu-Gly-Cys*-NH₂ (SEQ ID NO:149) | 6 | 0 |
| Ala-Tyr-Ile-Gln-Asn-Ala-Ala-*Leu-Gly-Cys*-NH₂ (SEQ ID NO:150) | 0 | 0 |
| Ala-Tyr-Ile-Gln-Asn-Ala-*Ala-Leu-Cys*-Gly-NH₂ (SEQ ID NO:151) | 0 | 0 |
| Ala-Tyr-Ile-Gln-Asn-*Ala-Ala-Cys*-Leu-Gly-NH₂ (SEQ ID NO:152) | 0 | 1 |
| Ala-Tyr-Ile-Gln-*Asn-Ala-Cys*-Pro-Leu-Gly-NH₂ (SEQ ID NO:153) | 2 | 0 |
| Ala-Tyr-Ile-Gln-*Asn-Ala-Cys*-Ala-Leu-Gly-NH₂ (SEO ID NO:154) | 31 | 0 |
| Ala-Tyr-Ile-*Gln-Asn-Cys*-Ala-Pro-Leu-Gly-NH₂ (SEQ ID NO:155) | 42 | 0 |
| Ala-Tyr-*Ile-Gln*-Cys-Asn-Ala-Pro-Leu-Gly-NH₂ (SEQ ID NO:156) | 18 | 0 |
| Ala-Tyr-Ile-Cys-Gln-Asn-Ala-Pro-Leu-Gly-NH₂ (SEQ ID NO:157) | 0 | 0 |
| *Ala-Tyr-Cys*-Ile-Gln-Asn-Ala-Pro-Leu-Gly-NH₂ (SEQ ID NO:158) | 6 | 1 |

Here too the MCD is shown in italics, with the endogenous Cys residues in the first and sixth positions replaced with an Ala. The data on this set of compounds demonstrates that the compounds are selective for the oxytocin receptor. The metallopeptide of SEQ ID NO:155 had maximal activity, while the two immediately adjacent metallopeptides, SEQ ID NO:154 and SEQ ID NO:156, were also active. It was again remarkable to observe that the most active metallopeptide was also constrained by metal complexation in the same region as the parent disulfide bridge constrained oxytocin. However, in this case, the metal ion induced constraint identified a more specified pair of amino acid residues, Gln-Asn, as the main amino acid residues that were structurally organized in a bioactive disposition. In the parent peptide four amino acid residues are within the disulfide constrain. The metallopeptide approach has therefore identified a more precise pharmacophore model.

### Example 6

A discrete library of peptides were developed based on the known angiotension ligand Sar-Arg-Val-Tyr-Ile-His-Pro-Thr (SEQ ID NO:159), wherein Sar is sarcosine, which served as the parent peptide. (Takei Y. et al., Gen Comp Endorinol 90:214, 1993) The peptides were made as described generally in Example 1, and were complexed with rhenium as described therein. The resulting metallopeptides, shown in **Table 31** below, were then screened for activity as described below.

The screening of compounds for binding to the angiotensin-II receptor was performed using cell membranes obtained from human neuroblastoma cells (KAN-TS). The assay was performed in triplicates, generally as described in Example 4 for oxytocin, except for measurement of receptor bound radioactivity. For angiotensin, a radioiodinated tracer ligand was used (instead of a tritiated ligand), which radioiodinated tracer facilitated direct measurement of bound radioactivity using a gamma counter. A final 1-3 nM concentration of ¹²⁵I-Tyr⁴, Sar¹, Ile⁸-Angiotensin II ligand (obtained from Perkin Elemer - NEN Life Sciences) was used as radiotracer and angiotensin-II (1 µM final assay concentration) was used to measure non-specific binding. After filtration of the incubation medium, followed by washings, drying the filters and punching the filters into test tubes, the filters were counted for radioactivity in a gamma counter. An activity profile for the test compounds was generated by ability to inhibit specific binding of the radiotracer to its receptor.

| **Table 31** | |
|---|---|
| **Peptide Sequences for Re Complexation to Form Metallopeptides and Percent Inhibit of Binding to Angiotensin Receptor** | |
| **Re Complexed Sequence** | **% Inhibition at 1 µM** |
| Sar-Arg-Val-Tyr-Ile-*His-Gly-Cys*-Thr (SEQ ID NO:160) | 5 |
| Sar-Arg-Val-Tyr-*Ile-His-Cys*-Pro-Thr (SEQ ID NO:161) | 60 |
| Sar-Arg-Val-*Tyr-Ile-Cys*-His-Pro-Thr (SEQ ID NO:162) | 20 |
| Sar-Arg-*Val-Tyr-Cys*-Ile-His-Pro-Thr (SEQ ID NO:163) | 12 |
| Sar-*Arg*-*Val*-*Cys*-Tyr-Ile-His-Pro-Thr (SEQ ID NO:164) | 1 |
| S*ar*-*Arg*-*Cys*-Val-Tyr-Ile-His-Pro-Thr (SEQ ID NO:165) | 1 |
| Sar-Arg-Val-Tyr-*Ile-His-Cys*-Gly-Thr (SEQ ID NO:166) | 11 |

Since the Pro was in the next to last position, it was not substituted except in SEQ ID NO:160, where it was substituted with Gly. In SEQ ID NO:166 Gly was substituted for Pro; it can be seen that the percent inhibition with SEQ ID NO:166 is significantly less than in SEQ ID NO:161, which differ only in the substitute of Gly for Pro, thereby demonstrating that the secondary amino group of Pro contributes to binding, and may be employed, in part, to define the pharmacophore of the receptor.

### Example 7

A discrete library of peptides were synthesized based on the amyloid beta-protein related peptides of **Table 3**. The following peptides of **Table 32** were synthesized, using an automated peptide synthesis machine, complexed with Re to form a resulting metallopeptide, which was then purified by HPLC.

| **Table 32** |
|---|
| **Synthesized Amyloid Beta-Protein Related Peptides For Use in Metallopeptides** |
| Ac-Leu-Pro-Phe-Phe-Asp-Cys-NH₂ (SEQ ID NO:167) |
| Ac-Leu-Pro-Phe-Phe-Cys-Asp-NH₂ (SEQ ID NO:168) |
| Ac-Leu-Ala-Phe-Phe-Cys-Asp-NH₂ (SEQ ID NO:169) |
| Ac-Leu-Ala-Phe-Cys-Phe-Asp-NH₂ (SEQ ID NO:170) |
| Ac-Leu-Ala-Cys-Phe-Phe-Asp-NH₂ (SEQ ID NO:171) |
| Ac-Leu-Pro-Phe-Phe-Asp-D-Cys-NH₂ |
| Ac-Leu-Pro-Phe-Phe-D-Cys-Asp-NH₂ |
| Ac-Leu-Ala-Phe-Phe-D-Cys-Asp-NH₂ |
| Ac-Leu-Ala-Phe-D-Cys-Phe-Asp-NH₂ |
| Ac-Leu-Ala-D-Cys-Phe-Phe-Asp-NH₂ |

The preceding examples can be repeated with similar success by substituting the generically or specifically described reactants and/or operating conditions of this invention for those used in the preceding examples.

Although the invention has been described in detail with particular reference to these preferred embodiments, other embodiments can achieve the same results. Variations and modifications of the present invention will be obvious to those skilled in the art.

## Claims

1. A method of determining a secondary structure binding to a receptor, which secondary structure is part of a known parent polypeptide that binds to the receptor, wherein the known parent polypeptide has a primary structure that is known to consist of n ordered amino acid residues, the method **characterized by**:
(a) constructing a plurality of peptides of the formula R₁-C-R₂, wherein each of the plurality of peptides of the formula R₁-C-R₂ is **characterized in that**:
R₁ comprises from 2 to n amino acid residues, such residues being the same as residues in the parent polypeptide and in the same order as amino acid residues in the parent polypeptide primary structure;
C is an L- or D-3-mercapto amino acid providing both a nitrogen (N) and a sulfur (S) for metal ion complexation;
R₂ comprises from 0 to n-2 residues, such residues being the same as residues in the parent polypeptide and in the same order as amino acid residues in the parent polypeptide primary structure, and forming with R₁ a sequence in the same order as in the parent polypeptide primary structure with C either (i) inserted between two adjacent residues corresponding to two adjacent residues in such primary structure, in which case R₁ and R₂ together contain a number of residues equal to n, or (ii) substituted for a single residue corresponding to a single residue in such primary structure, in which case R₁ and R₂ together contain a number of residues that is one less than n ;
wherein each member of the plurality of peptides differs by at least either R₁ or R₂ constituting a different number of residues or a different series of residues;
on the proviso that any cysteine residue in R₁ or R₂ is substituted with a glycine, alanine, serine, aminoisobutyric acid, dehydroalanine residue or S-protected cysteine, and further on the proviso that any proline residue in R₁ occupying either of the two positions immediately adjacent the amino-terminus side of C is substituted with a glycine, alanins, serine, aminoisobutyric acid, dehydroalanine residue or other neutral mimetic of an amino acid of less than about 150 MW and providing an N for metal ion complexation;
(b) complexing each of the plurality of peptides of the formula R₁-C-R₂ to a rhenium or technetium metal ion, thereby forming a: plurality of R₁-C-R₂ metallopeptides;
(c) screening each of the plurality of R₁-C-R₂ metallopeptides for binding to the receptor for the known parent polypeptide; and
(d) selecting the R₁-C-R₂ metallopeptide binding with the receptor for the known parent polypeptide whereby such R₁-C-R₂ metallopeptide includes the secondary structure binding to the target of interest.

2. The method of claim 1 wherein the L- or D-3-mercapto amino acid is L- or D-cysteine, L- or D-penicillamine, 3-mercapto phenylalanine, or a homologue of any of the foregoing.

3. The method of claim 1 wherein screening for binding to the receptor comprises competing a known binding partner for binding to the known receptor with the R₁-C-R₂ metallopeptide.

4. The method of claim 3 wherein the known binding partner is the parent polypeptide.

5. The method of claim 1 wherein screening for binding to the receptor comprises a functional assay.

6. The method of claim 1 wherein the receptor is a biological receptor capable of transmitting a signal, and screening further comprises determining whether the R₁-C-R₂ metallopeptide induces transmission of the signal.

7. The method of claim 1 wherein the receptor is a biological receptor capable of transmitting a signal, and screening further comprises determining whether the R₁-C-R₂ metallopeptide inhibits transmission of the signal in the presence of a binding partner to the target of interest known to induce transmission of the signal,

8. The method of claim 1 wherein R₁ and R₂ are each the same as residues in the parent polypeptide and in the same order as residues in the parent polypeptide primary structure.

9. The method of claim 1 wherein the peptides of the formula R₁-C-R₂ are constructed by a chemical method of peptide synthesis.

10. The method of claim 9 wherein the chemical method of peptide synthesis is solid phase synthesis.

11. The method of claim 9 wherein the chemical method of peptide synthesis is solution phase synthesis.

12. The method of claim 9 wherein C further comprises an orthogonal S-protecting group compatible with the chemical method of peptide synthesis, which orthogonal S-protecting group is cleavable at or prior to metal ion complexation.

13. The method of claim 1 wherein the peptides of the formula R₁-C-R₂ are constructed by expression in biological systems.

14. The method of claim 13 wherein expression in biological systems comprises use of a recombinant vector.

15. The method of claim 1 wherein the number of residues comprising R₁ and R₂ is one less than n.

16. The method of claim 1 wherein the number of residues comprising R₁ and R₂ is equal to n.

17. The method of claim 1 wherein the R₁-C-R₂ metallopeptide is stable in solution.

18. The method of claim 1 wherein the R₁-C-R₂ metallopeptide is a stable solid when not in solution.

19. The method of claim 1 further **characterized by** the peptides of the formula R₁-C-R₂ including an N-terminus free amino group or acetyl group.

20. The method of claim 1 further **characterized by** the peptides of the formula R₁-C-R₂ including a C-terminus free carboxylate or amide group.

## Patentansprüche

1. Ein Verfahren zum Bestimmen einer Sekundärstruktur, die sich an einen Rezeptor bindet, wobei die Sekundärstruktur ein Teil eines bekannten Vorgängerpolypeptids ist, das sich an den Rezeptor bindet, wobei das bekannte Vorgängerpolypeptid eine Primärstruktur aufweist, von der man weiß, dass sie aus n geordneten A-minsäureresten besteht, wobei das Verfahren durch folgende Schritte **gekennzeichnet** ist:
(a) Herstellen einer Mehrzahl von Peptiden der Formel R₁-C-R₂, wobei jedes der Mehrzahl von Peptiden der Formel R₁-C-R₂ **dadurch gekennzeichnet ist, dass**:
R₁ von 2 bis n Aminosäurereste aufweist, wobei derartige Reste dieselben sind wie Reste in dem Vorgängerpolypeptid und in derselben Reihenfolge wie Aminosäurereste in der Vorgänger-polypeptid-Primärstruktur;
C eine L- oder D-3-Mercaptoaminosäure ist, die sowohl einen Stickstoff (N) als auch einen Schwefel (S) für eine Metallionenkomplexbildung liefert;
R₂ von 0 bis n-2 Reste aufweist, wobei derartige Reste dieselben sind wie Reste in dem Vorgängerpolypeptid und in derselben Reihenfolge wie Aminosäurereste in der Vorgänger-polypeptid-Primärstruktur, und mit R₁ eine Sequenz in derselben Reihenfolge wie bei der Vorgängerpolypeptid-Primärstruktur bilden, wobei C entweder (i) zwischen zwei benachbarte Reste eingefügt ist, die zwei benachbarten Resten in einer derartigen Primärstruktur entsprechen, wobei in diesem Fall R₁ und R₂ zusammen eine Anzahl von Resten enthalten, die gleich n ist, oder (ii) statt eines Einzelrestes eingesetzt wird, der einem Einzelrest in einer derartigen Primärstruktur entspricht, wobei in diesem Fall R₁ und R₂ zusammen eine Anzahl von Resten enthalten, die eins weniger als n beträgt;
wobei sich jeder Angehörige der Mehrzahl von Peptiden zumindest darin unterscheidet, dass entweder R₁ oder R₂ eine unterschiedliche Anzahl von Resten oder eine unterschiedliche Serie von Resten bildet;
unter der Maßgabe, dass jeglicher Cysteinrest in R₁ oder R₂ durch einen Glycin-, Alanin-, Serin-, Aminoisobuttersäure-, Dehydroalaninrest oder S-geschütztes Cystein substituiert wird, und ferner unter der Maßgabe, dass jeglicher Prolinrest in R₁, der eine der zwei Positionen, die unmittelbar zu der Amino-Terminus-Seite von C benachbart sind, besetzt, durch einen Glycin-, Alanin-, Serin-, Aminoisobuttersäure-, Dehydroalaninrest oder eine andere neutrale Nachahmform einer Aminosäure von weniger als etwa 150 relative Molekülmasse, die ein N für eine Metallionenkomplexbildung liefert, substituiert wird;
(b) Komplexieren jedes der Mehrzahl von Peptiden der Formel R₁-C-R₂ zu einem Rhenium- oder Technetium-Metallion, wodurch eine Mehrzahl von R₁-C-R₂-Metallopeptiden gebildet wird;
(c) Prüfen jedes der Mehrzahl von R₁-C-R₂-Metallopeptiden bezüglich eines Bindens an den Rezeptor für das bekannte Vorgängerpolypeptid; und
(d) Auswählen des R₁-C-R₂-Metallopeptids, das mit dem Rezeptor für das bekannte Vorgängerpolypeptid bindet, wodurch ein derartiges R₁-C-R₂-Metallopeptid die Sekundärstruktur, die sich an das interessierende Target bindet, umfasst.

2. Das Verfahren gemäß Anspruch 1, bei dem die L- oder D-3-Mercaptoaminosäure L- oder D-Cystein, L- oder D-Penicillamin, 3-Mercapto-Phenylalanin oder ein Homolog eines Beliebigen der Vorstehenden ist.

3. Das Verfahren gemäß Anspruch 1, bei dem das Prüfen bezüglich eines Bindens an den Rezeptor ein Konkurrieren eines bekannten Bindungspartners bezüglich eines Bindens an den bekannten Rezeptor mit dem R₁-C-R₂-Metallopeptid umfasst.

4. Das Verfahren gemäß Anspruch 3, bei dem der bekannte Bindungspartner das Vorgängerpolypeptid ist.

5. Das Verfahren gemäß Anspruch 1, bei dem das Prüfen bezüglich eines Bindens an den Rezeptor eine Funktionsuntersuchung umfasst.

6. Das Verfahren gemäß Anspruch 1, bei dem der Rezeptor ein biologischer Rezeptor ist, der in der Lage ist, ein Signal zu senden, und das Prüfen ferner ein Bestimmen umfasst, ob das R₁-C-R₂-Metallopeptid ein Senden des Signals bewirkt.

7. Das Verfahren gemäß Anspruch 1, bei dem der Rezeptor ein biologischer Rezeptor ist, der in der Lage ist, ein Signal zu senden, und das Prüfen ferner ein Bestimmen umfasst, ob das R₁-C-R₂-Metallopeptid ein Senden des Signals in der Gegenwart eines Bindungspartners an das interessierende Target, von dem man weiß, dass es ein Senden des Signals bewirkt, hemmt.

8. Das Verfahren gemäß Anspruch 1, bei dem R₁ und R₂ jeweils dasselbe sind wie Reste in dem Vorgängerpolypeptid und in derselben Reihenfolge wie Reste in der Vorgängerpolypeptid-Primärstruktur.

9. Das Verfahren gemäß Anspruch 1, bei dem die Peptide der Formel R₁-C-R₂ anhand eines chemischen Verfahrens einer Peptidsynthese hergestellt werden.

10. Das Verfahren gemäß Anspruch 9, bei dem das chemische Verfahren der Peptidsynthese eine Festphasensynthese ist.

11. Das Verfahren gemäß Anspruch 9, bei dem das chemische Verfahren der Peptidsynthese eine Lösungsphasensynthese ist.

12. Das Verfahren gemäß Anspruch 9, bei dem C ferner eine orthogonale S-Schutzgruppe umfasst, die mit dem chemischen Verfahren der Peptidsynthese kompatibel ist, wobei die orthogonale S-Schutzgruppe bei oder vor einer Metallionenkomplexbildung spaltbar ist.

13. Das Verfahren gemäß Anspruch 1, bei dem die Peptide der Formel. R₁-C-R₂ anhand einer Expression in biologischen Systemen hergestellt werden.

14. Das Verfahren gemäß Anspruch 13, bei dem die Expression in biologischen Systemen eine Verwendung eines Rekombinationsvektors umfasst.

15. Das Verfahren gemäß Anspruch 1, bei dem die Anzahl von Resten, die R₁ und R₂ umfassen, eins weniger als n beträgt.

16. Das Verfahren gemäß Anspruch 1, bei dem die Anzahl von Resten, die R₁ und R₂ umfassen, gleich n ist.

17. Das Verfahren gemäß Anspruch 1, bei dem das R₁-C-R₂-Metallopeptid in Lösung stabil ist.

18. Das Verfahren gemäß Anspruch 1, bei dem das R₁-C-R₂-Metallopeptid ein stabiler Feststoff ist, wenn es sich nicht in Lösung befindet.

19. Das Verfahren gemäß Anspruch 1, ferner **dadurch gekennzeichnet, dass** die Peptide der Formel R₁-C-R₂ eine N-Terminus-freie Aminogruppe oder Acetylgruppe umfassen.

20. Das Verfahren gemäß Anspruch 1, ferner **dadurch dadurch gekennzeichnet, dass** die Peptide der Formel R₁-C-R₂ ein(e) C-Terminus--freie (s) Carboxylat oder Amidgruppe umfassen.

## Revendications

1. Méthode pour déterminer une structure secondaire se liant à un récepteur, structure secondaire qui fait partie d'un polypeptide parental connu qui se lie au récepteur, dans laquelle le polypeptide parental connu a une structure primaire qui est connue en tant que structure consistant en n résidus d'aminoacides ordonnés, méthode **caractérisée par** les étapes consistant à :
(a) construire une pluralité de peptides de formule R₁-C-R₂, dans laquelle chacun de la pluralité de peptide de formule R₁-C-R₂ est **caractérisé en ce que** :
R₁ comprend 2 à n résidus d'aminoacides, ces résidus étant identiques aux résidus dans le polypeptide parental et étant dans le même ordre que les résidus d'aminoacides dans la structure primaire du polypeptide parental ;
C représente un L- ou D-3-mercapto-aminoacide fournissant à la fois un atome d'azote N et un atome de soufre S pour la complexation d'un ion métallique,
R₂ comprend 0 à n-2 résidus, ces résidus étant identiques aux résidus présents dans le polypeptide parental et étant dans le même ordre que les résidus d'aminoacides dans la structure primaire du polypeptide parental, et formant avec R₁ une séquence dans le même ordre que dans la structure primaire du polypeptide parental avec C soit (i) inséré entre deux résidus adjacents correspondants à deux résidus adjacents dans cette structure primaire, auquel cas R₁ et R₂ contiennent conjointement un nombre de résidus égal à n, soit (ii) substitué en remplacement d'un résidu unique correspondant à un résidu unique dans cette structure primaire, auquel cas R₁ et R₂ contiennent conjointement un nombre de résidus qui est inférieur d'une unité à n ;
où chaque membre de la pluralité de peptides présente au moins comme différence la présence de R₁ ou R₂ représentant un nombre différent de résidus ou bien une série différente de résidus ;
sous réserve que n'importe quel résidu cystéine dans R₁ ou R₂ soit substitué par un résidu glycine, alanine, sérine, acide aminoisobutyrique ou déhydroalanine ou un résidu cystéine à atome de S protégé, et sous réserve en outre que n'importe quel résidu proline dans R₁ occupant l'une des deux positions immédiatement adjacentes au côté amino-terminal de C soit substitué par un résidu glycine, alanine, sérine, acide aminoisobutyrique ou déhydroalanine ou autre résidu neutre mimétique d'un aminoacide ayant un PM inférieur à environ 150 et fournissant un atome de N pour la complexation avec un ion métallique ;
(b) complexer chacun de la pluralité de peptides de formule R₁-C-R₂ avec un ion de métal rhénium ou technétium, en formant ainsi une pluralité de métallopeptides R₁-C-R₂ ;
(c) tester chacun de la pluralité de métallopeptides R₁-C-R₂ pour la liaison à un récepteur pour le polypeptide parental connu ; et
(d) sélectionner le métallopeptide R₁-C-R₂ se liant au récepteur pour le polypeptide parental connu, ce métallopeptide R₁-C-R₂ comprenant la structure secondaire se liant à la cible intéressante.

2. Méthode suivant la revendication 1, dans laquelle le L- ou D-3-mercapto-aminoacide est la L- ou D-cystéine, la L- ou D-pénicillamine, la 3-mercapto-phénylalanine, ou un homologue de n'importe lequel des acides précités.

3. Méthode suivant la revendication 1, dans laquelle le test de liaison au récepteur comprend la compétition d'un partenaire de liaison connu pour la liaison au récepteur connu avec le métallopeptide R₁-C-R₂.

4. Méthode suivant la revendication 3, dans laquelle le partenaire de liaison connu est le polypeptide parental.

5. Méthode suivant la revendication 1, dans laquelle le test de liaison au récepteur comprend une analyse fonctionnelle.

6. Méthode suivant la revendication 1, dans laquelle le récepteur est un récepteur biologique capable de transmettre un signal, et le test comprend en outre l'étape consistant à déterminer si le métallopeptide R₁-C-R₂ induit la transmission du signal.

7. Méthode suivant la revendication 1, dans laquelle le récepteur est un récepteur biologique capable de transmettre un signal, et le test comprend en outre l'étape consistant à déterminer si le métallopeptide R₁-C-R₂ inhibe la transmission du signal en présence d'un partenaire de liaison à la cible intéressante connu pour induire la transmission du signal.

8. Méthode suivant la revendication 1, dans laquelle R₁ et R₂ sont chacun identiques aux résidus dans le polypeptide parental et dans le même ordre que les résidus dans la structure primaire du polypeptide parental.

9. Méthode suivant la revendication 1, dans laquelle les peptides de formule R₁-C-R₂ sont construits par un procédé chimique de synthèse de peptides.

10. Méthode suivant la revendication 9, dans laquelle le procédé chimique de synthèse de peptides est la synthèse en phase solide.

11. Méthode suivant la revendication 9, dans laquelle le procédé chimique de synthèse de peptides est la synthèse en solution.

12. Méthode suivant la revendication 9, dans laquelle C comprend en outre un groupe orthogonal protecteur de l'atome de S compatible avec le procédé chimique de synthèse de peptides, groupe orthogonal protecteur de l'atome de S qui est clivable lors de ou avant la complexation avec un ion métallique.

13. Méthode suivant la revendication 1, dans laquelle les peptides de formule R₁-C-R₂ sont construits par expression dans des systèmes biologiques.

14. Méthode suivant la revendication 13, dans laquelle l'expression dans des systèmes biologiques comprend l'utilisation d'un vecteur recombinant.

15. Méthode suivant la revendication 1, dans laquelle le nombre de résidus constituant R₁ et R₂ est inférieur de 1 à n.

16. Méthode suivant la revendication 1, dans laquelle le nombre de résidus constituant R₁ et R₂ est égal à n.

17. Méthode suivant la revendication 1, dans laquelle le métallopeptide R₁-C-R₂ est stable en solution.

18. Méthode suivant la revendication 1, dans laquelle le métallopeptide R₁-C-R₂ est une substance solide stable lorsqu'il n'est pas en solution.

19. Méthode suivant la revendication 1, **caractérisée en outre par** les peptides de formule R₁-C-R₂ comprenant un groupe amino ou acétyle libre à l'extrémité N-terminale.

20. Méthode suivant la revendication 1, **caractérisée en outre par** les peptides de formule R₁-C-R₂ comprenant un groupe carboxylate ou amide libre à l'extrémité C-terminale.
